# EUROPÄISCHE PATENTSCHRIFT

(11) **EP 1 019 423 B1**
(45) Veröffentlichungstag und Bekanntmachung des Hinweises auf die Patenterteilung: **26.02.2003**
(21) Anmeldenummer: 98950062.4
(22) Anmeldetag: 21.09.1998
(51) Int. Cl.: C07H 1/00

(54) **VERFAHREN ZUR HERSTELLUNG EINES PENTOPYRANOSYL-NUCLEOSIDS**
METHOD FOR PRODUCING A PENTOPYRANOSYL NUCLEOSIDE
PROCEDE DE PREPARATION D'UN NUCLEOSIDE DE PENTOPYRANOSYLE

(30) Priorität: 22.09.1997 DE 19741715
(43) Veröffentlichungstag der Anmeldung: 19.07.2000
(73) Patentinhaber: Nanogen Recognomics GmbH, 65926 Frankfurt am Main (DE)
(72) Erfinder: MICULKA, Christian, D-65929 Frankfurt am Main (DE); WINDHAB, Norbert, D-65795 Hattersheim (DE); BRANDSTETTER, Tilmann, D-80339 München (DE); BURDINSKI, Gerhard, D-56355 Nastätten (DE)
(74) Vertreter: Ackermann, Joachim, Dr.
(86) Internationale Anmeldenummer: EP9805998
(87) Internationale Veröffentlichungsnummer: WO99015540

(56) Entgegenhaltungen:
- S.PITSCH AT AL.: "147. Why Pentose and Not Hexose-Nucleic Acids ? Pyranosyl-RNA ('p-RNA')." HELVETICA CHIMICA ACTA., Bd. 76, Nr. 7, 1993, Seiten 2161-2183, XP002094190 BASEL CH in der Anmeldung erwähnt
- M.BÖHRINGER ET AL.: "110. Warum Pentose- und nicht Hexose-Nucleinsäuren ? Oligonucleotide aus 2',3'-Dideoxy-B-D-glucopyranosyl-Bausteine n ('Homo-DNS?): Herstellung." HELVETICA CHIMICA ACTA., Bd. 75, Nr. 5, 1992, Seiten 1416-1477, XP002096856 BASEL CH

## Beschreibung

Die vorliegende Erfindung betrifft ein Pentopyranosyl-Nucleosid 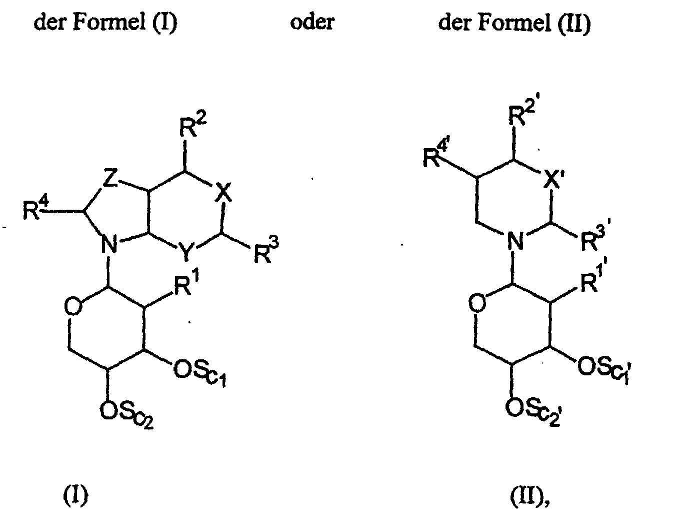 deren Herstellung und Verwendung zur Herstellung eines Therapeutikums, Diagnostikums und/oder elektronischen Bauteils.

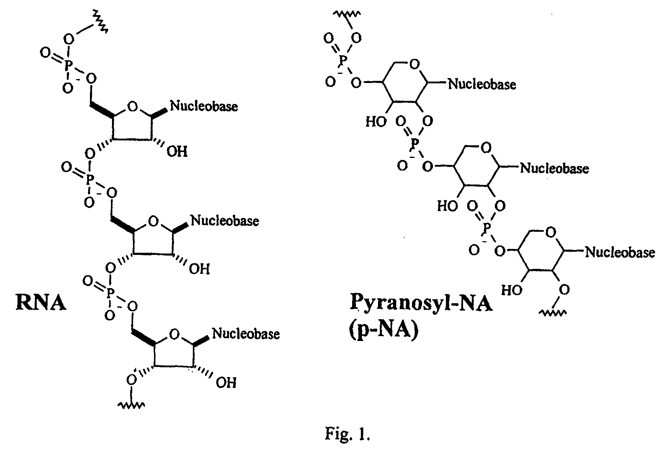

Pyranosyl-Nucleinsäuren (p-NA's) sind im allgemeinen zur natürlichen RNA isomere Strukturtypen, bei denen die Pentose-Einheiten in der Pyranoseform vorliegen und durch Phosphodiestergruppen zwischen den Positionen C-2' und C-4' repetitiv verknüpft sind (Fig. 1). Unter "Nucleobase" werden dabei die kanonischen Nucleobasen A, T, U, C, G, aber auch die Paare Isoguanin/Isocytosin und 2,6-Diaminopurin/Xanthin und im Sinne der vorliegenden Erfindung auch andere Purine und Pyrimidine verstanden. p-NA's, und zwar die von der Ribose abgeleitete p-RNA's, wurden zum erstenmal von Eschenmoser et al. beschrieben (siehe Pitsch, S. et al. Helv. Chim. Acta 1993, 76, 2161; Pitsch, S. et al. Helv. Chim Acta 1995, 78, 1621; Angew. Chem. 1996, 108, 1619-1623). Sie bilden ausschließlich sogenannte Watson-Crick-gepaarte, d. h. Purin-Pyrimidin- und Purin-Purin-gepaarte, antiparallele, reversibel "schmelzende", quasi-lineare und stabile Duplices. Homochirale p-RNA-Stränge entgegengesetzten Chiralitätssinns paaren ebenfalls kontrollierbar und sind in der gebildeten Duplex streng nicht-helical. Diese für den Aufbau supramolekularer Einheiten wertvolle Spezifität hängt mit der relativ geringen Flexibilität des Ribopyranosephosphat-Rückgrats sowie mit der starken Neigung der Basenebene zur Strangachse und der hieraus folgenden Tendenz zu intercatenarer Basenstapelung im resultierenden Duplex zusammen und läßt sich letzlich auf die Teilnahme eines 2',4'-cis-disubstituierten Ribopyranoserings am Aufbau des Rückgrates zurückführen. Diese wesentlich besseren Paarungseigenschaften machen p-NA's gegenüber DNA und RNA für die Anwendung des Aufbaus supramolekularer Einheiten zu bevorzugten Paarungssystemen. Sie bilden ein zu natürlichen Nucleinsäuren orthogonales Paarungsystem, d. h. sie paaren nicht mit in der natürlich Form vorkommenden DNA's und RNA's, was im besonderen im diagnostischen Bereich von Bedeutung ist.

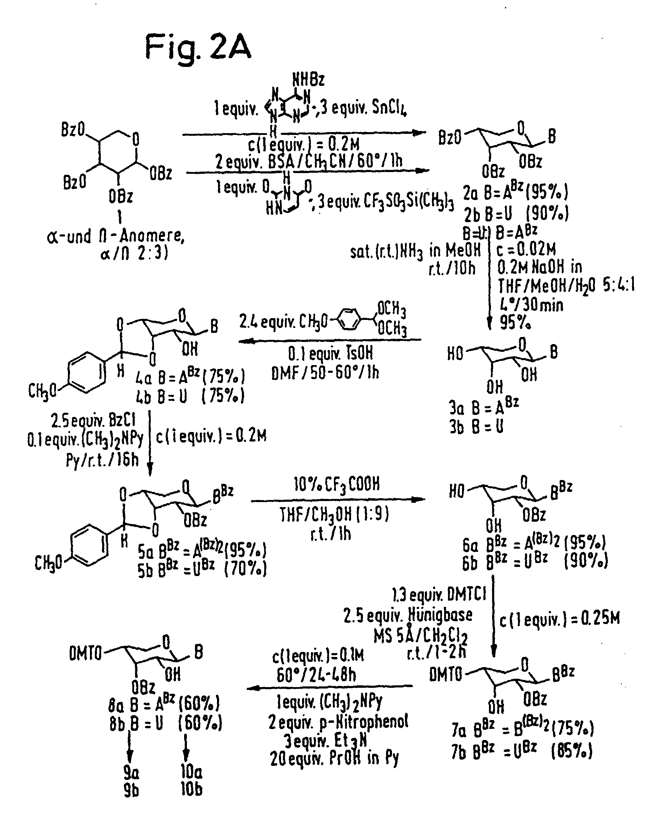

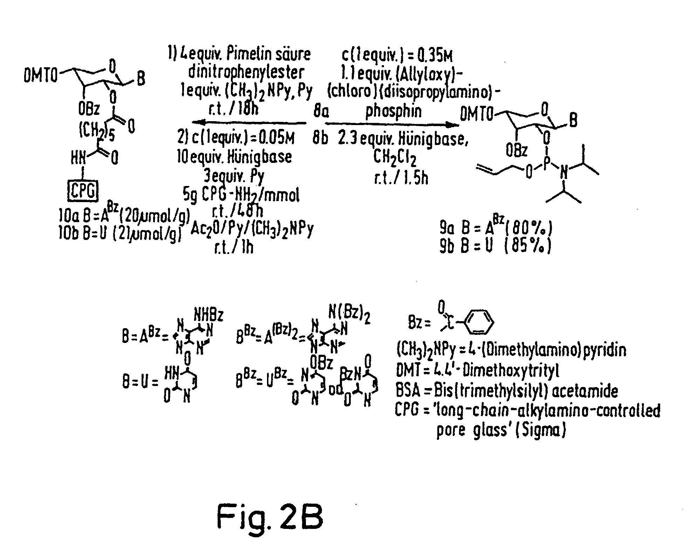

Eschenmoser et al. (1993, supra) hat zum ersten Mal eine p-RNA, wie in Fig. 2 dargestellt und nachstehend erläutert, hergestellt.

Hierbei wurde eine geeignete geschützte Nucleobase mit dem Anomerengemisch der Tetrabenzoyl-Ribopyranose durch Einwirken von Bis(trimethylsilyl)acetamid und einer Lewis-Säure wie z. B. Trimethylsilyl-trifluormethansulfonat zur Reaktion gebracht (analog H. Vorbrüggen, K. Krolikiewicz, B. Bennua, Chem. Ber. 1981, 114, 1234.). Unter Baseneinwirkung (NaOH in THF(Methanol/Wasser im Falle der Purine; gesättigter Ammoniak in MeOH im Falle der Pyrimidine) wurden die Acylschutzgruppen vom Zucker abgespalten, und das Produkt unter saurer Katalyse mit p-Anisaldehyddimethylacetal in 3',4'-Position geschützt. Das Diastereomerengemisch wurde in 2'-Stellung acyliert, das 3',4'methoxybenzylidengeschützte 2'-Benzoat durch saure Behandlung, z. B. mit Trifluoressigsäure in Methanol deacetalisiert, und mit Dimethoxytritylchlorid umgesetzt. Die 2'→3'-Wanderung des Benzoats wurde durch Behandlung mit p-Nitrophenol/4-(Dimethylamino)pyridin/Triethylamin/Pyridin/n-Propanol eingeleitet. Fast alle Reaktionen wurden durch Säulenchromatographie aufgearbeitet. Der so synthetisierte Schlüsselbaustein, das 4'-DMT-3'-benzoyl-1'-Nucleobasen-Derivat der Ribopyranose, wurde dann zum Teil phosphityliert bzw. über einen Linker an eine feste Phase gebunden.

Bei der anschließenden automatisierten Oligonucleotidsynthese wurde die trägergebundene Komponente in 4'-Position wiederholt sauer entschützt, ein Phosphoramidit unter Einwirkung eines Kupplungsreagenz, z. B. ein Tetrazolderivat, angekuppelt, noch freie 4'-Sauerstoffatome acetyliert und das Phosphoratom oxidiert, um so das oligomere Produkt zu erhalten.

Anschließend wurden die restlichen Schutzgruppen abgespalten, das Produkt über HPLC gereinigt und entsalzt.

Das beschriebene Verfahren von Eschenmoser et al. (1993, supra) zeigt jedoch folgende Nachteile:
1. Der Einsatz nicht anomerenreiner Tetrabenzoyl-pentopyranosen (H. G. Fletcher, J. Am. Chem. Soc. 1955, 77, 5337) zur Nucleosidierungsreaktion mit Nucleobasen verringert durch die Notwendigkeit rigoros geschnittener Chromatographien in den nachfolgenden Arbeitsschritten die Ausbeuten des Endproduktes.
2. Die Synthese ist mit fünf Reaktionsstufen, ausgehend von Ribopyranosen, die in 1'-Stellung eine Nucleobase aufweisen, bis zum geschützten 3'-Benzoat, sehr langwierig und eine Durchführung im industriellen Maßstab ist kaum möglich. Zusätzlich zu dem hohen Zeitaufwand sind die erhaltenen Ausbeuten an Monomerbausteinen gering: 29 % im Falle des Purinbausteins Adenin, 24 % im Falle des Pyrimidinbausteins Uracil.
3. Bei der Synthese der Oligonucleotide wird 5-(4-Nitrophenyl)-1H-tetrazol als Kupplungsreagenz in der automatisierten p-RNA-Synthese eingesetzt. Die Konzentration dieses Reagenz in der Lösung von Tetrazol in Acetonitril ist dabei so hoch, daß regelmäßig das 5-(4-Nitrophenyl)-1H-tetrazols in den dünnen Schläuchen des Synthesizers auskristallisiert und die Synthese somit zu einem vorzeitigen Ende kommt. Zudem wurde beobachtet, daß die Oligomeren mit 5-(4-Nitrophenyl)-1H-tetrazol verunreinigt waren.
4. Die beschriebene Aufarbeitung von p-RNA-Oligonucleotiden, im speziellen die Abspaltung der basenlabilen Schutzgruppen mit Hydrazinlösung, gelingt bei hohem Thymidin-Anteil im Oligomeren nicht immer.

Aufgabe der vorliegenden Erfindung war es daher ein neues Verfahren zur Herstellung von Pentopyranosyl-Nucleosiden bereitzustellen, wodurch die Herstellung von bekannten und neuen Pentopyranosyl-Nucleosiden im größeren Maßstab als nach bekannten Verfahren ermöglicht werden soll und die oben beschriebenen Nachteile vermieden werden.

Gegenstand der vorliegenden Erfindung ist daher ein Verfahren zur Herstellung eines Pentopyranosyl-Nucleosids, bei dem ausgehend von dem ungeschützten Pentopyranosid
(a) in einem ersten Schritt zuerst die 2'-, 3'- oder 4'-Position des Pentopyranosids geschützt wird, und
(b) in einem zweiten Schritt eine der verbleibenden Positionen an der 2'-, 3'- oder 4'.

In einer bevorzugten Ausführungsform wird ein Pentopyranosyl-Nucleosid der Formel (I), 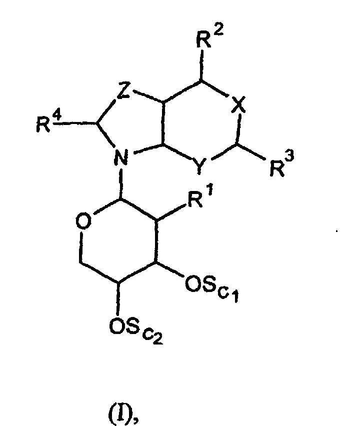 worin
R¹ gleich H, OH, Hal mit Hal gleich Br oder Cl, oder ein Rest ausgewählt aus 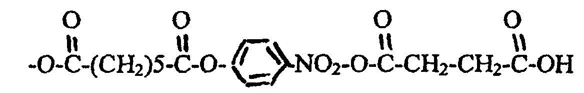 oder -O-P[N(i-Pr)₂] (OCH₂CH₂CN) mit i-Pr gleich Isopropyl, oder eine 0-2' Schutzgruppe, vorzugsweise eine basen- oder metallkatalysiert abspaltbaren Schutzgruppe, insbesondere eine Acylgruppe, besonders bevorzugt eine Benzoylgruppe, R², R³ und R⁴ unabhängig voneinander, gleich oder verschieden, jeweils H, Hal mit Hal gleich Br oder Cl, NRSR6, OR⁷, SR⁸, =O, CₙH₂ₙ₊₁ mit n eine ganze Zahl von 1-12, vorzugsweise 1-8, insbesondere 1-4, eine β-eliminierbare Gruppe, vorzugsweise eine Gruppe der Formel -OCH₂CH₂R¹⁸ mit R¹⁸ gleich ein Cyano- oder p-Nitrophenylrest oder ein Fluorenylmethyloxycarbonyl-(Fmoc)-Rest, oder (CₙH₂ₙ)NR¹⁰R¹¹ mit R¹⁰R¹¹ gleich H, CₙH₂ₙ₊₁ oder R¹⁰R¹¹ verbunden über einen Rest der Formel 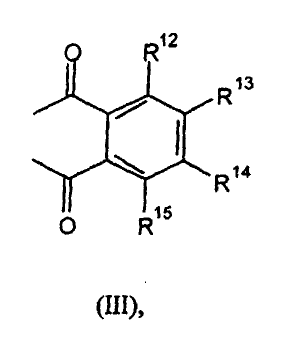 worin R¹², R¹³, R¹⁴ und R¹⁵ unabhängig voneinander, gleich oder verschieden, jeweils H, OR⁷, wobei R⁷ die oben genannte Bedeutung hat, oder CₙH₂ₙ₊₁, oder CₙH₂ₙ₋₁, wobei n die oben genannte Bedeutung hat, bedeuten und
R⁵, R⁶, R⁷ und R⁸ unabhängig voneinander, gleich oder verschieden, jeweils H, CₙH₂ₙ₊₁, oder CₙH₂ₙ₋₁, wobei n die oben genannte Bedeutung hat, -C(O)R⁹ mit R⁹ gleich ein linearer oder verzweigter, gegebenenfalls substituierter Alkyl-, Aryl-, vorzugsweise Phenyl-Rest,
X, Y und Z unabhängig voneinander, gleich oder verschieden, jeweils =N-, =C(R¹⁶)- oder -N(R¹⁷)- mit R¹⁶ und R¹⁷ unabhängig voneinander, gleich oder verschieden, jeweils H oder CₙH₂ₙ₊₁ oder (CₙH₂ₙ)NR¹⁰R¹¹ mit den oben genannten Bedeutungen, bedeutet, und
S_{c1} und S_{c2} unabhängig voneinander, gleich oder verschieden, jeweils H oder eine Schutzgruppe ausgewählt aus einer Acyl-, Trityl- oder Allyloxycarbonylgruppe, vorzugsweise eine Benzoyl- oder 4, 4'-Dimethoxytrityl-(DMT-)gruppe,
oder der Formel (II) 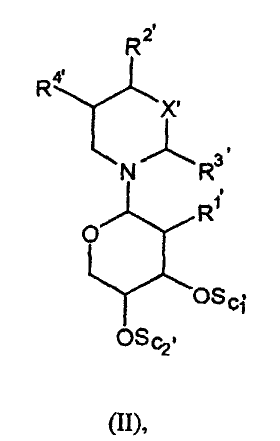 worin R^{1'} gleich H, OH, Hal mit Hal gleich Br oder Cl oder oder ein Rest ausgewählt aus 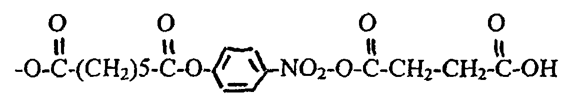 oder -O-P[N(i-Pr)₂] (OCH₂CH₂CN) mit i-Pr gleich Isopropyl, oder eine 0-2' Schutzgruppe, vorzugsweise eine basen- oder metallkatalysiert abspaltbaren Schutzgruppe. insbesondere eine Acylgruppe, besonders bevorzugt eine Benzoylgruppe, R^{2'},R^{3'} und R^{4'} unabhängig voneinander, gleich oder verschieden, jeweils H, Hal mit Hal gleich Br oder Cl, =O, CₙH₂ₙ₊₁ oder OCₙH₂ₙ₋₁, eine β-eliminierbare Gruppe, vorzugsweise eine Gruppe der Formel -OCH₂CH₂R¹⁸ mit R¹⁸ gleich ein Cyano- oder p-Nitrophenylrest oder ein Fluorenylmethyloxycarbonyl-(Fmoc)-Rest, oder (CₙH₂ₙ)NR^{10'}R^{11'}, wobei R^{10'}, R^{11'}, unabhängig voneinander die oben genannte Bedeutung von R¹⁰ bzw. R¹¹ hat, und
X' jeweils =N-, =C(R^{16'})- oder -N(R^{17'}) bedeutet, wobei R^{16'} und R^{17'} unabhängig voneinander die oben genannte Bedeutung von R¹⁶ bzw. R¹⁷ haben, und S_{c1'} bzw. S_{c2'} die oben genannte Bedeutung von S_{c1} bzw. S_{c2} haben..

Das erfindungsgemäße Pentopyranosyl-Nucleosid ist im allgemeinen ein Ribo-, Arabino-, Lyxo- und/oder Xylo-pyranosyl-Nucleosid, vorzugsweise ein Ribopyranosyl-Nucleosid, wobei der Pentopyranosyl-Teil D-konfiguriert, aber auch L-konfiguriert sein kann.

Üblicherweise handelt es sich bei dem erfindungsgemäßen Pentopyranosyl-Nucleosid um ein Pentopyranosyl-purin, -2,6-diaminopurin, -6-purinthiol, -pyridin, -pyrimidin, -adenosin, -guanosin, -isoguanosin, -6-thioguanosin, -xanthin, -hypoxanthin, -thymidin, -cytosin, -isocytosin , -indol, -tryptamin, -N-phthaloyltryptamin, -uracil, -coffein, -theobromin, -theophyllin, -benzotriazol oder -acridin, insbesondere um ein Pentopyranosyl-purin, -pyrimidin, -adenosin, -guanosin, -thymidin, -cytosin, tryptamin, -N-phthalotryptamin oder -uracil.

Unter die Verbindungen fallen auch Pentopyranosyl-Nucleoside, die als Linker verwendet werden können, d. h. als Verbindungen mit funktionellen Gruppen, die kovalent an Biomoleküle, wie z. B. in ihrer natürlichen Form vorkommende oder modifizierte Nucleinsäuren, wie DNA, RNA aber auch p-NA's, vorzugsweise pRNA's, binden können. Dies ist überraschend, da für p-NA's noch keine Linker bekannt sind.

Beispielsweise fallen hierunter Pentopyranosyl-Nucleoside, bei denen R², R³, R⁴, R^{2'}, R^{3'} und/oder R^{4'} ein 2-Phthalimidoethyl- oder Allyloxy-Rest bedeutet. Bevorzugt sind gemäß der vorliegenden Erfindung beispielsweise Uracil-basierende Linker, bei denen vorzugsweise die 5-Position des Uracils modifiziert wurde, z. B. N-Phthaloylaminoethyluracil, aber auch Indolbasierende Linker, vorzugsweise Tryptaminderivate, wie z. B. N-Phthaloyltryptamin.

Überraschenderweise werden durch die vorliegende Erfindung auch besser handhabbare Pentopyranosyl-N,N-Diacylnucleoside, vorzugsweise Purine, insbesonders Adenosin, Guanosin oder 6-Thioguanosin, bereitgestellt, deren Nucleobase auf einfache Weise vollkommen entschützt werden können. Daher gehören zu der Erfindung auch Pentopyranosyl-Nucleoside, bei denen R², R³, R⁴, R^{2'}, R^{3'} und/oder R^{4'} ein Rest der Formel -N[C(O)R⁹]₂ bedeutet, insbesondere N⁶, N⁶-Dibenzoyl-9-(β-D-ribopyranosyl)-adenosin.

Weiterhin ist es überraschend, daß die vorliegende Erfindung Pentopyranosyl-Nucleoside bereitstellt, die ausschließlich am 3'-Sauerstoffatom des Pentopyranosid-Teils eine Schutzgruppe, vorzugsweise eine basen- oder metallkatalysiert abspaltbare Schutzgruppe, insbesondere eine Acylgruppe, besonders bevorzugt eine Benzoylgruppe, tragen. Diese Verbindungen dienen z. B. als Ausgangsstoffe zur direkten Einführung einer weiteren Schutzgruppe, vorzugsweise einer säure- oder basenlabilen Schutzgruppe, insbesondere einer Tritylgruppe, besonders bevorzugt eine Dimethoxytritylgruppe, an das 4'-Sauerstoffatom des Pentopyranosid-Teils ohne zusätzliche, die Ausbeute verringernde Schritte, wie z. B. zusätzliche Reinigungsschritte.

Darüberhinaus stellt die vorliegende Erfindung Pentopyranosyl-Nucleoside bereit, die ausschließlich am 4'-Sauerstoffatom des Pentopyranosid-Teils eine Schutzgruppe, vorzugsweise einer säure- oder basenlabilen Schutzgruppe, insbesondere einer Tritylgruppe, besonders bevorzugt eine Dimethoxytritylgruppe, tragen. Auch diese Verbindungen dienen z. B. als Ausgangsstoffe zur direkten Einführung einer weiteren Schutzgruppe, vorzugsweise einer basen- oder metallkatalysiert abspaltbaren Schutzgruppe, insbesondere einer Acylgruppe, besonders bevorzugt einer Benzoylgruppe, z. B. an dem 2'-Sauerstoffatom des Pentopyranosid-Teils, ohne zusätzliche, die Ausbeute verringernde Schritte, wie z. B. zusätzliche Reinigungsschritte.

Im allgemeinen können die erfindungsgemäßen Pentopyranosid-Nucleoside in einer sogenannten Ein-Topf-Reaktion umgesetzt werden, was die Ausbeuten erhöht und daher besonders vorteilhaft ist.

Folgende Verbindungen stellen bevorzugte Beispiele der Pentopyranosyl-Nucleoside dar:
A) [2',4'-Di-O-Benzoyl)-β-ribopyranosyl]-Nucleoside, insbesondere ein [2',4'-Di-O-Benzoyl)-β-ribopyranosyl]-adenin,- guanin, -cytosin, -thymidin, -uracil, -xanthin oder -hypoxanthin, sowie ein N-Benzoyl-2',4' -di-O-benzoyl-ribopyranasyl=Nucleosid, insbesondere ein -adenin, - guanin oder -cytosin, sowie ein N-Isobutyroyl-2', 4'-di-Obenzoyl-ribopyranosyl-Nucleosid, insbesondere ein -adenin, -guanin oder -cytosin, sowie ein O⁶-(2-Cyanoethyl)-N²-isobutyroyl-2',4'-di-O-benzoyl-ribopyranosyl-Nucleosid, insbesondere ein -guanin, sowie ein O⁶(2-(4-Nitrophenyl)ethyl)-N²-isobutyroyl-2',4'-di-O-benzoylribopyranosyl-Nucleosid, ins-besondere ein -guanin.
B) β-Ribopyranosyl-Nucleoside, insbesondere ein β-Ribopyranosyl-adenin, -guanin, -cytosin, -thymidin oder -uracil, -xanthin oder hypoxanthin, sowie ein N-Benzoyl-, N-Isobutyroyl-, O⁶ -(2-Cyanoethyl)- oder O⁶-(2-(4-Nitrophenyl)ethyl)-N²-isobutylroyl-β-ribopyranosyl-Nucleosid.
C) 4'-DMT-pentopyranosyl-Nucleoside, vorzugsweise ein 4'-DMT-ribopyranosyl-Nucleosid, insbesondere ein 4'-DMT-ribopyranosyl-adenin, -guanin, -cytosin, -thymidin, -uracil, -xanthin, -hypoxanthin, sowie ein N-Benzoyl-4'-DMT-ribopyranosyl-Nucleosid, insbesondere ein N-Benzoyl-4'-DMT-ribopyranosyl-adenin, -guanin oder -cytosin, sowie ein N-Isobutyroyl-4'-DMT-ribopyranosyl-Nucleosid, insbesondere ein N-Isobutyroyl-4'-DMTribopyranosyl-adenin, -guanin oder -cytosin sowie ein O⁶-(2-Cyanoethyl)-N²-isobutyroyl-4'-DMT-ribopyranosyl-Nucleosid, insbesondere ein O⁶-(2-Cyanoethyl)-N²-isobutyroyl-4'-DMTribopyranosyl-guanin, sowie ein O⁶-(2-(-4-Nitrophenyl)ethyl)-N²-isobutyroyl-4'-DMTribopyranosyl-Nucleosid, insbesondere ein O⁶-(2-(4-Nitrophenyl)ethyl)-N²-isobutyroyl-4'-DMT-ribopyranosyl-guanin.
D) β-Ribopyranosyl-N,N'-dibenzoyl-adenosin oder β-Ribopyranosyl-N,N'-dibenzoylguanosin.

Als Vorstufe für die Oligonucleotidsynthese eigenen sich beispielsweise 4'-DMTpentopyranosyl-Nucleoside-2'-phosphitamid/-H-phosphonat, vorzugsweise ein 4'DMTribopyranosyl-Nucleosid-2'-phosphitamid/-H-phosphonat, insbesondere ein 4'-DMTribopyranosyl-adenin-, -guanin-, cytosin-, -thymidin-, -xanthin-, -hypoxanthin-, oder -uracil-2'-phosphitamid/-H-phosphonat sowie ein N-Benzoyl-4'-DMT-ribopyranosyl-adenin-,guanin- oder -cytosin-2'-phosphitamid/-H-phosphonat sowie ein N-Isobutylroyl-4'-DMTribopyranosyl-adenin-, -guanin- oder -cytosin-2'-phosphitamid/-H-phosphonat, O⁶-(2-Cyanoethyl)-4'-DMT-ribopyranosyl-guanin-, -xanthin-, -hypoxanthin-2'-phosphitamid/-Hphosphonat oder O⁶-(2-(4-Nitrophenyl)ethyl)-N²-isobutyroyl-4'-DMT-ribopyranosyl-guanin, und für die Kopplung an den festen Träger beispielsweise 4'-DMT-pentopyranosyl-Nucleoside-2'-succinat, vorzugsweise ein 4'-DMT-ribopyranosyl-Nucleosid-2'-succinat, insbesondere ein 4'DMT-ribopyranosyl-adenin-, -guanin-, -cytosin-, -thymidin- -xanthin-, -hypoxanthin- oder -uracil-2'-succinat sowi ein N-Benzoyl-4'-DMT-ribopyranosyl-adenin-, -guanin- oder -cytonsin-2'-succinat sowie ein N-Isobutyroyl-4'-DMT-ribopyranosyl-adenin-,guanin- oder -cytosin-2'- succinat, O-(2-Cyanoethyl)-4'-DMT-ribopyranosyl-guanin-2'succinat sowie ein O⁶-(2-(4-Nitrophenyl)ethyl)-N²-isobutyroyl-4'-DMT-ribopyranosylguanin-2'- succinat.

Das erfindungsgemäße Verfahren ist nicht auf die in der zitierten Literatur beschriebenen Nucleobasen beschränkt, sondern kann überraschenderweise mit einer Vielzahl von natürlichen und synthetischen Nucleobasen erfolgreich durchgeführt werden. Zudem ist es besonders überraschend, daß das erfindungsgemäße Verfahren im Vergleich zu dem literaturbekannten Verfahren in großen Ausbeuten und mit einer Zeitersparnis von durchschnittlich 60% durchgerührt werden kann, was für die industrielle Anwendung besonders vorteilhaft ist. Zusätzlich sind mit dem erfindungsgemäßen Verfahren die bei dem in der Literatur beschriebenen Verfahren nötigen Reinigungsschritte, z. B. chromatographische Zwischenreinigungen, nicht notwendig und die Reaktionen können teilweise als sogenannnte Ein-Topf-Reaktion durchgeführt werden, was die Raum/Zeit-Ausbeuten deutlich erhöht.

In einer besonderen Ausführungsform erfolgt im Falle einer 2'-geschützten Position eine Umlagerung der Schutzgruppe von der 2'-Position zur 3'-Position, die im allgemeinen in Gegenwart einer Base, insbesondere in Gegenwart von N-Ethyldiisopropylamin und/oder Triethylamin durchgeführt wird. Diese Reaktion kann gemäß der vorliegenden Erfindung besonders vorteilhaft in dem gleichen Reaktionsbehältnis als Ein-Topf-Reaktion durchgerührt werden.

In einer weiteren bevorzugten Ausführungsform ist das Pyranosyl-nucleosid durch eine säurelabile, basenlabile oder metallkatalysiert abspaltbare Schutzgruppe S_{c1}, S_{c2}, S_{c1'} oder S_{c2'} geschützt, wobei vorzugsweise die Schutzgruppen S_{c1} und S_{c1'} von den Schutzgruppen S_{c2} bzw. S_{c2'} verschieden sind.

Im allgemeinen handelt es sich bei den genannten Schutzgruppen um eine Acylgruppe, vorzugsweise um eine Acetyl-, Benzoyl-, Nitrobenzoyl- und/oder Methoxybenzoyl-Gruppe, um Tritylgruppen, vorzugsweise um eine 4, 4'-Dimethoxytrityl-(DMT)-Gruppe oder um eine β-eliminierbare Gruppe, vorzugsweise eine Gruppe der Formel -OCH₂ CH₂ R¹⁸ mit R¹⁸ gleich ein Cyano- oder p-Nitrophenylrest oder eine Fluorenylmethyloxycarbonyl-(Fmoc)Gruppe.

Besonders bevorzugt ist es. wenn die 2'- oder 3'-Position durch eine basenlabile oder metallkatalysiert abspaltbare Schutzgruppe, vorzugsweise durch eine Acylgruppe, insbesondere durch eine Acetyl-, Benzoyl-, Nitrobenzoyl- und/oder Methoxybenzoylgruppe, geschützt wird und/oder die 4'-Position durch eine säure- oder basenlabile Schutzgruppe, vorzugsweise durch eine Trityl- und/oder Fmoc-Gruppe, insbesondere durch eine DMT-Gruppe geschützt wird.

Das erfindungsgemäße Verfahren kommt folglich im Gegensatz zu dem literaturbekannten Verfahren ohne acetalische Schutzgruppen, wie Acetale oder Ketale, aus, was zusätzliche chromatographische Zwischenreinigungen vermeidet und folglich die Reaktionen als Ein-Topf-Reaktionen mit überraschend hohen Raum/Zeit-Ausbeuten durchführen läßt.

Die Einführung der genannten Schutzgruppen erfolgt vorzugsweise bei tiefen Temperaturen, da diese hierdurch überraschenderweise selektiv eingeführt werden können.

So erfolgt beispielsweise die Einführung einer Benzoylgruppe durch Umsetzen mit Benzoylchlorid in Pyridin bzw. in einem Pyridin/Methylenchlorid-Gemisch bei tiefen Temperaturen. Die Einführung einer DMT-Gruppe kann beispielsweise durch Umsetzen mit DMTCI in Anwesenheit einer Base, z. B. von N-Ethyldiisopropylamin (Hünig-Base), und z. B. von Pyridin, Methylenchlorid oder einem Pyridin/Methylenchlorid-Gemisch bei Raumtemperatur erfolgen.

Es ist auch vorteilhaft, wenn nach der Acylierung und/oder nach der gegebenenfalls erfolgten Umlagerung von der 2'- zu der 3'-Position die Reaktionsprodukte chromatographisch gereinigt werden. Eine Reinigung nach der Tritylierung ist gemäß dem erfindungsgemäßem Verfahren nicht notwendig, was besonders vorteilhaft ist.

Das Endprodukt kann, falls notwendig, noch durch Kristallisation weiter gereinigt werden.

Ein anderer Gegenstand der vorliegenden Erfindung ist ein Verfahren zur Herstellung eines Ribopyranosyl-Nucleosids, bei dem
(a) eine geschützte Nucleobase mit einer geschützten Ribopyranose umgesetzt wird,
(b) die Schutzgruppen von dem Ribopyranosyl-Teil des Produktes aus Schritt (a) abgespalten werden, und
(c) das Produkt aus Schritt (b) gemäß dem oben näher beschriebenen Verfahren umgesetzt wird.

Hierbei ist es zur Vermeidung weiterer zeit- und materialaufwendiger Chromatographien vorteilhaft, nur anomerenreine geschützte Pentopyranosen, wie z. B. Tetrabenzoyl-pentopyranosen, vorzugsweise β-Tetrabenzoyl-ribopyranosen (R. Jeanloz, J. Am. Chem. Soc. 1948, 70,4052), einzusetzen.

In einer weiteren Ausführungsform wird ein Pentopyranosyl-Nucleosid-Linker gemäß Formel (II), worin R^{4'} (CₙH₂ₙ)NR^{10'}R^{11'} bedeutet und R^{10'}R^{11'} über einen Rest der Formel (III) mit der bereits bezeichneten Bedeutung verbunden ist, durch folgendes Verfahren auf vorteilhafte Weise hergestellt:
(a) eine Verbindung der Formel (II) mit R^{4'} gleich (CₙH₂ₙ)OS_{c3} oder (CₙH₂ₙ)Hal, worin n die oben genannte Bedeutung hat, S_{c3} eine Schutzgruppe, vorzugsweise eine Mesylat-Gruppe, und Hal Chlor oder Brom bedeutet, wird mit einem Azid, vorzugsweise in DMF, umgesetzt, anschließend wird
(b) das Reaktionsprodukt aus (a), vorzugsweise mit Triphenylphosphin z. B. in Pyridin reduziert, dann
(c) das Reaktionsprodukt aus (b) mit einem entsprechenden Phthalimid, z. B. N-Ethoxycarbonylphthalimid, umgesetzt, und
(e) die Hydroxyl-Schutzgruppen des Pyranosyl-Teils des Produktes aus (c), z. B. mit Methylat, abgespalten, und
(f) die weiteren Schritte, wie oben bereits beschrieben, durchgeführt.

Daneben weisen Indolderivate als Linker den Vorzug der Fluoreszenzfähigkeit auf und sind daher für Nanotechnologie-Anwendungen, bei denen es ggf. um den Nachweis kleinster Substanzmengen geht, besonders bevorzugt. So wurden Indol-1-riboside bei N. N. Suvorov et al., Biol. Aktivn. Soedin., Akad. Nauk SSSR 1965, 60 und Tetrahedron 1967,23,4653 bereits beschrieben. Allerdings gibt es kein analoges Verfahren, 3-substituierte Derivate herzustellen.. Im allgemeinen erfolgt ihre Herstellung über die Bildung eines Aminals der ungeschützten Zuckerkomponente und einem Indolin, welches dann durch Oxidation in das Indol-1-ribosid übergeführt wird. Beschrieben wurden z. B. Indol-1-glucoside und -1-arabinoside (Y. V. Dobriynin et al, Khim.-Farm. Zh. 1978, 12, 33), deren 3-substituierte Derivate meist über Vielsmeier-Reaktion hergestellt wurden. Dieser Weg der Einführung von Aminoethyl-Einheiten in 3-Position des Indols ist für eine industrielle Anwendung jedoch zu aufwendig.

In einer weiteren besonderen Ausführungsform wird daher ein Pentopyranosyl-Nucleosid-Linker gemäß Formel (I), worin X und Y unabhängig voneinander, gleich oder verschieden, jeweils =C(R¹⁶) mit R¹⁶ gleich H oder CₙH₂ₙ und Z =C(R¹⁶)- mit R¹⁶ gleich (CₙH₂ₙ)NR¹⁰R¹¹ durch folgendes Verfahren auf vorteilhafte Weise hergestellt:
(a) das entsprechende Indolin, z. B. N-Phthaloyltryptamin, wird mit einer Pyranose, z. B. D-Ribose, zum Nucleosidtriol umgesetzt, dann werden
(b) die Hydroxylgruppen des Pyranosyl-Teils des Produktes aus (a) vorzugsweise mit Acylgruppen, z. B. mittels Essigsäureanhydrid, geschützt, anschließend wird
(c) das Produkt aus (b), z. B. durch 2,3-Dichlor-5,6-dicyanoparachinon, oxidiert, und
(d) die Hydroxyl-Schutzgruppen des Pyranosyl-Teils des Produktes aus (c) werden z. B. mittels Methylat abgespalten und anschließend werden
(e) die weiteren Schritte, wie oben bereits beschrieben, durchgeführt.

Dieses Verfahren läßt sich jedoch nicht nur bei Ribopyranosen anwenden, sondern auch bei Ribofuranosen und 2'-Deoxyribofuranosen bzw. 2'-Deoxyribopyranosen, was besonders vorteilhaft ist. Als Nucleosidierungspartner der Zucker wird vorzugsweise Tryptamin, insbesondere N-Acylderivate des Tryptamins, vor allem N-Phthaloyltryptamin verwendet.

In einer weiteren Ausführungsform werden die 4'- geschützten, vorzugsweise, die 3', 4'geschützten Pentopyranosyl-Nucleoside in einem weiteren Schritt phosphityliert oder an eine feste Phase gebunden.

Die Phosphitylierung erfolgt beispielsweise durch Phosphorigsäuremonoallylesterdiisopropyl-amidchlorid in Anwesenheit einer Base, z. B. N-Ethyldiisopropylamin oder durch Phosphortrichlorid und Imidazol bzw. Tetrazol und nachfolgender Hydrolyse unter Basenzusatz. Im ersten Fall ist das Produkt ein Phosphoramidit und im zweiten Fall ein H-Phosphonat. Die Bindung eines geschützten erfindungsgemäßen Pentopyranosyl-Nucleosids an eine feste Phase, z. B. "long-chain-alkylamino-controlled pore glass" (CPG, Sigma Chemie, München) kann beispielsweise wie bei Eschenmoser et al. (1993) beschrieben erfolgen.

Die erhaltenen Verbindungen dienen z. B. für die Herstellung von Pentopyranosyl-Nucleinsäuren.

Ein weiterer Gegenstand der vorliegenden Erfindung ist daher ein Verfahren zur Herstellung einer Pentopyranosyl-Nucleinsäure, mit folgenden Schritten:
(a) in einem ersten Schritt wird ein geschütztes Pentopyranosyl-Nucleosid, wie oben bereits beschrieben, an eine feste Phase gebunden wird und
(b) in einem zweiten Schritt wird das gemäß Schritt (a) an eine feste Phase gebundene 3'-, 4'geschützte Pentopyranosylnukleosid um ein phosphityliertes 3'-, 4'-geschütztes Pentopyranosyl-Nucleosid verlängert und anschließend z. B. durch eine wäßrige Jodlösung oxidiert wird, und
(c) Schritt (b) solange mit gleichen oder unterschiedlichen phosphitylierten 3'-, 4'geschützten Pentopyranosyl-Nucleosiden wiederholt, bis die gewünschte Pentopyranosyl-Nucleinsäure vorliegt.

Als Kupplungsreagenz bei Einsatz von Phosphoramiditen eignen sich saure Aktivatoren wie Pyridinium-Hydrochlorid besonders Benzimidazoliumtriflat, vorzugsweise nach Umkristallisieren in Acetonitril und nach Lösen in Acetonitril, da im Gegensatz zu 5-(4-Nitrophenyl)-1H-tetrazol als Kupplungsreagenz keine Verstopfung der Kupplungsreagenz-Leitungen und eine Verunreinigung des Produktes erfolgt.

Als Kupplungsreagenz beim Einsatz von H-Phosphonaten eignen sich besonders Arylsulfonylchloride, Diphenylchlorophosphat, Pivaloylchlorid oder Adamantoylchlorid.

Weiterhin ist es vorteilhaft durch Zusatz von einem Salz, wie Natriumchlorid, zur schutzgruppenabspaltenden Hydrazinolyse von Oligonukleotiden, insbesondere von p-NA's, vorzugsweise von p-RNA's, Pyrimidinbasen, vor allem Uracil und Thymin, vor einer Ringöffnung zu schützen, die das Oligonukleotid zerstören würde. Allyloxygruppen können vorzugsweise durch Palladium [Pd(0)]-Komplexe z. B. vor der Hydrazinolyse abgespalten werden.

In einer weiteren besonderen Ausführungsform können in Schritt (a) und/oder Schritt (b) auch Pentofuranosyl-nucleoside, z. B. das in ihrer natürlichen Form vorkommende Adenosin, Guanosin, Cytidin, Thymidin und/oder Uracil, eingebaut werden, was z. B. zu einer gemischten p-NA-DNA bzw. p-NA-RNA führt.

In einer anderen besonderen Ausführungsform kann in einem weiteren Schritt ein Allyloxy-Linker der Formel

S_{c4}NH(CₙH₂ₙ)CH(OPS_{c5}S_{c6})CₙH₂ₙS_{c7} (IV),

worin S_{c4} und S_{c7} unabhängig voneinander, gleich oder verschieden, jeweils eine Schutzgruppe insbesondere ausgewählt aus Fmoc und/oder DMT,
S_{c5} und S_{c6} unabhängig voneinander, gleich oder verschieden, jeweils eine Allyloxy- und/oder Diisopropylamino-Gruppe bedeuten, eingebaut werden, n hat die bereits oben genannte Bedeutung.

Ein besonders bevorzugter Allyloxy-Linker ist (2-(S)-N-Fmoc-O'-DMT-O²allyloxydiisopropylaminophosphinyl-6-amino-1,2-hexandiol).

Ausgehend von z. B. Lysin können somit in wenigen Reaktionsschritten amino-terminale Linker aufgebaut werden, die sowohl eine aktivierbare Phosphorverbindung als auch eine säurelabile Schutzgruppe, wie DMT, tragen und daher leicht in der automatisierbaren Oligonucleotidsynthese verwendet werden können (siehe z. B. P. S. Nelson et al., Nucleic Acid Res. 1989, 17, 7179; L. J. Arnold et al., WO 8902439). Das Repertoire wurde in der vorliegenden Erfindung durch einen Lysin-basierender Linker erweitert, bei dem anstelle der sonst üblichen Cyanoethyl-Gruppe am Phosphoratom eine Allyloxy-Gruppe eingebracht wurde, und welcher daher in vorteilhafter Weise in der Noyori-Oligonucleotid-Methode eingesetzt werden kann (R. Noyori, J. Am. Chem. Soc. 1990, 112, 1691-6).

p-NA's und insbesondere die p-RNA's bilden untereinander stabile Duplices und paaren im allgemeinen nicht mit den in ihrer. natürlichen Form vorkommenden DNA's und RNA's. Diese Eigenschaft macht p-NA's zu bevorzugten Paarungssystemen.

Solche Paarungssysteme sind supramolekulare Systeme nicht kovalenter Wechselwirkung, die sich durch Selektivität, Stabilität und Reversiblität auszeichnen, und deren Eigenschaften bevorzugt thermodynamisch, d. h. durch Temperatur, pH-Wert und Konzentration beeinflußt werden. Solche Paarungssysteme können z. B. aufgrund ihrer selektiven Eigenschaften auch als "molekularer Klebstoff" für die Zusammenführung von unterschiedlichen Metallclustern zu Cluster-Verbänden mit potentiell neuen Eigenschaften verwendet werden [siehe z. B. R. L. Letsinger, et al., Nature 1996, 382, 607-9; P. G. Schultz et al., Nature 1996, 382, 609-11]. Folglich eignen sich die p-NA's auch für die Anwendung im Bereich der Nanotechnologie, beispielsweise zur Herstellung neuer Materialien, Diagnostika und Therapeutika sowie mikroelektronischer, photonischer bzw. optoelektronischer Bauteile und für das kontrollierte Zusammenführen molekularer Species zu supramolekularen Einheiten, wie z. B. für den (kombinatorischen) Aufbau von Proteinassemblies [siehe z. B. A. Lombardi, J. W. Bryson, W. F. DeGrado, Biomoleküls (Pept. Sci.) 1997, 40, 495-504], da p-NA's Paarungssysteme bilden, die stark und thermodynamisch kontrollierbar sind. Eine weitere Anwendung ergibt sich daher gerade im diagnostischen und drug discovery-Bereich durch die Möglichkeit, funktionelle, bevorzugt biologische Einheiten wie Proteine oder DNA/RNA-Abschnitte, mit einem p-NA-Code zu versehen, der nicht mit den natürlichen Nucleinsäuren interferiert (siehe z. B. WO93/20242).

Ein Biomolekül, z. B. DNA oder RNA. kann zum nicht-kovalenten Verbinden (Linken) mit einem anderen Biomolekül, z. B. DNA oder RNA, verwendet werden, wenn beide Biomoleküle Abschnitte enthalten, die aufgrund komplementärer Sequenzen von Nucleobasen durch Ausbildung von Wasserstoffbrücken aneinander binden können. Derartige Biomoleküle finden z. B. in analytischen Systemen zur Signalamplifizierung Verwendung, wo ein in seiner Sequenz zu analysierendes DNA-Molekül über einen solchen nicht-kovalenten DNA-Linker zum einen an einen festen Träger immobilisiert, und zum anderen an ein signalverstärkendes branchedDNA-Molekül (bDNA) gebunden werden soll (siehe z. B. S. Urdea, Bio/Technol. 1994, 12, 926 oder US-Patent Nr. 5,624,802). Ein wesentlicher Nachteil der zuletzt beschriebenen Systeme ist, daß sie den Verfahren zur Nucleinsäure-Diagnostik durch Polymerase-Chain-Reaction (PCR) (K. Mullis, Methods Enzymol. 1987, 155, 335) hinsichtlich der Empfindlichkeit bis jetzt unterlegen sind. Das ist u. a. darauf zurückzuführen, daß die nicht-kovalente Bindung vom festen Träger an das zu analysierende DNA-Molekül ebenso wie die nicht-kovalente Bindung des zu analysierenden DNA-Moleküls nicht immer spezifisch erfolgt, wodurch es zu einer Vermischung der Funktionen "Sequenzerkennung" und "nicht-kovalente Bindung" kommt. Die Verwendung von p-NA's als orthogonales Paarungssystem, welches nicht in das DNA- bzw. RNA-Paarungsgeschehen eingreift, löst dieses Problem auf vorteilhafte Weise, wodurch die Empfindlichkeit der beschriebenen analytischen Verfahren deutlich erhöht werden kann.

Die gemäß dem erfindungsgemäßen Verfahren hergestellten Pentopyranosyl-Nucleoside oder Pentopyranosyl-Nucleinsäuren eignen sich daher zur Herstellung eines Arzneimittels, wie z. B. eines Therapeutikums, Diagnostikums und/oder elektronischen Bauteils, beispielsweise in Form eines Konjugates, d. h. in Kombination mit einem Biomolekül.

Konjugate sind im Sinne der vorliegenden Erfindung kovalent gebundene Hybride aus p-NA's und anderen Biomolekülen, vorzugsweise ein Peptid, Protein oder eine Nucleinsäure, beispielsweise ein Antikörper oder ein funktioneller Teil davon oder eine in ihrer natürlichen Form vorkommende DNA und/oder RNA. Funktionelle Teile von Antikörper sind beispielsweise Fv-Fragmente (Skerra & Plückthun (1988) Science 240, 1038), einzelkettige Fv-Fragmente (scFv; Bird et al. (1988), Science 242, 423; Huston et al. (1988) Proc. Natl. Acad. Sci. U.S.A., 85, 5879) oder Fab-Fragmente (Better et al. (1988) Science 240, 1041).

Unter Biomolekül versteht man im Sinne der vorliegenden Erfindung eine natürlich vorkommende oder eine von einer natürlich vorkommenden Substanz abgeleitete Substanz.

In einer bevorzugten Ausführungsform handelt es sich dabei um p-RNA/DNA- bzw p-RNA/RNA-Konjugate.

Konjugate werden dann vorzugsweise verwendet, wenn die Funktionen "Sequenzerkennung" und "nicht-kovalente Bindung" in einem Molekül realisiert werden müssen, da die erfindungsgemäßen Konjugate zwei zueinander orthogonale Paarungssysteme enthalten.

Für die Herstellung von Konjugaten sind sowohl sequentielle als auch konvergente Verfahren geeignet.

In einem sequentiellen Verfahren wird z. B. nach erfolgter automatisierter Synthese eines p-RNA-Oligomeren direkt am gleichen Synthesizer - nach Umstellung der Reagenzien und des Kupplungsprotokolls - z. B. ein DNA-Oligonukleotid weitersynthetisiert. Dieser Vorgang läßt sich auch in umgekehrter Reihenfolge durchführen.

In einem konvergenten Verfahren werden z. B. p-RNA-Oligomere mit Aminoterminalen-Linkem und z. B. DNA-Oligomere mit z. B. Thiol-Linkern in getrennten Vorgängen synthetisiert. Anschließend erfolgt vorzugsweise eine Jodacetylierung des p-RNA-Oligomeren und die Kupplung der beiden Einheiten nach literaturbekannten Protokollen (T. Zhu et al., Bioconjug. Chem. 1994, 5, 312).

Konvergente Verfahren erweisen sich aufgrund ihrer Flexibilität als besonders bevorzugt.

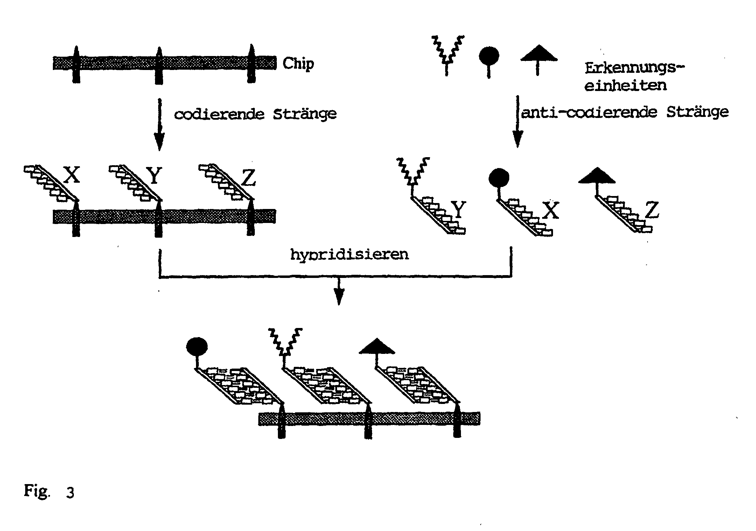

Unter dem Begriff Konjugat im Sinne der vorliegenden Erfindung sind auch sogenannte Arrays zu verstehen. Arrays sind Anordnungen von immobilisierten Erkennungsspecies, die speziell in der Analytik und Diagnostik eine wichtige Rolle bei der simultanen Bestimmung von Analyten spielen. Beispiele sind Peptide-Arrays (Fodor et al., Nature 1993, 364, 555) und Nucleinsäure-Arrays (Southern et al. Genomics 1992, 13, 1008; Heller, US-Patent Nr. 5,632,957). Eine höhere Flexibilität dieser Arrays kann dadurch erreicht werden, daß die Erkennungsspecies an codierende Oligonucleotide gebunden werden und die zugehörigen, komplementären Stränge an bestimmte Positionen auf einem festen Träger. Durch Aufbringen der codierten Erkennungsspecies auf den "anti-codierten" festen Träger und Einstellung von Hybridisierungsbedingungen werden die Erkennungsspecies an den gewünschten Positionen nicht-kovalent gebunden. Dadurch können verschiedene Typen von Erkennungsspecies, wie z. B. DNA-Abschnitte, Antikörper, nur durch Anwendung von Hybridisierungsbedingungen gleichzeitig auf einem festen Träger angeordnet werden (siehe Fig. 3.). Voraussetzung hierzu sind aber äußerst starke, selektive - um die codierenden Abschnitte möglichst kurz zu halten - und mit natürlicher Nucleinsäure nicht interferierender Codons und Anticodons notwendig. p-NA's, vorzugsweise p-RNA's eignen sich hierzu in besonders vorteilhafter Weise.

Unter dem Begriff "Träger" versteht man im Sinne der vorliegenden Erfindung Material, insbesondere Chipmaterial, das in fester oder auch gelartiger Form vorliegt. Als Trägermaterialien eignen sich beispielsweise Keramik, Metall, insbesondere Edelmetall, Gläser, Kunststoffe, kristalline Materialien bzw. dünne Schichten des Trägers, insbesondere der genannten Materialien, oder (bio)molekulare Filamente wie Cellulose, Gerüstproteine.

Die vorliegende Erfindung betrifft daher auch die Verwendung von Pentopyranosyl-Nucleinsäuren, vorzugsweise Ribopyranosyl-Nucleinsäuren zur Codierung von Erkennungsspecies, bevorzugt natürliche DNA- oder RNA-Stränge oder Proteine insbesondere Antikörper oder funktionelle Teile von Antikörpern. Diese können dann gemäß Fig. 4. mit den zugehörigen Codons auf einem festen Träger hybridisiert werden. Damit kann auf einem festen Träger, der in Form eines Arrays mit Codons ausgestattet ist, nur durch Einstellung von Hybridisierungsbedingungen mit immer neuen Kombinationen von Erkennungsspecies an den gewünschten Positionen immer neue, diagnostisch nützliche Arrays aufgebaut werden. Wird dann der Analyt, beispielsweise eine biologische Probe wie Serum o. ä. aufgebracht, dann werden die zu detektierenden Species in einem bestimmten Muster auf dem Array gebunden, welches dann indirekt (z. B. durch Fluoreszenzmarkierung der Erkennungsspecies) oder direkt (z. B. durch Impedanzmessung am Anknüpfungspunkt der Codons) registriert wird. Dann wird die Hybridisierung durch geeignete Bedingung aufgehoben (Termperatur, Salze, Lösungsmittel, elektrophoretische Vorgänge) so daß wieder nur der Träger mit den Codons zurückbleibt. Dieser wird dann erneut mit anderen Erkennungsspecies beladen und wird z. B. für den gleichen Analyten für die Ermittlung eines anderen Musters verwendet. Die immer neue Anordnung von Erkennungsspecies im Array-Format und die Verwendung von p-NA's als Paarungssysteme ist gegenüber anderen Systemen, siehe z. B. WO 96/13522 (s. 16, unten), besonders vorteilhaft.

Ein weiterer Gegenstand der vorliegenden Erfindung bezieht sich daher insbesondere auch auf ein Diagnostikum enthaltend ein oben beschriebenes Pentopyranosyl-Nucleosid oder ein erfindungsgemäßes Konjugat, wie oben bereits näher beschrieben.

Die folgenden Figuren und Beispiele sollen die Erfindung näher beschreiben, ohne sie zu beschränken.

### BESCHREIBUNG DER FIGUREN

- Fig. 1: zeigt einen Ausschnitt aus der Struktur von RNA in ihrer natürlich vorkommenden Form (links) und in Form einer p-NA (rechts).
- Fig. 2: zeigt schematisch die Synthese eines p-Ribo(A,U)-Oligonucleotids nach Eschenmoser et al. (1993).
- Fig. 3: zeigt schematisch eine Anordnung von immobilisierten Erkennungsstrukturen (Arrays) auf einem festen Träger.

### BEISPIELE

### Beispiel 1

### Synthese des 1-{3'-O-Benzoyl-4'-O-[(4,4'-dimethoxytriphenyl)-methyl]-β-D-ribo-pyranosyl}-thymins

Zuerst 4'-Substitution, dann 2'-Substitution, dann Wanderungsreaktion:

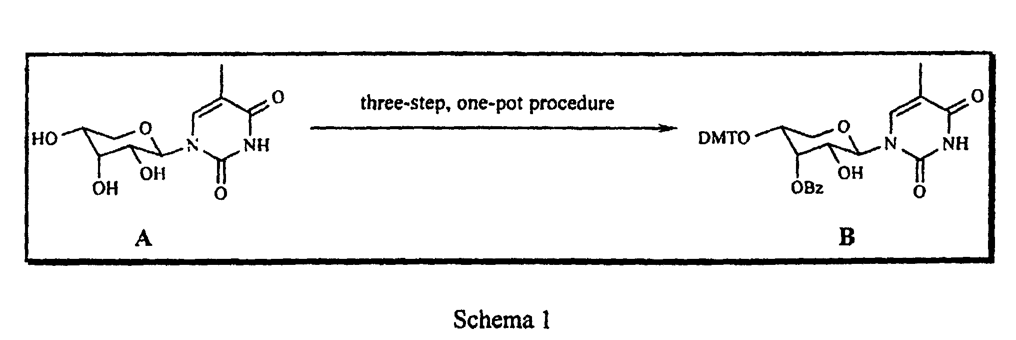

Unter Argonatmosphäre wurden 51,6 g (200 mmol) 1-(β-D-Ribo-pyranosyl)-thymin A in 620 ml wasserfreiem Pyridin gelöst, 71,4 ml (2,1 eq.) N-Ethyldiisopropylamin und 100 g Molsieb (4 Å) zugesetzt und 15 min lang mit KPG-Rührer gerührt. Man löste 92 g (272 mmol; 1,36 eq.) Dimethoxytritylchlorid (DMTCI) in 280 ml (frisch von festem NaHCO₃ destilliertem) Chloroform, und tropfte diese Lösung innerhalb von 30 min bei -6 bis -5 °C der Triollösung zu. Es wurde 1 h bei dieser Temp., dann über Nacht bei Raumtemperatur (RT.) gerührt, wieder gekühlt, und weitere 25 g (74 mmol; 0,37 eq.) DMTCI in 70 ml Chloroform zugesetzt. Man ließ auf RT. kommen und rührte 4 h nach.
Eine kleine Probe wurde entnommen, wäßrig aufgearbeitet und chromatographiert, um die analytischen Daten des 1-{4'-O-[(4,4'-dimethoxytriphenyl)-methyl]-β-D-ribo-pyranosyl}thymins zu erhalten:
^{**1**}**H-NMR** (300 MHz, CDCl3): 1,70 (bs, 2H, OH); 1,84 (d, 3H, Me); 2,90 (bs, 1 H, OH); 3,18, 3,30 (2m, 2H, H(5')), 3,62 (bs, 1H, H(3')); 3,70-3,82 (m, 8 H, 2 OMe, H(4'), H(2')); 5,75 (d, J = 9,5 Hz, 1H, H(1')), 6,85 (m, 4H, Harom); 6,96 (m, 1 H, Harom), 7,20 (m, 9H, Harom, H(6)), 8,70 (bs, 1 H, H(3).)

Das Reaktionsgemisch wurde mit 2,46 g (20,5 mmol; 0,1 eq.) 4-Dimethylaminopyridin (DMAP) versetzt, auf-6 °C gekühlt und zwischen -6 und -1 °C innerhalb von 15 min 27,9 ml (0,24 mol; 1,2 eq.) Benzoylchlorid (BzCl) in 30 ml Pyridin zugetropft und 10 min nachgerührt. Zur vollständigen Umsetzung wurden dann im Abstand von 25 min noch jeweils 2,8 ml (24 mmol; 0,12 eq.) BzCl unter Kühlung zugesetzt und schließlich 20 min gerührt.

Man setzte bei RT. 460 ml wasserfreies Pyridin, 841 ml (11,2 mol; 56 eq.) n-Propanol, 44 g (0,316 mol; 1,58 eq.) p-Nitrophenol, 21,7 g (0,18 mol; 0,9 eq.) DMAP und 136 ml (0,8 mol; 4 eq.) N-Ethyldiisopropylamin zu und rührte bei 61 - 63 °C 48 h lang. Dann blieb der Ansatz bei RT. 60 h stehen. Das Reaktionsgemisch wurde noch 24 h lang auf 61- 63 °C erwärmt, auf RT, abgekühlt und am Rotavapor eingeengt. Der Rückstand wurde in 2 l Ethylacetat aufgenommen, das Molsieb abfiltriert, die org. Phase dreimal mit je 1 l Wasser extrahiert, einmal mit 1,2 l 10 %iger Citronensäure 5 min ausgerührt und die org. Phase wieder abgetrennt, einmal mit 1 l Wasser und zum Abschluß mit 1 l gesättigter NaHCO₃-Lösung extrahiert. Die org. Phase wurde mit Natriumsulfat getrocknet, filtriert und eingeengt (220 g Rückstand).

Man filtrierte zuerst über Kieselgel 60 (20 x 10 cm) mit Stufengradienten Heptan/Ethylacetat, 1:1 bis 0:1) zur Vorreinigung, dann wurde an Kieselgel 60 chromatographiert (30 x 10 cm; Stufengradient Dichlormethan/Ethylacetat, 1:0 bis 1:1).
- Man erhielt:: 40 g unpolare Fraktionen
52,9 g 1-{3'-O-Benzoyl-4'-O-[(4,4'-dimethoxytriphenyl)-methyl]-β-D-ribo-pyranosyl}-thymin B
34,5 g verunreinigtes B
3,4 g polare Fraktionen

Die verunreinigte Fraktion wurde erneut chromatographiert (KG 60, 45 x 10 cm; Dichlormethan/Ethylacetat, 3:1) und lieferte weitere 11,3 g **B**.

Gesamtausbeute: 64,2 g (97 mmol) **B**, d. s. 48 % Ausbeute. ¹H-NMR entspricht.

### Beispiel 2

### Synthese des N⁴-Benzoyl-1-{3'-O-benzoyl-4'-O-[(4,4'-dimethoxytriphenyl)-methyl]-β-D-ribopyranosyl}-cytosins

Zuerst 2'-Substitution, dann 4'-Substitution, dann Wanderungsreaktion:

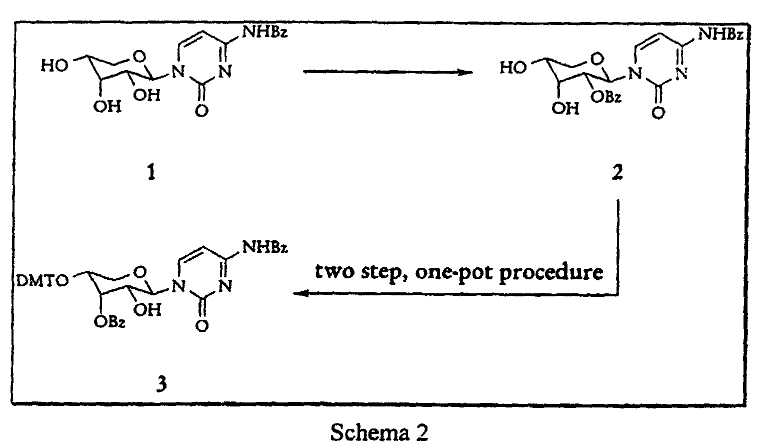

Alle Ansätze wurden unter N₂-Atmosphäre durchgeführt.

### N⁴-Benzoyl-1-(2'-O-benzoyl-β-D-ribo-pyranosyl)-cytosin 2:

54,0 g (0,155 mol) N⁴-Benzoyl-1-(β-D-ribo-pyranosyl)-cytosin **1** wurden in 830 ml Dimethylformamid (DMF) und 1,5 l Pyridin (beide Lösungsm. getrocknet und gelagert über Molsieb 3 Å) unter Erwärmen auf 124 °C gelöst. Bei -58° bis -63 °C wurden 23,0 g (0,163 mol; 1,05 eq.) BzCl, gelöst in 210 ml Pyridin, in 3,5 h zugetropft. Der Ansatz wurde im Kühlbad über Nacht gerührt. 90,3 g (1,5 mol; 10 eq.) n-Propanol wurden eingerührt und der Ansatz bei 40 °C im HV. eingeengt. Durch zweimalige Zugabe von 150 ml Toluol und erneutes Einengen wurden Pyridinreste entfernt. 124,3 g Rückstand wurden in 500 ml CH₂Cl₂ gelöst, zweimal mit je 300 ml halbkonzentrierter NaHCO₃-Lösung ausgerührt, und der ausgefallene Feststoff abfiltriert und getrocknet: 60,7 g Rückstand. Die CH₂Cl₂-Phase wurde eingeengt: 25,0 g. Getrennte Chromatographie an Kieselgel 60 (40 x 10 cm) mit Gradienten (AcOEt/iso-Hexan, 4:1, dann reines AcOEt, dann AcOEt/MeOH, 19:1 bis 2:1) lieferte (DC (Kieselgel, AcOEt)):

| | | | |
|---|---|---|---|
| 16,8 g | 2',4'-Dibenzoat | (24 %) | R_{f} 0,5. |
| 12,4 g | **1** | (23%) | R_{f} 0,0. |
| 35,4 g | **2** | (51 %) | R_{f} 0,14. |

### N⁴-Benzoyl-1-{3'-O-benzoyl-4'-O-[(4,4'-dimethoxytriphenyl)-methyl]-β-D-ribopyranosyl}-cytosin 3:

35,4 g (78 mmol) **2** wurden in 390 ml CH₂Cl₂ und 180 ml Pyridin (beides wasserfrei) gelöst und 0.94 (7,8 mmol; 0,1 eq.) DMAP, 34,6 ml (203 mmol; 2,6 eq.) N-Ethyldiisopropylamin und 33,1 g (98 mmol; 1,25 eq.) DMTCI zugegeben und 2 h bei RT. gerührt.
DC (Kieselgel, AcOEt): R_{f} 0,6.
CH₂Cl₂ wurde bei 30 °C abgezogen, der Rückstand mit 640 ml Pyridin, 9,37 (78 mmol; 1,0 eq.) DMAP, 32,5 ml (234 mmol; 3,0 eq.) Et₃N, 21,7 g (156 mmol; 2,0 eq.) p-Nitrophenol und 93,8 g (1,56 mol; 20 eq.) n-Propanol versetzt und 42 h bei 65 °C gerührt. Der Ansatz wurde am HV. bei 50 °C eingeengt, zweimal mit je 250 ml Toluol versetzt und eingeengt. Der Rückstand wurde in 1 l CH₂Cl₂ aufgenommen, dreimal mit je 500 ml verdünnter NaHCO₃-Lsg. ausgerührt, die org. Phase mit Na₂SO₄ getrocknet und eingeengt: 92,5 g Rückstand. Chromatographie an Kieselgel 60 (50 x 10 cm) mit Gradienten (Methyl-tert. Butylether/iso-Hexan, 2:1 bis 4:1, dann Methyl-tert. Butylether/AcOEt, 1:4, dann AcOEt/MeOH, 1:1 bis 1:3) lieferte 44,7 g Produkt-haltige Fraktion, die aus 540 ml CH₂Cl₂/Methyl-tert. Butylether, 1:5, umkristallisiert wurde. Das Kristallisat wurde erneut aus 300 ml CH₂Cl₂/Methyl-tert. Butylether, 1:1, umkristallisiert.
**3:** DC (Kieselgel, CHCl₃/i-PrOH 49:1): R_{f} 0,14.
- Man erhielt:: 30,0 g N⁴-Benxoyl-1-{3'-O-benzoyl-4'-O-[(4,4'-dimethoxytriphenyl)-methyl]-β-D-ribo-pyranosyl}-cytosin 3
d. s. 51 % Ausbeute bezogen auf **2**. ¹H-NMR entspricht.

### Beispiel 3

### Synthese des N⁶-Benzoyl-9-{3'-O-benzoyl-4'-O-[(4,4'-dimethoxytriphenyl)-methyl]-β-D-ribopyranosyl}-adenins

Zuerst 2'-Substitution, dann 4'-Substitution, dann Wanderungsreaktion:

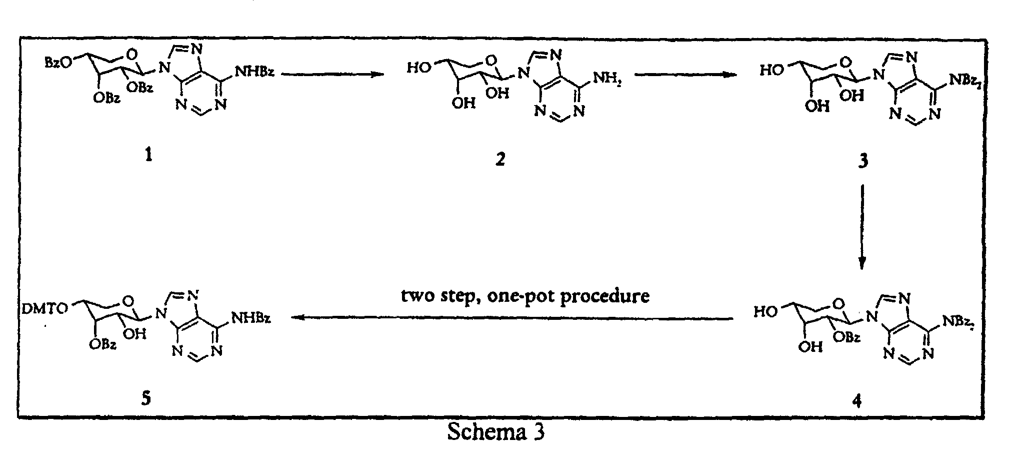

### 9-(β-D-Ribo-pyranosyl)-adenin 2:

68.37 g (100 mmol) N⁶-Benzoyl-9-(2',3',4'-tri-O-benzoyl-β-D-ribo-pyranosyl)-adenin **1** wurde in 300 ml NH₃-gesättigtem MeOH über Nacht bei RT. gerührt und das Kristallisat abfiltriert: 23,5 g (88 %) **2**.
DC (Kieselgel, AcOEt/MeOH 2:1): R_{f} 0,23.
^{**1**}**H-NMR** (300 MHz, DMSO): 3,56-3,78 (m, 3H, H(4'), H(5')); 4,04 (m, 1H, H(3')); 4.23 (ddd, J = 2,5, 8, 9,5 Hz, H(2')), 4,89 (d, J = 6 Hz, 1H, OH), 5,07 (d, J = 7 Hz, 1H, OH), 5,12 (d, J = 4 Hz, 1H, OH), 5,63 (d, J = 9,5 Hz, 1H, H(1')), 7,22 (s, 2H, NH2), 8,14 (s, 1H, H(2)), 8,29 (s, 1H, H(8)).
^{**13**}**C-NMR** (75 MHz, DMSO): 65,0 (t, C(5')); 66,6 (s, C(4')), 68,1 (s, C(3')), 71,1 (s, C(2')), 79,6 (s, C(1')); 118.6 (C(5)); 139,5 (s, C(8)), 149,9 (s, C(4)), 152,5 (s, C(2)), 155,8 (s, C(6)).

### N⁶,N⁶-Dibenzoyl-9-(β-D-ribo-pyranosyl)adenin 3:

Unter N₂-Atmosphäre wurden 16,8 g (62,9 mmol) **2** in 500 ml wasserfreiem Pyridin suspendiert und auf-4 bis - 10 °C gekühlt. Innerhalb von 20 min wurden 40 ml (199 mmol; 5 eq.) Trimethylchlorsilan zugetropft und 2,5 h unter Kühlung gerührt.
Bei -10 bis -15 °C wurden 36,5 ml (199 mmol; 5 eq.) Benzoylchlorid, gelöst in 73 ml Pyridin, innerhalb von 25 min zugesetzt, 10 min unter Kühlung und 2 h bei RT. gerührt (DC-Kontrolle (Kieselgel, AcOEt/Heptan 1:1): R_{f} 0,5). Man kühlte wieder auf -10 °C, ließ 136 ml H₂O (Temp. max. +8 °C) zulaufen und rührte über Nacht bei RT. Nach vollständigem Umsatz wurde das Lösungsmittel abgezogen und der Rückstand zweimal in je 200 ml Toluol aufgenommen und wieder eingedampft. Man versetzte mit je 500 ml Et₂O und H₂O, ließ mechanisch 2 h rühren, filtrierte das in beiden Phasen nur wenig lösliche Produkt ab, wusch mit Et₂O und H₂O und trocknete über P₂O₅ am HV.: 23,8 g (80 %) **3**.
DC (Kieselgel, AcOEt/MeOH 9:1): R_{f} 0,35.
^{**1**}**H-NMR** (300 MHz, DMSO): 3,60-3,80 (m, 3H, H(4'), H(5')); 4,06 (bs, 1H, H(3')); 4.30 (ddd. J = 2,5, 8, 9,5 Hz, H(2')), 4,93 (d, J = 6 Hz, 1H, OH), 5,20 (d. J = 4 Hz, 1H, OH), 5,25 (d, J = 4 Hz, 1H, OH), 5,77 (d, J = 9,5 Hz, 1H, H(1')), 7,47 (m, 4 H, Harom), 7,60 (m, 2H, Harom), 7,78 (m, 4H, Harom), 8,70 (s, 1H, H-C(2), 8,79 (s, 1H, H(8)).
^{**13**}**C-NMR** (75 MHz, DMSO): 66,2 (t, C(5')); 66,5 (s, C(4')), 68,0 (s, C(3')), 71,0 (s, C(2')), 80,4 (s, C(1')); 112.42 (C(5)); 126,9 (s, C(5)), 126,9, 128,9, 133,3, 133,4 (arom. C), 146,0 (s, C(8)), 150,7 (s, C(4)), 151,8 (s, C(2)), 153,3 (s, C(6)), 172,0 (s, C=O)).

### N⁶,N⁶-Dibenzoyl-9-(2'-O-benzoyl-β-D-ribo-pyranosyl}-adenin 4:

Unter N₂-Atmosphäre wurden 26,4 g (55,5 mmol) **3** in 550 ml wasserfreiem CH₂Cl₂ und 55 ml Pyridin (jeweils gelagert über Molsieb) gelöst, mit 0,73 g (5,55 mmol; 0,1 eq.) DMAP versetzt und auf-87 bis -90 °C abgekühlt. Innerhalb von 1 h wurden 8,58 g (61 mmol; 1,1 eq.) BzCl in 14 ml Pyridin zugetropft und über 60 h (Wochenende) bei -78 °C belassen. Der Ansatz wurde eingeengt, zweimal mit je 100 ml Toluol versetzt und eingedampft, um Pyridin zu entfernen. Chromatographie an Kieselgel 60 (20 x 10 cm) mit Gradienten (AcOEt/Heptan, 1:1 bis 9:1) lieferte 23,2 g **4**.
**4**: DC (Kieselgel, AcOEt): R_{f}0,34.
I-H-phosphonat

### N⁶-Benzoyl-9-{3'-O-benzoyl-4'-O-[(4,4'-dimethoxytriphenyl)-methyl] β-D-ribo-pyranosyl}-adenin 5:

23,2 g (40 mmol) 4 wurden in 160 ml wasserfreiem CH₂Cl₂ gelöst und in Folge mit 14,9 g (56 mmol; 1,1 eq.) DMTCI und 17,7 ml (104 mmol; 2,6 eq.) N-Ethyldiisopropylamin versetzt. Nach 2 h Rühren bei RT wurden weitere 4,0 g (11,8 mmol; 0,3 eq.) DMTCI zugefügt und weitere 40 min gerührt. Der Ansatz wurde bei 350-520 mbar und 35 °C am Rotavapor eingeengt.
DC (Kieselgel, AcOEt/Heptan 1:1): R_{f} 0,18.
Der Rückstand wurde in 260 ml wasserfreiem Pyridin gelöst und in Folge mit 51 ml (679 mmol; 17 eq.) n-Propanol, 16,6 ml (120 mmol; 3 eq.) Et₃N, 11,1 g (80 mmol; 2 eq.) p-Nitrophenol und 5,3 g (44 mmol; 1,1 eq.) DMAP versetzt und bei 60-63 °C 23 h lang gerührt. Dann blieb der Ansatz bei RT. 21 h stehen. Das Reaktionsgemisch wurde am Rotavapor eingeengt. Der Rückstand wurde zweimal mit je 200 ml Toluol versetzt und eingeengt, in CH₂Cl₂ gelöst und dreimal mit Wasser extrahiert.
Chromatographie an Kieselgel 60 (30 x 10 cm) mit Gradienten (AcOEt/Heptan, 1:2 bis 1:0; dann AcOEt/MeOH, 1:0 bis 9:1) lieferte 13 g **5**.
**5**: DC (Kieselgel, AcOEt/Heptan 4:1): R_{f} 0,2.
- Man erhielt:: 13 g N⁶-Benzoyl-9-{3'-O-benzoyl-4'-O-[(4,4'-dimethoxytriphenyl)methyl]-β-D-ribo-pyranosyl}-adenin 5
d. s. 30 % Ausbeute bezogen auf **3**. ¹H-NMR entspricht.
Zeitersparnis gegenüber literaturbekanntem Verfahren: 50 %.

### Beispiel 4

### Synthese von 9-[3'-O-Benzoyl-4'-O-((4,4'-dimethoxytriphenyl)-methyl)-β-D-ribopyranosyl]-2-O-allyl-2-N-isobutyroyl-guanin

Zuerst 3'-Substitution, dann 4'-Substitution:

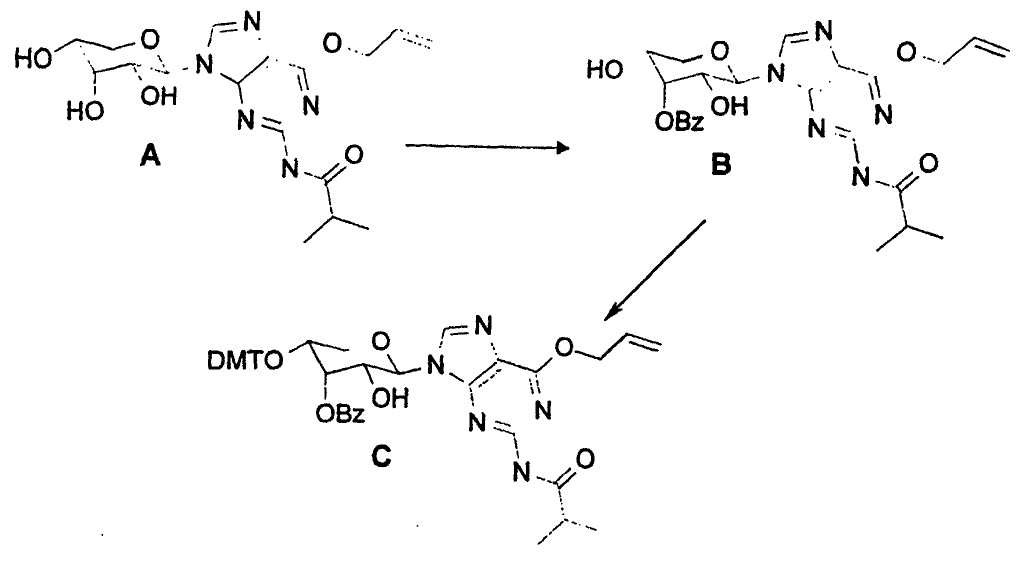

### 9-[3'-O-Benzoyl-β-D-ribopyranosyl]-2-O-allyl-2-N-isobutyroyl-guanin B

Das G-Triol A (393 mg, 1.0 mmol) wurde in 4 ml trockenem Dichlormethan gelöst. Man versetzte mit Benzoesäuretrimethylorthoester (0.52 ml, 3.0 mmol) und Camphersulfonsäure (58 mg, 0.25 mmol) und rührte 15 h bei Raumtemperatur. Dann wurde die Mischung auf 0 °C abgekühlt und mit 2 ml einer auf 0 °C vorgekühlten Mischung von Acetonitril, Wasser und Trifluoressigsäure (50:5:1) versetzt. Man rührte 10 min und entfernte das Lösungsmittel im Vakuum. Der Rückstand wurde durch Flashchromatographie an Kieselgel (2.3 x 21 cm) mit Dichlormethan/Methanol 100:3 gereinigt. Man erhielt 25 mg (5%) 4-O-Benzoylverbindung 139 mg (28%) Mischfraktionen und 205 mg (41 %) der gewünschten 3-O-Benzoylverbindung **B.**
¹H-NMR (300 MHz, CDCl₃): 1.12, 1.14 (2 d, J = 7.0 Hz, 2 x 3 H, NHCOCHMe₂), 2.78 (hep, J = 7 Hz, 1 H, NHCOCHMe₂), 3.85 (dd, J = 6.0,11.0 Hz, 1 H, H-5'_{eq}), 3.94 (app. T, J = 11.0 Hz, 1 H, H-5'ₐₓ), 4.12 (ddd, J = 2.5, 6.0, 11.0 Hz, 1 H, H-4'), 4.52 (dd, J=3.5,9.5 hz, 1 H, H-2'), 5.00 (dt, J = 1.5, 6.0 Hz, 2 H, All), 5.19 (dq, J = 1.5, 10.0 Hz, 1 H, All), 5.39 (dq, 1.5, 16.5 Hz, 1 H, All), 5.85 (bt, J = 3.0 Hz, 1 H, H-3'), 5.97 (d, J = 9.5 Hz, 1 H, H-1'), 6.07 (ddd, J = 6.0, 10.0, 16.5 Hz, 1 H, All), 7.40-7.58 (m, 3 H, Bz), 8.10-8.16 (m, 2 H, Bz), 8.28 (s, 1 H, H-8).

### 9-[3'-O-Benzoyl-4'-O-((4,4'-dimethoxytriphenyl)-methyl)-β-D-ribopyranosyl]-2-O-allyl-2-N-isobutyroyl-guanin C

Das Diol **B** (101 mg, 0.2 mmol) wurde in 3.2 ml trockenem Dichlormethan suspendiert. Man versetzte mit 171 µl (1.0 mmol) N-Ethyldiisopropylamin, 320 µl (3.96 mmol) Pyridin und 102 mg (0.3 mmol) DMTCI und rührte bei Raumtemperatur. Nach 24 h wurden weitere 102 mg (0.3 mmol) DMTCl zugegeben und nochmals 24 h gerührt. Dann wurde mit 30 ml Dichlormethan verdünnt. Die Lösung wurde mit 20 ml 10%iger wässriger Citronensäurelösung und 10 ml gesättigter Natriumbicarbonatlösung gewaschen, über MgSO₄ getrocknet und im Vakuum eingeengt. Der Rückstand wurde durch Flashchromatographie an Kieselgel (2.3 x 20 cm) mit Dichlormethan/Methanol 100:1 gereinigt. Man erhielt 39 mg des bekannten, gewünschten Produkts C (24%).

### Beispiel 5

### Synthese von p-RNA-Linkersystemen

Im folgenden werden drei Wege beschrieben, die die Bereitstellung von Linkern, die einen Amino-Terminus aufweisen, der dann zum Anlinken funktioneller Einheiten verwendet werden kann, ermöglichen:

### 5.1 Uracil-basierender Linker

### auf der Basis der Modifizierung der 5-Position des Uracils.

Die Herstellung von Hydroxyethyluracil **28** gelingt im großen Maßstab nach bekannter Methode (J.D. Fissekis, A. Myles, G.B. Brown, J. Org. Chem. **1964**, 29, 2670). g-Butyrolacton **25** wurde mit Ameisensäuremethylester formyliert, das Natriumsalz **26** zum Harnstoffderivat **27** umgesetzt und dieses zum Hydroxyethyluracil **28** cyclisiert (Schema 4).

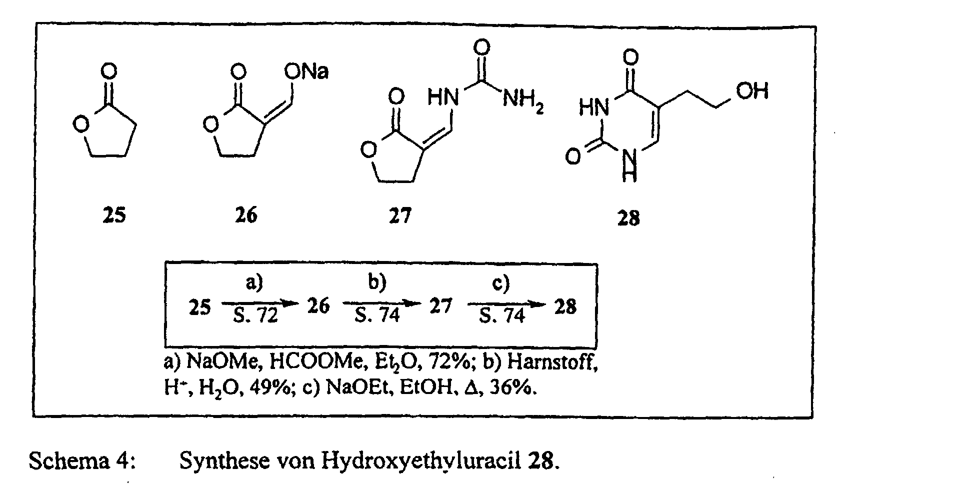

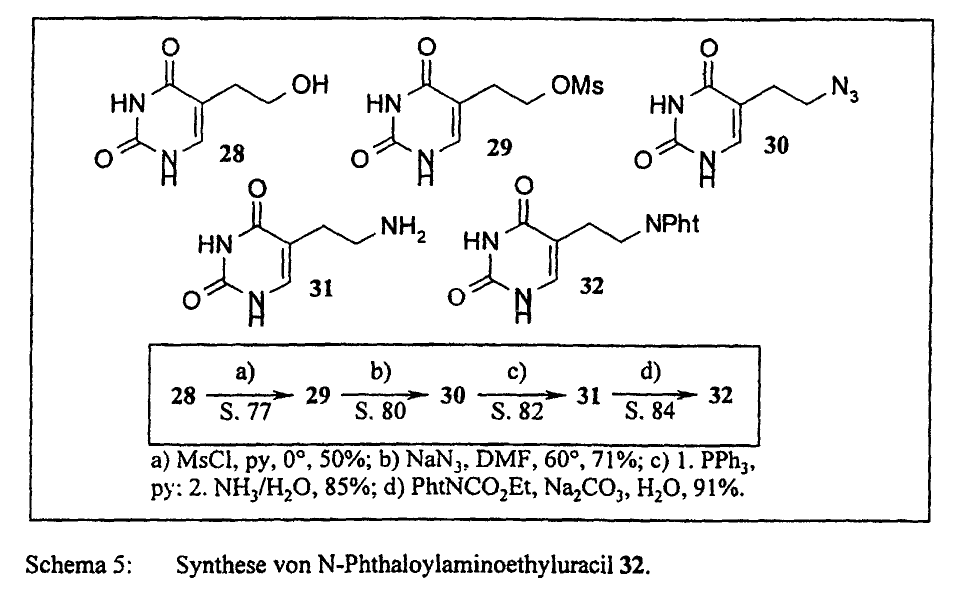

Hydroxyethyluracil **28** wurde mit Methansulfonsäurechlorid in Pyridin mesyliert zu **29** (J.D. Fissekis, F. Sweet, J. Org. Chem. **1973**, 38, 264).

Die folgenden Stufen wurden neu erfunden: Mit Natriumazid in DMF wurde **29** zum Azid **30** umgesetzt und dieses mit Triphenylphosphin in Pyridin zum Aminoethyluracil **31** reduziert. Die Aminofunktion in **31** wurde schließlich mit N-Ethoxycarbonylphtalimid geschützt (Schema 5). Nukleosidierung von Ribosetetrabenzoat **33** mit N-Phtaloylaminoethyluracil **32** lieferte das Ribosetribenzoat **34** in guten Ausbeuten. Das anomere Zentrum des Pyranoseringes ist, wie aus der Kopplungskonstanten zwischen H-C(1') und H-C(2') von J = 9.5 Hz klar ersichtlich, β-konfiguriert. Anschließende Abspaltung der Benzoatschutzgruppen mit NaOMe in MeOH lieferte das Linkertriol **35**. **35** wurde bei -78°C in Pyridin/Dichlormethan 1:10 in Gegenwart von DMAP mit Benzoylchlorid umgesetzt. Dabei erhielt man neben dem gewünschten 2'-Benzoat **36** (64%) auch 2',4'-dibenzoyliertes Produkt (22%), welches gesammelt und analog der Methanolyse von **34** nach **35** wieder in das Triol **35** umgewandelt wurde. Das 2'-Benzoat **36** wurde mit Dimethoxytritylchlorid in Gegenwart von Hünig-Base in Dichlormethan in 4'-Position in Ausbeuten größer 90% trityliert. Die Umlagerung von 4'-DMT-2'-benzoat **37** zum 4'-DMT-3'-benzoat **38** erfolgte in Gegenwart von DMAP, p-Nitrophenol und Hünig-Base in n-Propanol/Pyridin 5:2. Nach Chromatographie wird **38** erhalten. 4'-DMT-3'-benzoat **38** wurde abschließend mit ClP(OAll)N(iPr)₂ in Gegenwart von Hünig-Base zum Phosphoramidit **39** umgesetzt (Schema 6). Dieser läßt sich ohne Änderung der Syntheseprotokolle für die automatisierte Oligonucleotidsynthese einsetzen.

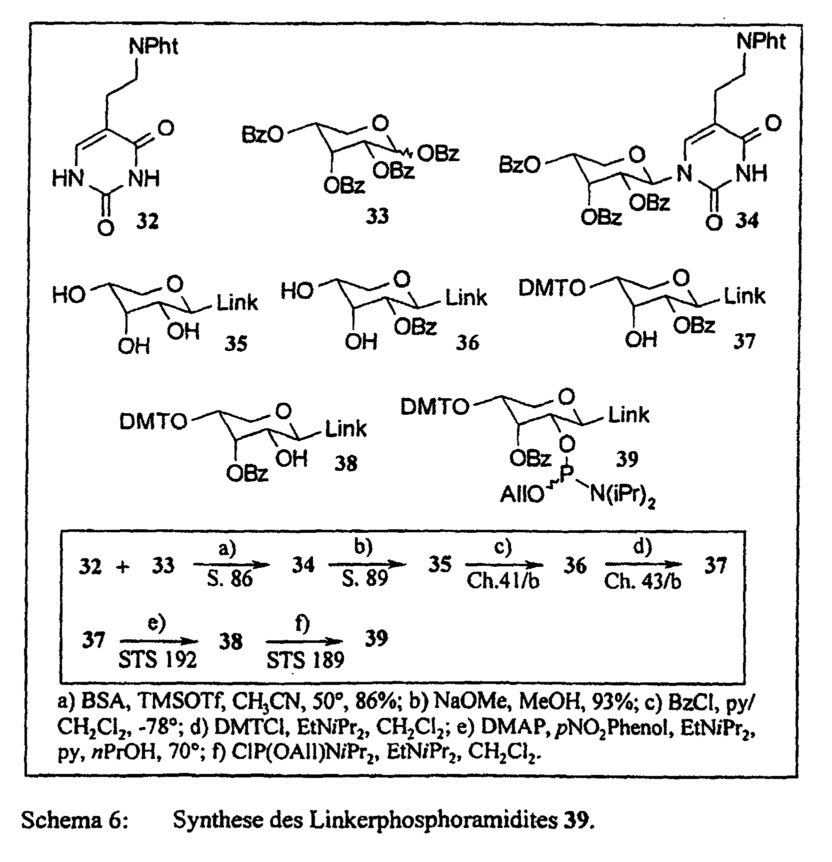

### Ausführung:

### Synthese eines Uracil-Linker-Bausteins

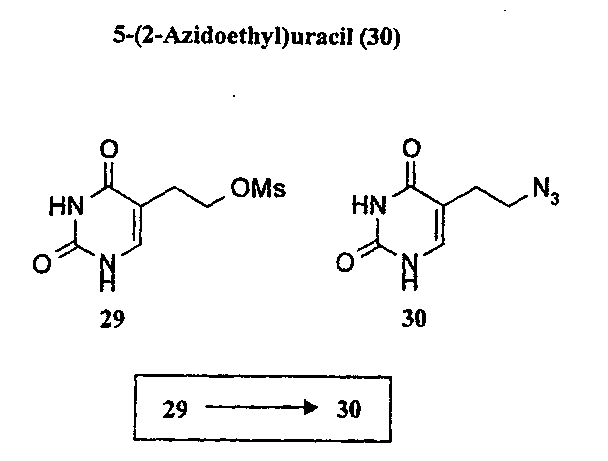

### 1. Durchführung

In einem mit Innenthermometer und Rückflußkühler ausgestatteten 500-ml-Dreihalskolben wurden 26.0 g (0.11 mol) **29** in 250 ml DMF gelöst und mit 10.8 g (0.166 mol) Natriumazid versetzt. Die Suspension wurde im Anschluß vier Stunden bei 60°C gerührt (DC-Kontrolle, CHCl₃/MeOH 9:1). Das DMF wurde abdestilliert und der Rückstand mit 150 ml Wasser verrührt. Der Feststoff wurde abfiltriert, mit ca. 50 ml Wasser gewaschen und über Nacht im Vakuum im Exsiccator über Phosphorpentoxid getrocknet. Man erhielt 14.2 g (71%) **30** in Form eines farblosen Feststoffes vom Schmp. 230-235°C (u. Zers.).

### 2. Analytische Daten

### 5-(2-Azidoethyl)uracil (30):

- **Schmp.:**: 230-235°C u. Zersetz..
- **DC:**: CHCl₃/MeOH 9:1, R_{f} 0.48.
- **UV** (MeOH):: λₘₐₓ 263.0(7910).
- **IR** (KBr):: 3209s, 3038s, 2139s, 1741s, 1671s, 1452m, 1245m, 1210m.
- ^{**1**}**H-NMR** (300 MHz, d₆-DMSO):: 2.46 (t, 2H, J(CH₂CH₂N, CH₂CH₂N) = 7.0, CH₂CH₂N); 3.40 (t, 2H, J(CH₂CH₂N, CH₂CH₂N) = 7.0, CH₂CH₂N); 7.36 (s, H-C(6)); 11.00 (br. s, 2H, H-N(1), H-N(3)).
- **MS** (ESI⁺):: 180.0 [M+H]..

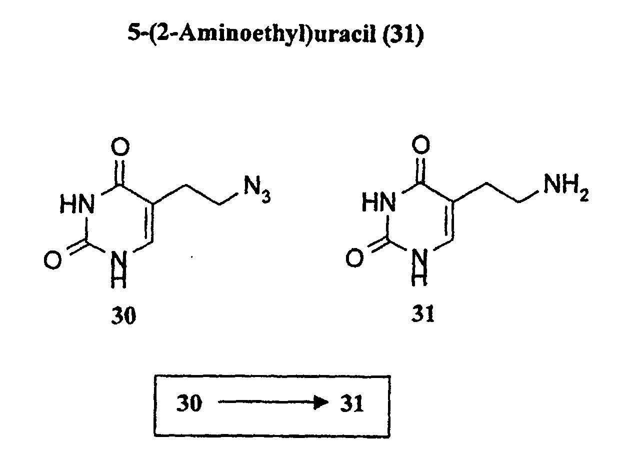

### 1. Durchführung

In einem mit Innenthermometer und Rückflußkühler ausgestatteten 250-ml-Dreihals-kolben wurden 14.2 g (78.0 mmol) **30** in 175 ml Pyridin suspendiert und mit 61.4 g (234 mmol) Triphenylphosphin versetzt²⁾. Es wurde fünf Stunden auf 60°C erwärmt und über Nacht bei Raumtemp. gerührt (DC-Kontrolle, CHCl₃/MeOH 5:1). Zur Suspension wurden 40 ml einer 25%igen Ammoniaklösung gegeben, die daraufhin aufklarte. Die Lösungsmittel wurden i.v.i.RV entfernt. Der Rückstand wurde in 200 ml CH₂Cl₂/MeOH 1:1 30 min bei Raumtemp. gerührt, der Niederschlag abfiltriert und mit CH₂Cl₂ gewaschen. Nach Trocknen im Vakuum im Exsiccator über Phosphorpentoxid erhielt man 10.0 g (85%) **31** vom Schmp. 214-220°C.

### 2. Analytische Daten

### 5-(2-Aminoethyl)uracil (31):

- **Schmp.:**: 214-220°C u. Gasentwicklung, vorher sintern.
- **DC:**: CHCl₃/MeOH/HOAc/H₂O 85:20:10:2, R_{f} 0.07.
- **UV** (MeOH):: λₘₐₓ 263.0 (6400).
- **IR**(KBr):: 3430m, 3109s, 1628s, 1474m, 1394s, 1270s, 1176w, 1103m, 1021m, 966m, 908m, 838m.
- ^{**1**}**H-NMR** (300 MHz, d₆-DMSO):: 2.21 (t, 2H, J(CH₂CH₂N, CH₂CH₂N) = 6.8, CH₂CH₂N); 2.59 (t, 2H, J(CH₂CH₂N, CH₂CH₂N) = 6.8, CH₂CH₂N); 5.90 (v. br. s, 4H, H-N(1), H-N(3), NH₂); 7.19 (s, H-C(6)).
- **MS** (ESI⁻):: 153.9 [M-H].

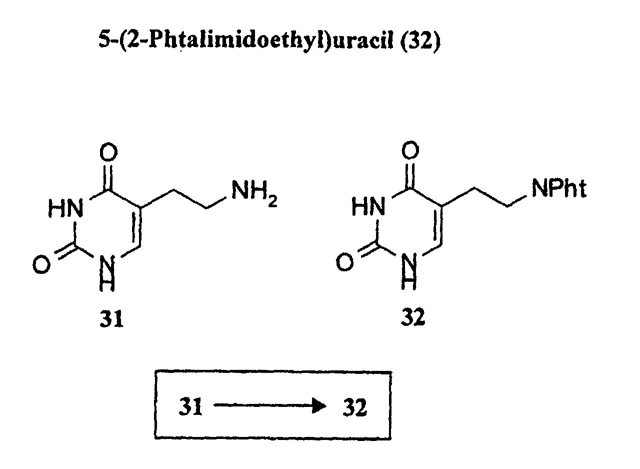

### 1. Durchführung

In einem 250-ml-Rundkolben wurden 9.6 g (61.8 mmol) **31** in 100 ml Wasser suspendiert und mit 6.64 g (62.6 mmol) Na₂CO₃ versetzt. Nach 15 min Rühren bei Raumtemp. gab man portionsweise 14.3 g (65 mmol) N-Ethoxycarbonylphtalimid zu und rührte drei Stunden bei Raumtemp. (DC-Kontrolle, CHCl₃/MeOH 5:1). Die nunmehr zähe, weiße Suspension wurde vorsichtig¹⁾ mit konz. Salzsäure auf pH 4 eingestellt und der weiße Niederschlag abfiltriert. Nach Waschen mit Wasser wurde der Feststoff im Vakuum im Exsiccator über Phosphorpentoxid getrocknet. Dies lieferte 16.0 g (91%) **32** vom Schmp. 324-327°C.

### 2. Analytische Daten

### 5-(2-Phtalimidoethyl)uracil (32):

- **Schmp.:**: 324-327°C u. Zersetz..
- **DC:**: CHCl₃/MeOH 5:1, R_{f} 0.51.
- **UV** (MeOH):: λₘₐₓ 263.0 (5825); λ 298.0 (sh., 1380).
- **IR** (KBr):: 3446m, 3216m, 1772m, 1721s, 1707s, 1670s, 1390m.
- ^{**1**}**H-NMR** (300 MHz, d₆-DMSO):: 2.49 (t, 2H, J(CH₂CH₂N, CH₂CH₂N) = 6.0, CH₂CH₂N); 3.71 (t, 2H, J(CH₂CH₂N, CH₂CH₂N) = 6.0, CH₂CH₂N); 7.24 (s, H-C(6)); 7.84 (m_{c}, 4H, NPht); 10.76 (br. s, H-N(1), H-N(3)).
- **MS** (ESI⁻):: 284.0 [M-H].

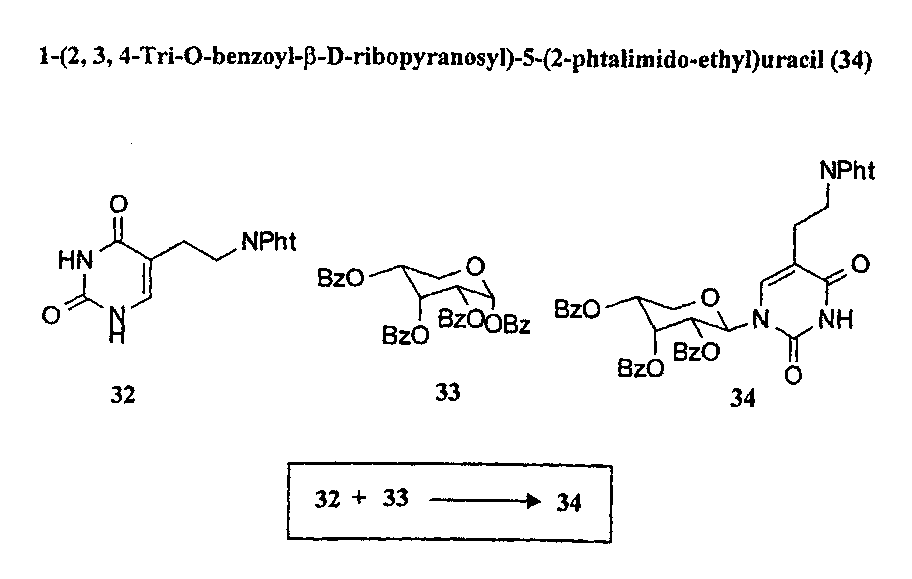

### 1. Durchführung

In einem 250-ml-Dreihalskolben, ausgestattet mit Argonüberleitung, Innenthermometer und Septum, wurden 7.00 g (24 mmol) **32** und 13.6 g (24 mmol) **33** in 120 ml Acetonitril suspendiert. Dann wurden mittels Spritze zunächst 12.2 ml (50 mmol) BSA zugegeben und nach 30 min Rühren nochmals 7 ml (28 mmol) BSA zugegeben. Nach kurzzeitigem Erwärmen auf 40°C klarte sich die Reaktionsmischung auf. Bei Raumtemp. wurden 13 ml (72 mmol) TMSOTf mittels Spritze zugegeben. Nach einer Stunde konnte noch keine Produktbildung beobachtet werden (DC-Kontrolle, AcOEt/n-Heptan 1:1). Daher wurden weitere 13 ml (72 mmol) TMSOTf zugegeben. Im Anschluß wurde der Reaktionsansatz auf 50°C erwärmt. Nach 2.5 h Rühren bei 50°C (DC-Kontrolle) wurde auf RT. gekühlt, auf eine eiskalte Mischung von 250 ml AcOEt und 190 ml ges. NaHCO₃-Lösung und 10 min intensiv ausgerührt. Man wusch nochmals mit 100 ml NaHCO₃-Lösung und extrahierte die wäßrigen Phasen nochmals mit 100 ml AcOEt. Die ver. org. Phasen wurden mit MgSO₄ getrocknet und die Lösungsmittel i.V.i.RV entfernt. Nach Trocknen im ÖPV erhielt man 20.9 g Rohprodukt. Chromatographie an Kieselgel (h = 25 cm, ϕ = 5 cm, AcOEt/n-Heptan 1:1) lieferte ein DCeinheitliches, schaumiges Produkt, das mit Et₂O digeriert wurde. Filtration und Trocknen im ÖPV ergab 15 g (86%) **34**.

### 2. Analytische Daten

### 1-(2, 3, 4-Tri-O-benzoyl-β-D-ribopyranosyl)-5-(2-phtalimidoethyl)uracil (34):

- **Schmp.:**: 124°C (sintern).
- **DC:**: AcOEt/n-Heptan 1:1, R_{f} 0.09.
- **UV** (MeOH):: λₘₐₓ 263.0 (11085); λ 299.0 (sh., 1530).
- **IR** (KBr):: 3238w, 3067w, 1772m, 1710s, 1452m, 1395m, 1266s, 1110s, 1070m, 1026m.
- ^{**1**}**H-NMR** (300 MHz, CDCl₃):: 2.79 (m_{c}, 2H. CH₂CH₂N); 3.96 (m_{c}, 2H, CH₂CH₂N); 4.06 (dd, J(H_{eq}-C(5'), Hₐₓ-C(5')) = 11.0, J(H_{eq}-C(5'), H-C(4')) = 6.0, H_{eq}-C(5')); 4.12 (t, J(Hₐₓ-C(5'), H_{eq}-C(5')) = J(Hₐₓ-C(5'), H-C(4')) = 11.0, Hₐₓ-C(5')); 5.39 (dd, J(H-C(2'), H-C(1')) = 9.5, J(H-C(2'), H-C(3')) = 2.9, H-C(2')); 5.46 (ddd, J(H- C(4'), Hₐₓ-C(5')) = 11.0, J(H-C(4'), H_{eq}-C(5')) = 6.0, J(H-C(4'), H-C(3')) = 2.9, H-C(4')); 6.26 (ψt, J ≈ 2.6, H-C(3')); 6.36 (d, J(H-C(1'), H-C(2')) = 9.5, H-C(1')); 7.24-7.40, 7.44-7.56, 7.61-7.66, 7.72-7.80, 7.84-7.90, 8.06-8.13 (6m, 16H, 3 Ph, H-C(6)); 7.70, 7.82 (2 m_{c}, 4H, NPht); 8.37 (s, H-N(3)).
- ^{**13**}**C-NMR** (75 MHz, CDCl₃):: 21.19 (CH₂CH₂N); 36.33 (CH₂CH₂N); 64.07 (C(5')); 66.81, 68.22 (C(4'), C(2')); 69.29 (C(3')); 78.59 (C(1')); 112.42 (C(5)); 123.31, 132.05, 133.89 (6C, Pht); 128.33, 128.47, 128.47, 128.83, 128.86, 129.31, 129.83, 129.83, 129.94, 133.55, 133.62, 133.69 (18C, 3 Ph); 135.87 (C(6)); 150.39 (C(2)); 162.78 (C(4)); 164.64, 165.01, 165.41 (3C, O₂CPh); 168.43 (2C, CO-Pht).
- **MS** (ESI⁺):: 730.2 [M+H].

| | | |
|---|---|---|
| **Anal.:** | ber. für C₄₀H₃₁N₃O₁₁ (729.70): | C 65.84, H 4.28, N 5.76; |
| | gef.: | C 65.63, H 4.46, N 5.53. |

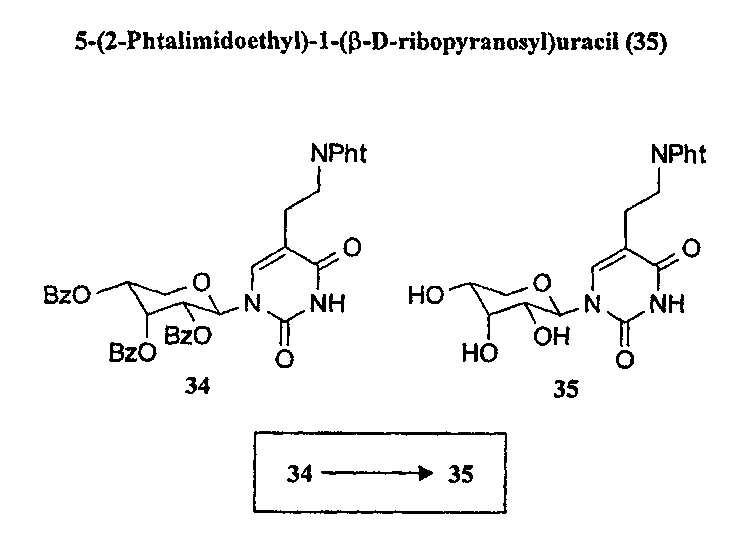

### 1. Durchführung

In einem 1-l-Rundkolben wurden 15 g (20 mmol) **34** in 500 ml MeOH gelöst, mit 324 mg (6 mmol) NaOMe versetzt und über Nacht unter Wasserausschluß bei Raum-temp. gerührt (DC-Kontrolle, AcOEt/n-Heptan 1:1). Es wurde solange Amberlite IR-120 zur entstandenen Suspension gegeben bis der pH-Wert <7 war. Es wurde der Feststoff in der Hitze gelöst, heiß vom Ionentauscher abfiltriert und mit MeOH gewaschen. Nach Entfernen der Lösungsmittel wurde der Rückstand zweimal mit je 150 ml Wasser coevaporiert. Dies lieferte 9 g Rohprodukt, das 10 min in 90 ml MeOH unter Rückfluß erhitzt wurde. Nach Abkühlen auf Raumtemp. versetzte man mit 60 ml Et₂O und bewahrte über Nacht bei 4°C auf. Filtration, Waschen mit Et₂O und Trocknen im ÖPV lieferte 7.8 g (93%) **35**.

### 2. Analytische Daten

### 5-(2-Phtalimidoethyl)-1-(β-D-ribopyranosyl)uracil (35):

- **Schmp.:**: 137°C (sintern).
- **DC:**: CHCl₃/MeOH 5:1, R_{f} 0.21.
- **UV** (MeOH):: λₘₐₓ 263.0 (8575); λ 299.0 (sh., 1545).
- **IR** (KBr):: 3458s, 1772w, 1706s, 1400m, 1364m, 1304m, 1045m.
- ^{**1**}**H-NMR** (300 MHz, d₆-DMSO + 2 Tr. D₂O):: 2.55 (m_{c}, 2H, CH₂CH₂N); 3.28-3.61 (m, 4H, H-C(2'), H-C(4'), H_{eq}-C(5'), Hₐₓ-C(5')); 3.73 (m_{c}, 2H, CH₂CH₂N); 3.93 (m, H-C(3')); 5.50 (d, J(H-C(1'), H-C(2')) = 9.3, H-C(1')); 7.41 (s, H-C(6)); 7.84 (s, 4H, NPht).
- ^{**13**}**C-NMR** (75 MHz, d₆-DMSO):: 25.63 (CH₂CH₂N); 36.62 (CH₂CH₂N); 64.95 (C(5')); 66.29 (C(4')); 67.37 (C(2')); 71.12 (C(3')); 79.34 (C(1')); 110.39 (C(5)); 122.85, 131.54, 134.08 (6C, Pht); 137.92 (C(6)); 150.84 (C(2)); 163.18 (C(4)); 167.74 (2C, CO-Pht).
- **MS**(ESI⁻):: 416.1 [M-H].

### 1-(2'-O-Benzoyl-β-D-ribopyranosyl)-5-(2-phtatimidoethyl)-uracil

In einem ausgeheizten und mit Argon begasten 1-l-Vierhalskolben wurden 10.6 g (0.025 mmol) 5-(2-Phtalimidoethyl)-1-(β-D-ribopyranosyl)uracil in 20 ml Pyridin gelöst und mit 200 ml Dichlormethan versetzt. Es wurde auf -70°C abgekühlt, unter Kühlung 3.82 ml (0.033 mmol) Benzoylchlorid in 5 ml Pyridin und 20 ml Dichlormethan langsam zugetropft und 35 min bei -70°C gerührt. Das Reaktionsgemisch wurde auf 600 ml gekühlte Ammoniumchlorid-Lösung gegossen und die wäßrige Phase mit Ethylacetat extrahiert. Die vereinigten organischen Phasen wurden mit Wasser gewaschen, getrocknet und im Vakuum zur Trockene eingeengt. Chromatographie über Kieselgel (Ethylacetat/Heptan 1:1) lieferte 7.9 g (60%) 1-(2'-O-Benzoyl-β-D-ribopyranosyl)-5-(2-phtalimidoethyl)-uracil.
DC: R_{f} 0.24 (Ethylacetat/Heptan 4:1).
¹H-NMR (300 Mhz, d₆-DMSO): 2.67 (m_{c}, 2H, CH₂CH₂N); 3.66-3.98 (m, 5H, H-C(4'), H_{eq}-C(5'), Hₐₓ-C(5'), CH2CH2N); 4.51 (t, 1H, H-C(3')); 4.98 (dd, 1H, H-C(2')); 6.12 (d, 1H, H-C(1')); 7.19 (s. 1H. H-C(6)); 7.29-7.92 (m, 9H, OBz, NPht).

### 1-(2-O-Benzoyl-4-O-(4,4'-dimethoxytrityl)-β-D-ribopyranosyl)-5-(2-phtalimidoethyl)uracil

5.6 g (10.73 mmol) 1-(2-O-Benzoyl-β-D-ribopyranosyl)-5-(2-phtalimidoethyl)uracil wurden in 60 ml Dichlormethan gelöst, mit 4.72 g (13.95 mmol) 4,4'-Dimethoxytritylchlorid und 2.4 ml (13.95 mmol) N-Ethyl-diisopropylamin versetzt und 20 min bei RT gerührt. Das Reaktionsgemisch wurde mit 100 ml Dichlormethan verdünnt, mit Natriumhydrogencarbonat-Lösung und 20% Zitronensäure-Lösung gewaschen, getrocknet und im Vakuum zur Trockene eingeengt. Chromatographie über Kieselgel (Ethylacetat/Heptan 1:1 + 2% Triethylamin) lieferte 7.7 g (87%) 1-(2-O-Benzoyl-4-O-(4,4'-dimethoxytrityl)-β-D-ribopyranosyl)-5-(2-phtalimidoethyl)uracil.
DC: R_{f} 0.53 (Ethylacetat/Heptan 1:1 + 2% Triethylamin).
¹H-NMR (300 MHz, CDCl₃): 2.64 (m_{c}, 2H, CH₂CH₂N); 3.12 (m_{c}, 1H, H-C(4')); 3.59-3.63 und 3.72-3.92 (m, 5H, H-C(3'), H_{eq}-C(5'), Hₐₓ-C(5'), CH₂CH₂N); 3.81 und 3.82 (s, 6H, CH₃O); 4.70 (dd, 1H, H-C(2')); 6.09 (d, 1H, H-C(1')); 7.05 (s, 1H, H-C(6)); 6.84-7.90 (m, 22H, ODmt, OBz, NPht).

### 1-(3-O-Benzoyl-4-O-(4,4'-dimethoxytrityl)-β-D-ribopyranosyl)-5-(2-phtalimidoethyl)uracil

3 g (3.63 mmol) 1-(2-O-Benzoyl-4-O-(4,4'-dimethoxytrityl)-β-D-ribopyranosyl)-5-(2-phtalimidoethyl)uracil, 1 g (7.26 mmol) 4-Nitrophenol, 0.44 g (3.63 mmol) 4-(Dimethylamino)-pyridin und 3.75 ml (21.78 mmol) N-Ethyl-diisopropylamin wurden in 5.6 ml iso-Propanol und 25 ml Pyridin gelöst, auf 65°C erhitzt und 3 Tage bei 65°C gerührt. Die Lösung wurde im Vakuum zur Trockene eingeengt und der Rückstand in 150 ml Dichlormethan gelöst. Nach Waschen mit 20% Zitronensäure-Lösung und Natriumhydrogencarbonat-Lösung wurde über Magnesiumsulfat getrocknet. Chromatographie über Kieselgel (Ethylacetat/Dichlormethan/iso-Hexan 2:1:1) lieferte 2.27 g (76%) 1-(3-O-Benzoyl-4-O-(4,4'-dimethoxytrityl)-β-D-ribopyranosyl)-5-(2-phtalimidoethyl)uracil.
DC: R_{f} 0.27 (Ethylacetat/iso-Hexan 2:1 + 1% Triethylamin).
¹H-NMR (300 MHz, CDCl₃): 2.39 (m_{c}, 2H, CH₂CH₂N); 2.53 (m_{c}, 1H, H_{eq}-C(5')); 3.30 (dd, 1H, H-C(2')); 3.55 (m_{c}, 1H, Hₐₓ-C(5')); 3.69 (m_{c}, 2H, CH₂CH₂N); 3.78 und 3.79 (s, 6H, CH₃O); 3.79-3.87 (m, 1H, H-C(4')); 5.74 (d, 1H, H-C(1')); 5.77 (m_{c}, 1H. H-C(3')); 6.92 (s, 1H, H-C(6)); 6.74-8.20 (m, 22H, ODmt, OBz, NPht).

### 1-{2'-O-[(Allyloxy)(diisopropylamino)phosphino]-3'O-benzoyl-4'-O-[(4,4'dimethoxytriphenyl)-methyl]-β-D-ribo-pyranosyl}-5-(2-phtalimidoethyl)- uracil

88 mg (0.11 mmol) 1-(3-O-Benzoyl-4-O-{4,4'-dimethoxytrityl)-β-D-ribopyranosyl)-5-(2-phtalimidoethyl)uracil wurden in 5 ml Dichlormethan gelöst, mit 75 µl (0.44 mmol) N-Ethyldiisopropylamin und 70 µl (0.3 mmol) Allyloxy-chlor-(diisopropylamino)phosphin versetzt und 3 h bei Raumtemperatur gerührt. Nach Zugabe von weiteren 35 µl (0.15 mmol) Allyloxychlor-(diisopropylamino)phosphin zur Vervollständigung der Reaktion wurde noch 1 h bei Raumtemperatur gerührt und das Reaktionsgemisch im Vakuum eingeengt. Chromatographie an Kieselgel (Ethylacetat/Heptan: Gradient 1:2 auf 1:1 auf 2:1, jeweils mit 2% Triethylamin) lieferte 85 mg (76%) 1-{2'-O-[(Allyloxy)(diisopropylamino)phosphino]-3'O-benzoyl-4'-O-[(4,4'-dimethoxytriphenyl)-methyl]- β-D-ribo-pyranosyl}-5-(2-phtalimidoethyl)- uracil.
DC: R_{f} 0.36 (Ethylacetat/Heptan 2:1).
¹H-NMR (CDCl₃, 300 MHz): Ausgewählte charakteristische Lagen: 2.28, 2.52 (2 dd, J = 5.0, 11.0 Hz, 2 H, 2 H-5'), 3.79, 3.78 (app. 2 s, 12 H, OMe), 6.14 (1 bs, 1 H, H-3').
³¹P-NMR(CDCl₃): 149.8, 150.6

### 5.2 Indol-basierender Linker

N-Phthaloyltryptamin wird wie beschrieben aus Phthalsäureanhydrid und Tryptamin erhalten (Kuehne et al J. Org. Chem. **43,** 13, **1978,** 2733-2735). Dieses wird mit Boran-THF zum Indolin reduziert (analog A. Giannis, et al., Angew. Chem. 1989, 101, 220).

Das 3-substituierte Indolin wird zuerst mit Ribose zum Nucleosidtriol und dann mit Essigsäureanhydrid zum Triacetat umgesetzt. Man oxidiert mit 2,3-Dichlor-5,6-dicyanoparachinon und spaltet die Acetate mit Natriummethylat, benzoyliert selektiv in 2'-Position, DM-trityliert selektiv in 4'-Position, und führt die Wanderungsreaktion zum 3'-Benzoat durch. Die Bildung des Phosphoramidits erfolgt wie üblich. Dieser läßt sich ohne Änderung der Syntheseprotokolle für die automatisierte Oligonucleotidsynthese einsetzen.

### Durchführung

### 3-(N-Phthaloyl-2-aminoethyl)-indolin

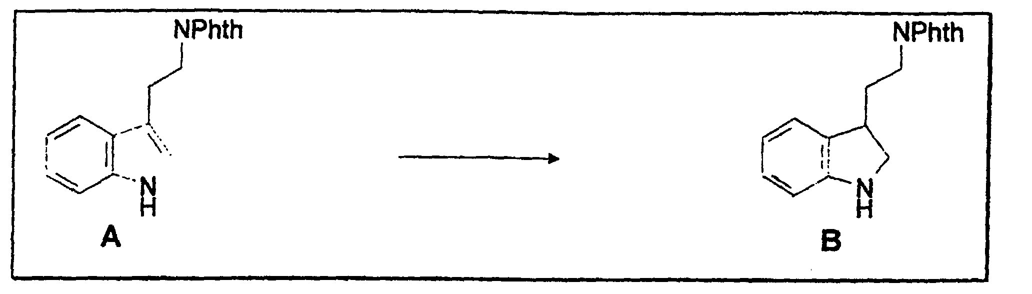

Unter Stickstoffatmosphäre wurden 51.4 g (177 mmol) Phthaloyltryptamin **A** in 354 ml 1M Boran-THF-Lösung (2 eq.) gelöst und auf 0 °C abgekühlt. Bei 0 °C wurde langsam 354 ml Trifluoressigsäure zugetropft (Vorsicht: Gasentwicklung) und 30 min. nachgerührt (DC-Kontrolle: EtOAc). Dann wurden 17.3 ml Wasser zugegeben, 10 min gerührt und im Vak. eingeengt. Der Rückstand wurde in 10%iger NaOH-Lösung / Dichlormethan gelöst, die organische Phase wurde abgetrennt, über NaSO₄ getrocknet, filtriert und im Vak. eingeengt. Der Rückstand (50.9 g) wurde aus heißem Ethanol (3 l) umkristallisiert. Man erhielt 41.4 g **B**, Smp. 161-162°C. Die Mutterlauge wurde im Vakuum eingeengt und der Rückstand erneut aus Ethanol umkristallisiert. Man erhielt weitere 3.2 g **B**, Smp. 158-159 °C.
Gesamtausbeute: 44.6 g (153 mmol) **B**, d. s. 86%.
¹H-NMR (CDCl₃, 300 MHz): 1.85-2.00, 2.14-2.28 (2 m, 2 x 1 H, CH₂CH₂NPhth), 2.70 (bs, 1 H, NH), 3.24-3.38, 3.66-3.86 (2 m, 5 H, CH₂CH₂NPhth, H-2a, H-2b, H-3), 6.62 (d, J = 8.0 Hz, 1 H, H-7), 6.66-6.72 (m, 1 H, H-5), 6.99 (app t, J = 7.5 Hz, 1 H, H-6), 7.14 (d, J = 8.0 Hz, 1 H, H-4), 7.64-7.74, 7.78-7.86 (2 m, 2 x 2 H, Phth).
¹³C-NMR (CDCl₃, 75 MHz): 32.70, 36.10 (2 t, C-2, CH₂CH₂NPhth), 39.62 (d, C-3), 53.04 (t, CH₂NPhth), 109.65 (d, C-7), 118.74 (d, C-5), 123.25 (d, Phth), 123.92, 127.72 (2 d, C-4, C-6), 131.81 (s, C-3a), 132.14 (s, Phth), 133.99 (d, Phth), 151.26 (s, C-7a), 168.38 (s, C=O).
Ber: C: 73.96, H: 5.52, N: 9.58; gef.: C: 73.89, H: 5.57, N: 9.55.
MS (ES⁺): 293 (MH⁺, 100%)

### 3-(N-Phthaloyl-2-aminoethyl)-1-(2',3',4'-tri-O-acetyl-β-D-ribo-pyranosyl)-indol

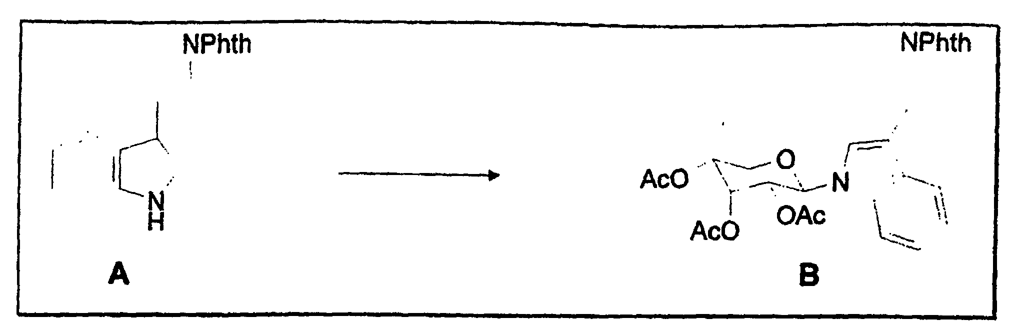

Unter Stickstoffatmosphäre wurden 45.2 g (155 mmol) **A** und 23.2 g (155 mmol; 1.0 eq.) D-Ribose in 750 ml trockenem Ethanol suspendiert und 4 h zum Rückfluß erhitzt (DC-Kontrolle: CH₂Cl₂/MeOH 10:1). Nach abkühlen auf RT wurde im Vak. eingeengt. Der Rückstand wurde in 300 ml Pyridin gelöst und unter Eiskühlung mit 155 ml Essigsäureanhydrid versetzt. Nach 15 min. wurde das Eisbad entfernt und 18 h bei RT gerührt (DC-Kontrolle: EtOAc/iso-Hexan 1:1). Diese Lösung wurde im Vakuum eingeengt und 3 mal mit je 300 ml Toluol coevaporiert. Das erhaltene Öl wird in 900 ml Dichlormethan gelöst und unter Eiskühlung mit 38.8 g (171 mmol; 1.1 eq.) 2,3-Dichlor-5,6-dicyanoparachinon versetzt. Nach 15 min. wurde das Eisbad entfernt und 1.5 h bei RT nachgerührt (DC-Kontrolle: EtOAc/iso-Hexan 1:1). Der ausgefallene Niedercshlag wurde abgesaugt und mit Dichlormethan gewaschen und verworfen. Das Filtrat wurde mit 600 ml ges. NaHCO₃-Lösung gewaschen. Der dabei ausgefallene Niederschlag wurde erneut abgesaugt und mit Dichlormethan gewaschen und verworfen. Die vereinigten organischen Extrakte wurden über NaSO₄ getrocknet und im Vak. eingeengt. Der Rückstand (90.9 g) wurde durch Flashchromatographie an Kieselgel 60 gereinigt (10 x 25 cm; EtOAc/iso-Hexan 2:3).
Man erhielt: 21.5 g reines **B** und 46.83 g Mischfraktionen, die nach erneuter Chromatographie weitere 20.4 g reines **B** lieferten.
Gesamtausbeute: 41.9 g (76 mmol) **B**, d. s. 49%.
¹H-NMR (CDCl₃, 300 MHz): 1.64, 1.98, 2.19 (3 s, 3 x 3 H, Ac), 3.06 (t, J = 8.0 Hz, 2 H, CH₂CH₂NPhth), 3.81-4.00 (m, 4 H, H-5'ax, H-5'eq, CH₂NPhth), 5.13 (ddd, J = 2.5, 6.0, 10.5 Hz, 1 H, H-4'), 5.36 (dd, J = 3.5, 9.5 Hz, 1 H, H-2'), 5.71 (d, J = 9.5 Hz, 1 H, H-1'), 5.74 (app t, J = 3.0 Hz, 1 H, H-3'), 7.02 (s, 1 H, H-2), 7.04-7.10, 7.13-7.19 (2 m, 2 x 1 H, H-5, H-6), 7.33 (d, J = 8.0 Hz, 1 H, H-7), 7.58-7.66, 7.72-7.80 (2 m, 5 H, Phth, H-4).
¹³C-NMR (CDCl₃, 75 MHz): 20.23, 20.65, 20.87 (3 q, Ac), 24.41, 38.28 (2 t, CH₂CH₂), 63.53 (t, C-5'), 66.24, 68.00, 68.64 (3 d, C-2', C-3', C-4'), 80.33 (d, C-1'), 109.79 (d, C-7), 113.95 (s, C-3), 119.33, 120.39, 122.04, 122.47 (4 d, C-4, C-5, C-6, C-7), 123.18 (d, Phth), 128.70, 132.17 (2 s, C-3a, Phth), 133.87 (d, Phth), 136.78 (s, C-7a), 168.24, 168.77, 169.44, 169.87 (4 s, C=O).
Ber: C: 63.50, H: 5.15, N: 5.11; gef.: C: 63.48, H: 5.16, N: 5.05.
MS (ES⁺): 566 (M+NH₄⁺, 82%), 549 (MH⁺, 74%), 114 (100%).

### 3-(N-Phthaleyl-2-aminoethyl)-1-β-D-ribo-pyranosyl-indol

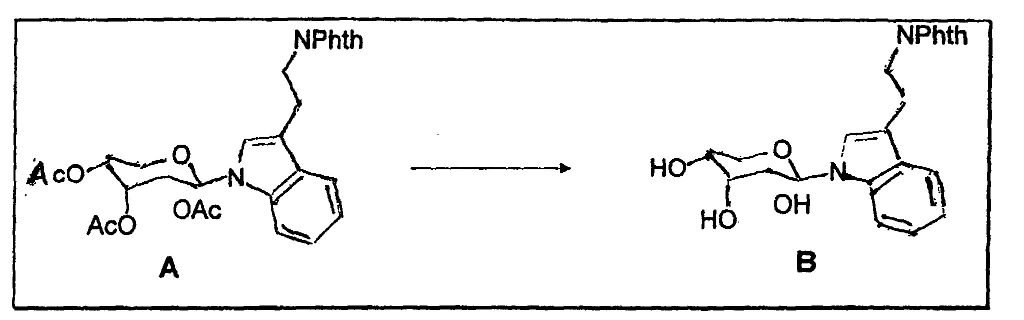

Unter Stickstoffatmosphäre wurden 44.1 g (80 mmol) **A** in 400 ml wasserfreiem Methanol gelöst. Unter Eiskühlung versetzte man mit 4.0 ml 30%iger Natriummethylatlösung und rührte dann 18 h bei RT. Der ausgefallene Niederschlag wurde abgesaugt und mit kaltem Ethanol gewaschen. Das Filtrat wurde im Vak. eingeengt. Der Rückstand wurde in Dichlormethan aufgenommen. Diese Lösung wurde mit ges. NaHCO₃-Lösung gewaschen, über NaSO₄ getrocknet und im Vak. eingeengt. Der erhaltene Rückstand wurde zusammen mit dem aus der Reaktionslösung ausgefallenen Niederschlag aus heißem Ethanol umkristallisiert. Man erhielt 22.6 g **B**, Smp. 196-198 °C. Die Mutterlauge wurde im Vakuum eingeengt und der Rückstand erneut aus Ethanol umkristallisiert. Man erhielt weitere 9.2 g **B**, Smp. 188-194°C.
Gesamtausbeute: 25.8 g **B**, d. s. 76%.

¹H-NMR (MeOD, 300 MHz): 3.09 (app. t, J = 7.0 Hz, 2 H, CH₂CH₂NPhth), 3.64-3.98 (m, 5 H, H-4', H-5'ax, H-5'eq, CH₂NPhth), 4.05 (dd, J=3.5, 9.5 Hz, 1 H, H-2'), 4.22 (app t, J = 3.0 Hz, 1 H, H-3'), 5.65 (d, J = 9.5 Hz, 1 H, H-1'), 6.95-7.05, 7.09-7.16 (2 m, 2 x 1 H, H-5, H-6), 7.25 (s, 1 H, H-2), 7.44 (d, J = 8.0 Hz, 1 H, H-7), 7.60 (d, J = 8.0 Hz, 1 H, H-4), 7.74-7.84 (m, 4 H, Phth).
¹³C-NMR (d₆-DMSO, 75 MHz): 23.87, 37.79 (2 t, CH₂CH₂NPhth), 64.82 (t, C-5'), 66.74 (d, C-4'), 68.41 (d, C-2'), 71.42 (d, C-3'), 81.37 (d, C-1'), 110.42 (d, C-7), 111.05 (s, C-3), 118.17, 119.21, 121.36, 122.92, 123.80 (5 d, C-2, C-4, C-5, C-6, NPhth), 127.86, 131.59 (2 s, C-3a, Phth), 134.27 (d, Phth), 136.62 (s, C-7a), 167.72 (s, C=O).
MS (ES⁻): 457 (M+OH⁻ +H₂O, 49%), 439 (M+OH⁻, 100%), 421 (M-H⁺, 28%)

### 1-(2'-O-Benzoyl-β-D-ribo-pyranosyl)-3-(N-phthaloyl-2-aminoethyl)-indol

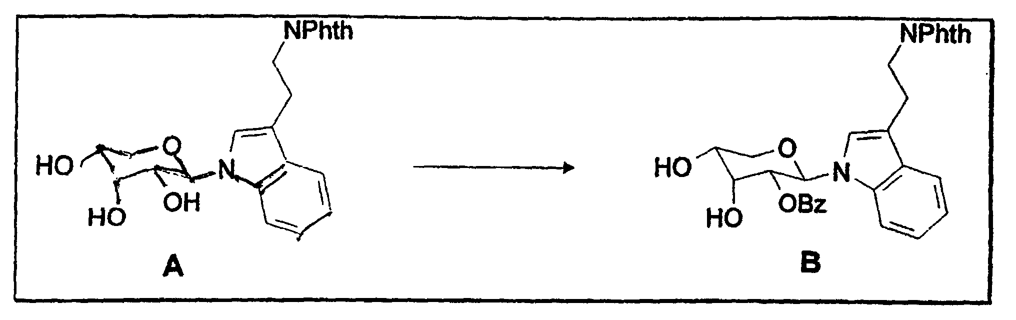

Unter Stickstoffatmoshäre wurde 10.6 g (25 mmol) **A** in 250 ml trockenem Dichlormethan aufgenommen. Man versetzt mit 305 mg DMAP (2.5 mmol) und 20 ml Pyridin. Es wurde erwärmt bis alles in Lösung war und anschließend auf -78 °C abgekühlt. Jetzt wurde 3.35 ml Benzoylchlorid (28.8 mmol) gelößt in 8 ml Dichlormethan innerhalb von 15 min zugetropft. DC-Kontrolle (EtOAc/Hexan 3:1) nach weiteren 30 min zeigte vollständige Reaktion an. Nach 45 min wurde die kalte Lösung über einen Faltenfilter direkt auf 200 ml ges. NH₄Cllösung gegeben und der Filterrückstand wurde mit Dichlormethan gewaschen. Die organische Phase wurde einmal mit Wasser gewaschen, über MgSO₄ getrochnet und eingeengt. Der Rückstand wurde 2 mal mit Toluol coevaporiert und durch Flashchromatographie an 10 x 20 cm Kieselgel mit EtOAc/Hexan 3:1 gereinigt. Man erhiehlt 8.1 g **B** (64%).
¹H-NMR (CDCl₃, 300 MHz): 2.45, 2.70 (2 bs, 2 x 1 H, OH), 3.04 (t, J = 8.0 Hz, 2 H, CH₂CH₂NPhth), 3.80-4.20 (m, 5 H, H-4', H-5'ax, H-5'eq, CH₂NPhth), 4.63 (bs, 1 H, H-3'), 5.46 (dd, J = 3.5, 9.5 Hz, 1 H, H-2'), 6.03 (d, J = 9.5 Hz, 1 H, H-1'), 7.08-7.31 (m, 5 H, H-2, H-5, H-6, Bz-m-H), 7.41-7.48 (m, 1 H, H-Bz-p-H), 7.50 (d, J = 8.0 Hz, 1 H, H-7), 7.64-7.79 (m, 7 H, Phth, H-4, Bz-o-H).
¹³C-NMR (d₆-DMSO, 75 MHz): 24.40, 38.22 (2 t, CH₂CH₂NPhth), 65.95 (t, C-5'), 66.65 (d, C-4'), 69.55 (d, C-3'), 71.87 (d, C-2'), 79.57 (d, C-1'), 109.96 (d, C-7), 113.70 (s, C-3), 119.21, 120.21, 122.11, 122.41, 123.14 (5 d, C-2, C-4, C-5, C-6, NPhth), 128.28 (d, Bz), 128.58, 128.59 (2 s, C-3a, Bz), 129.62 (d, Phth), 132.05 (s, Phth), 133.81 (Bz), 136.97 (s, C-7a), 165.12,168.29 (2 s, C=O).
MS (ES⁻): 525 (M-H⁺, 12%), 421 (M-PhCO⁺, 23%), 107 (100%).

### 1-{3'-O-Benzoyl-4'O-[(4,4'-dimethoxytriphenyl)-methyl]-β-D-ribo-pyranosyl}-3-(Nphthaloyl-2-aminoethyl)-indol

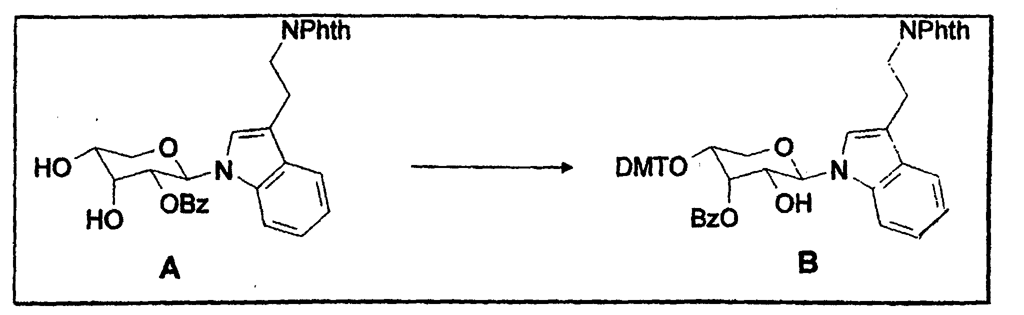

Unter Stickstoffatmoshäre wurde 8.9 g (16.9 mmol) **A** in 135 ml trockenem Dichlormethan suspendiert. Man versetzte mit 206 mg DMAP (1.68 mmol), 5.8 ml N-Ethyldiisopropylamin (33.7 mmol) und ca. 12 ml Pyridin (bis zur vollständigen Lösung). Jetzt wurde mit 34 g Molsieb 4Å versetzt und 30 min gerührt. Nach Abkühlen auf 0 °C wurde mit 11.4 g DMTCl (33.7 mmol) versetzt und nach Entfernen des Kühlbads 75 min gerührt Dann wurden nochmals 1.94 g (0.34 eq) und nach weiteren 40 min 1.14 g (0.2 eq) und nach weiteren 65 min 1.14 g DMTCl (0.2 eq) zugegeben. Nach 4.25 h war die Reaktion beendet. Man versetzte dann mit 25.3 ml n-Propanol (20 eq), rührte noch 30 min nach und engte dann vorsichtig ein (Schaumbildung). Der Rückstand wurde in 100 ml Pyridin gelößt. Man versetzte mit 1.85 g DMAP (15.1 mmol; 0.9 eq), 13.05 ml N-Ethyldiisopropylamin (101 mmol; 6.0 eq), 71 ml n-Propanol (940 mmol; 56 eq) und 3.74 g p-Nitrophenol (26.9 mmol; 1.6 eq). Diese Mischung wurde unter Stickstoff 96 h bei 75-80 °C gerührt. Nach Abkühlen auf Raumtemperatur wurde die Mischung über Celite filtiert und eingeengt. Der Rückstand wurde durch Flashchromatographie an 9 x 17 cm Kieselgel mit Toluol/Diethylether/Triethylamin 90:10:1 gereinigt. Die produkthaltuigen Fraktionen (9.25 g) wurden zunächst aus EtOAc umkristallisiert und anschließend aus Toluol/Methanol umgefällt. Man erhielt 5.86 g **B** (42%).

¹H-NMR (CDCl₃, 300 MHz): 2.64 (bs, 1 H, OH), 2.68 (dd, J = 5.0, 11.5 Hz, 1 H, H-5'eq), 2.94 (dd, J = 7.5, 16.0 Hz, 1 H, CH₂CH₂NPhth), 3.03 (dd, J = 8.0, 16.0 Hz, 1 H, CH₂CH₂NPhth), 3.67-3.74 (m, 1 H, H-5'ax), 3.69, 3.70 (2 s, 2 x 3 H, OMe), 3.85 (t, J = 7.5 Hz, 2 H, CH₂CH₂NPhth), 3.94 (ddd, J = 3.0, 5.0, 10.5 Hz, 1 H, H-4'), 4.03 (dd, J = 3.5, 9.0 Hz, 1 H, H-2'), 5.51 (d, J = 9.0 Hz, 1 H, H-1'), 5.86 (bs, 1 H, H-3'), 6.68-7.66 (m, 25 H), 8.19-8.30 (m, 2H).
¹³C-NMR (CDCl₃, 75 MHz): 24.16, 38.80 (2 t, CH₂CH₂NPhth), 55.25, 55.26 (2 q, Ome), 65.58 (t, C-5'), 68.29, 69.19, 73,83 (3 d, C-2', C-3', C-4'), 83.03 (d, C-1'), 87.31 (CAr₃)110.03 (d, C-7), 113.37, 113.47 (2 d), 113.53 (s, C-3), 118.95, 120.20, 122.28, 122.31, 123.10, 127.07, 128.02, 128.08, 128.68 (9 d), 128.74 (s), 130.02, 130.19, 130.22 (3 d), 130.37, 131.95 (2 s), 133.40, 133.83 (2 d), 135.98, 136.14, 136.56, 145.12, 158.82, 166.76, 168.52 (7 s, C-7a, 2 COMe, 2 C=O).

### 1-{2'O-(Allyloxy)(diisopropylamino)phosphino)-3'-O-Benzoyl-4'O-[(4,4'dimethoxytriphenyl)-methyl]-β-D-ribo-pyranosyl}-3-(N-phthaloyl-2-aminoethyl)-indol (2 Diastereomere)

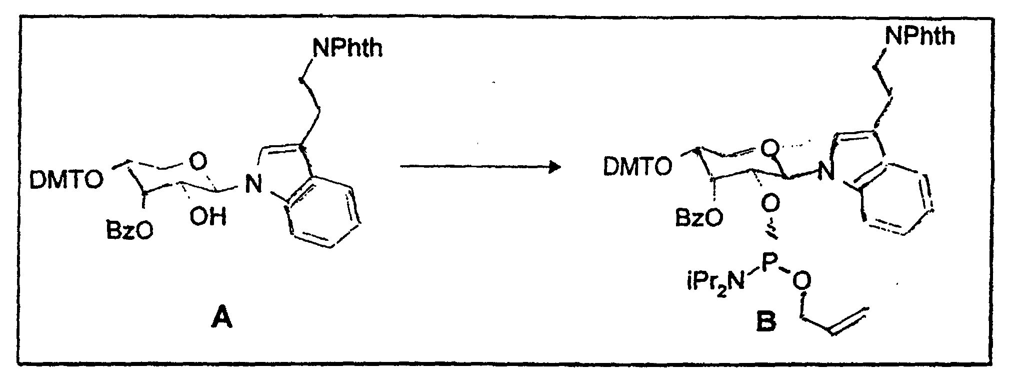

Unter Argonatmosphäre wurde 1658 mg Alkohol **A** (2.0 mmol) in 10 ml trockenem Dichlormethan gelößt. Man versetzte mit 1.03 ml N-Ethyldiisopropylamin (6.0 mmol) und 0.63 ml Phosphorigsäuremonoallylesterdiisopropylamidchlorid (2.8 mmol) und rührte 1 h bei Raumtemperatur. Dann wurde das überschüssige Phosphorylierungsreagenz durch Zugabe von 61 µl (0.8 mmol) Isopropanol zerstört. Nach 10 min wurde im Vakuum eingeengt und der Rückstand durch Flashchromatographie an 3.3 x 21 cm Kieselgel mit Hexan/EtOAc/NEt₃ (75:25:1) gereinigt. Die produkthaltigen Fraktionen wurden eingeengt, in CCl₄ aufgenommen und wieder eingeengt. Man erhielt 2.04 g eines fast farblosen Schaums (quant.), der so direkt zur Oligomerisierung verwendet werden kann und bei -20 °C mehrere Wochen haltbar ist. DC auf Kieselgel (EtOAc/Hexan/NEt₃ 33:66:1): 0.41
¹H-NMR (CDCl₃, 300 MHz): Ausgewählte charakteristische Lagen: 2.42, 2.53 (2 dd, J = 5.0, 11.0 Hz, 2 H, 2 H-5'eq), 3.76, 3.77, 3.78, 3.79 (4 s, 4 x 3 H, OMe), 5.70, 5.73 (2 d, J = 9.0 Hz, 2 H, 2 H-1'), 6.16, 6.29 (2 bs, 2 H, 2 H-3').
³¹P-NMR (CDCl₃): 150.6, 151.0

### 5.3 Lysin-basierender Linker

Die Synthese ist im Schema **7** abgebildet und wird im folgenden im Detail beschrieben.

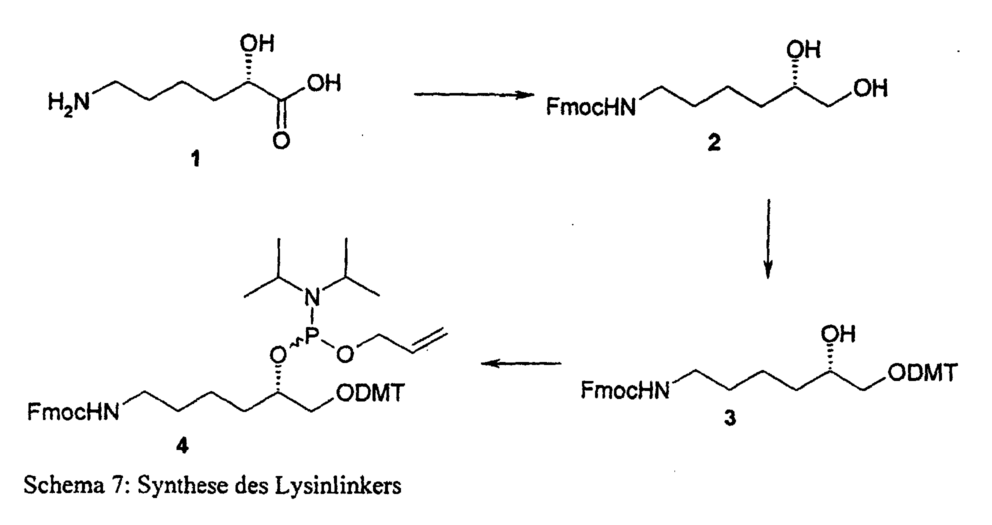

6-Amino-2(S)-hydroxyhexansäure (**1**) wurde nach literaturbekannter Weise durch Diazotierung und anschließebnde Hydrolyse aus L-Lysin hergestellt (K.-I. Aketa, Chem. Pharm Bull. 1976, 24, 621).

### 2-(S)-N-Fmoc-6-amino-1,2-hexandiol (2)

3.4 g LiBH₄ (156 mmol, 4 eq) werden unter Argon in 100 ml abs. THF gelöst (exotherm!). Nach Abkühlen auf ca. 30 °C werden 39.6 ml TMSCI (312 mmol, 8 eq) langsam zugetropft (Gasentwicklung!), wobei sich ein Niederschlag bildet. Anschließend werden im Argon-Gegenstrom 5.74 g 6-Amino-2(S)-hydroxyhexansäure (**1**) (39 mmol) portionsweise zugegeben und es wird auf 65 °C erwärmt, bis das DC (Kieselgel; i-PrOH/konz. NH₄OH/Wasser 7:2:1; Anfärben mit Ninhydrin) kein Edukt mehr zeigt (ca. 3 h). Unter Eiskühlung wird vorsichtig mit 120 ml Methanol versetzt (starke Gasentwicklung!). Das Lösungsmittel wird im Vakuum eingeengt, der Rückstand wird 3 mal mit je 200 ml Methanol coevaporiert und anschließend in 100 ml abs. DMF gelöst. Nach Zugabe von 16 ml Ethyldiisopropylamin (93.6 mmol, 2.4 eq) wird die Mischung auf 0°C gekühlt und portionsweise mit 12.1 g FmocCl (46.8 mmol, 1.2 eq) versetzt. Nach 15 Minuten wird das Kühlbad entfernt und bei Raumtemperatur gerührt bis das Edukt verbraucht ist (ca. 3 h; DC-Kontrolle: Kieselgel; CHCl₃/MeOH/HOAc/Wasser 60:30:3:5). Die Reaktionslösung wird auf 600 ml ges. NaHCO₃-Lösung gegeben. Der Niederschlag wird abfiltriert, mit 200 ml Wasser gewaschen und bei 50 °C am HV getrocknet bis zur Gewichtskonstanz (ca. 6 h). Man erhält 13.9 g eines farblosen Feststoffs, der aus Ethylacetat (40 ml)/n-Hexan (35 ml) umkristallisiert wird. Ausbeute: 9.05 g (65%).

¹H-NMR (300 MHz, CDCl₃): 7.68, 7.51 (2 d, J = 8.0 Hz, je 2 H, Ar-H), 7.32 (t, J = 8.0 Hz, 2 H, Ar-H), 7.23 (dt, J = 1.6, 8.0 Hz, 2 H, Ar-H), 4.92 (bs, 1 H. NH), 4.32 (d, J = 7.0 Hz, 2 H, OCOCH₂), 4.13 (bt, J = 7.0 Hz, 1 H, OCOCH₂CH), 3.64-3.58 (m, 1 H, H-1, H-1', H-2, H-6, H-6'), 3.54 (dd, J = 3.2, 11.0 Hz, 1 H, H-1, H-1', H-2, H-6, H-6'), 3.35 (dd, J = 7.4, 11.0 Hz, 1 H, H-1, H-1', H-2, H-6, H-6'), 3.16-3.06 (m, 2 H, H-1, H-1', H-2, H-6, H-6'), 3.0-2.0 (bs, 2 H, OH), 1.52-1.18 (m, 6 H, H-3, H-3', H-4, H-4', H-5, H-5').

2-(S)-N-Fmoc-O¹-DMT-6-amino-1,2-hexandiol (**3**) wurde nach WO 89/02439 DM-trityliert.

2-(S)-N-Fmoc-O¹-DMT-O²-allyloxydiisopropylaminophosphinyl-6-amino-1,2-hexandiol (**4**)

Zu einer Lösung von 670 mg des Alkohols (**3**) (1.02 mmol) in 10 ml abs. Dichlormethan werden unter Argon 0.51 ml Ethyldiisopropylamin (3.0 mmol, 3 eq) und 0.33 ml Chlor-N,N-diisopropylaminoallyloxyphosphin ( 1.5 mmol, 1.5 eq) gegeben. Man rührt 2 h bei Raumtemperatur, zieht das Lösungsmittel im Vakuum ab und reinigt den erhaltenen Rückstand durch Flashchromatographie an 3.2 x 16 cm Kieselgel (EtOAc/iso-Hexan/NEt₃ 20:80:1). Man erhält 839 mg (97%) eines leicht gelblichen Öls.

DC: Kieselgel; EtOAc/iso-Hexan/NEt₃ 50:50:1; UV; R_{f}= 0.77.

¹H-NMR (300 MHz, CDCl₃): 7.70-6.68 (m, 21 H, Ar-H), 4.92-4.62 (m, 1 H. NH), 4.31 (d, J = 7.0 Hz, 2 H, OCOCH₂), 4.13 (t, J = 7.0 Hz, 1 H, OCOCH₂CH), 3.98-3.40 (m, 5 H), 3.77 (2 s, je 3 H, OMe), 3.16-2.86 (m, 4 H), 2.58 (t, J = 7.0 Hz, 1 H, CHCN), 2.38 (t, 1 H, CHCN), 1.80-1.20 (m, 6 H), 1.20, 1,18, 1.17, 1.16, 1.15, 1.13, 1.08, 1,06 (8 s, 12 H, NMe).

³¹P-NMR (300 MHz, CDCl₃): 149.5, 149.0 (2 s)

### Beispiel 6

### Synthese eines p-RNA-Oligos der Sequenz 4'-Indollinker-A8-2' unter Verwendung von Benzimidazoliumtriflat als Kupplungsreagenz

108 mg Indollinker-Phosphoramidit und 244 mg A-Phosphoramidit werden in Synthesizergläschen eingewogen und für 3 h im Exsikkator über KOH zusammen mit dem mit 28,1 mg CPG-Träger, beladen mit A-Baustein, gefüllten Säulchen am HV belassen. Die Phosphoramidite werden in 1 ml (Indollinker) bzw. 2,5 ml (A-Phosphoramidit) Acetonitril gelöst und einige Kügelchen von dem Molsieb zugesetzt und über KOH geschlossen im Exsikkator belassen.
200 mg Jod werden unter starkem Rühren in 50 ml Acetonitril gelöst. Nachdem alles gelöst ist (Sichtkontrolle) werden 23 ml Wasser und 4,6 ml sym-Collidin zugegeben und die Lösung einmal gut durchmischt. Zum Detritylieren wird eine 6 %ige Lösung von Dichloressigsäure in Dichlormethan eingesetzt. Das Cappingreagenz (Acetanhydrid + Base) wird wie für Oligonucleotidsynthese üblich gekauft und verwendet.
Benzimidazoliumtriflat wird aus heißem Acetonitril umkristallisiert und getrocknet. Mit den fast farblosen Kristallen wird eine 0,1 M Lösung in wasserfreiem Acetonitril als Kupplungsreagenz hergestellt. Während der Synthese bleibt diese Lösung immer klar und es kommt zu keinen Verstopfungen der Synthesizerschläuche.

| Abgeänderter DNA-Kupplungscyclus am Eppendorf Ecosyn 300+ (DMT-on): | |
|---|---|
| Detritylierung 7 | Minuten |
| Kupplung | 1 Stunde |
| Capping | 1,5 Minuten |
| Oxidation | 1 Minute |

20 mg Tetrakis(triphenylphosphin)palladium wird in 1,5 ml Dichlormethan gelöst, 20 mg Diethylammoniumhydrogencarbonat, 20 mg Triphenylphosphin und der das Oligonucleotid tragende Glasträger zugesetzt, dicht verschlossen (Parafilm) und das Gläschen 5 h bei RT. bewegt. Dann wird der Glasträger über eine Analysennutsche abgesaugt, und mit Dichlormethan, mit Aceton und mit Wasser gewaschen.

Der Träger wird mit wäßriger 0,1 molarer Natriumdiethyldithiocarbamatlösung aufgeschlämmt und 45 min bei RT. belassen. Man saugt ab, wäscht mit Wasser, Aceton, Ethanol und Dichlormethan. Der Träger wird in 1,5 ml 24 %iger Hydrazinhydratlösung suspendiert, 24-36 h lang bei 4 °C gerüttelt und mit 0,1 molarem Triethylammoniumhydrogencarbonatpuffer (TEAB-Puffer) auf 7 ml verdünnt. Über eine Waters Sep-Pak Cartridge wurde hydrazinfrei gewaschen. Es wird mit 5 ml einer 80%igen Ameisensäurelösung versetzt, und nach 30 min zur Trockene einrotiert. Der Rückstand wird in 10 ml Wasser aufgenommen, mit Dichlormethan extrahiert, die wäßrige Phase eingeengt und nun HPL-chromatographiert (tR = 33 min, Gradient von Acetonitril in 0,1M Triethylammoniumacetat-Puffer). Übliche Entsalzung (Waters Sep-Pak Cartridge) liefert das Oligonucleotid.
Ausbeute: 17, 6 OD.
Substanzidentität nachgewiesen durch ESI-Massenspektroskopie:
M(ber) = 3082 D, (M+2H)²⁺(gef) = 1541,9 D.

### Beispiel 7

### Herstellung von Konjugaten

### 1. Sequentielles Verfahren

Zuerst wird, wie in Beispiel 2 beschrieben, ein p-RNA-Oligomeres der Sequenz A₈, d. h. ein Octamer, auf dem Eppendorf Ecosyn D 300+ hergestellt und dann die folgenden Reagenzien ausgetauscht: 6 %ige Dichloressigsäure gegen 2 %ige Trichloressigsäure, Jod in Collidin gegen Jod in Pyridin, Benzimidazoliumtriflatlösung gegen Tetrazollösung. Nach Änderung des Syntheseprogramms wird ein DNA-Oligomeres der Sequenz GATTC nach bekannten Methoden (M. J. Gait, Oligonucleotide Synthesis, IRL Press, Oxford, UK 1984) weiter synthetisiert. Die Deallylierung, Hydrazinolyse, HPL-Chromatographie und Entsalzung erfolgt wie für das p-RNA-Oligomere beschrieben (siehe oben) und liefert das gewünschte Konjugat.

### 2. Konvergentes Verfahren

Wie in Beispiel 2 beschrieben, wird ein p-RNA-Oligomeres mit der Sequenz 4'-Indollinker-A₈-2' hergestellt, aufgereinigt, und jodacetyliert. Ein DNA-Oligomeres der Sequenz GATTC-Thiol-Linker wird nach bekannten Methoden (M. J. Gait, Oligonucleotide Synthesis, IRL Press, Oxford, UK 1984) synthetisiert und aufgereinigt (3'-Thiol-Linker von Glen Research: Nr. 20-2933). Beim Stehenlassen der beiden Fragmente (T. Zhu et al., Bioconjug. Chem. 1994, 5, 312) in gepufferter Lösung entsteht das Konjugat, das abschließend über HPLC aufgereinigt wird.

### Beispiel 8

### Konjugation eines Biotinrestes an eine aminomodifizierte p-RNA:

Zuert wurde analog wie in Beispiel 6 beschrieben, ein p-RNA Oligomer der Sequenz TAGGCAAT, welches am 4'-Ende mittels des 5'-Aminomodifier 5 von Eurogentec (2-(2-(4-Monomethoxytrityl)aminoethoxy)ethyl-(2-cyanoethyl)-(N,N-diisopropyl)-phosphor-amidit) mit einer Aminogruppe versehen ist, synthetisiert und aufgearbeitet. Das Oligonucleotid (17,4 OD, 0,175 µmol) wurde in 0,5 ml basischem Puffer aufgenommen, 1,14 mg (2,5 µmol) Biotin-N-hydroxysuccinimidester in 114 µl DMF (abs.) gelöst und 1h bei RT stehengelassen. Das entstandene Konjugat wurde mittels präparativer HPLC gereinigt und das reine Produkt mit einer Sepak entsalzt.

Ausbeute: 8.6 OD (49%)
M (ber.) = 3080 D, M (fef) = 3080,4 D

### Beispiel 9

### Herstellung Cyanin- bzw. Biotin-markierter p-RNA online

Die verschiedenen A,T,G,C und Ind (Ind = Aminoethylindol als Nucleobase) Phosphoramidite wurden zuerst nach bekannten Verfahren hergestellt. Cyanin (Cy3-CE) und Biotin Phosphoramidite wurden bei Glen Research erhalten.

Die vollautomatische Festphasensynthese wurde mit jeweils 15 µmol durchgeführt. Ein Synthesezyklus besteht aus den folgenden Schritten
(a) Detritylierung: 5 Minuten mit 6% DCA (Dichloressigsäure) in CH₂Cl₂ (79 ml);
(b) Waschen mit CH₂Cl₂ (20 ml), Acetonitril (20 ml) und danach spülen mit Argon;
(c) Kupplung: Waschen des Harz mit dem Aktivator (0,5 M Pyridin.HCl in CH₂Cl₂, 0,2 ml; 30-minütiges Behandeln mit Aktivator (0,76 ml) und entsprechendes Phosphoramidit (0,76 ml : 8 eq; 0,1 M in Acetonitril) im Verhältnis 1:1;
(d) Capping: 2 Minuten mit 50% Cap A (10,5 ml) und 50% Cap B (10,5 ml) von Perseptive (Cap A: THF, Lutidine, Acetanhydrid; Cap B: 1-Methylimidazol, THF, Pyridin).
(e) Oxidation: 1 Minute mit 120 ml Iodlösung (400 mg Jod in 100 ml Acetonitril, 46 ml H₂O und 9,2 ml sym-Collidine).
(f) Waschen mit Acetonitril (22 ml).

Zur Erleichterung der nachfolgenden HPLC-Reinigung der Oligonucleotide wurde die letzte DMT(Dimethoxytrityl)- bzw. MMT (Monomethoxytrityl)-Schutzgruppe von Biotin- bzw. Cyanin-Monomeren nicht abgespalten. Der Nachweis der letzten Kupplung mit den modifizierten Phorphoramiditen erfolgt nach der Synthese mit 1% des Harzes mittels Tritylkationabsorption in UV (503 nm).

### Aufarbeitung des Oligonucleotids:

Die Abspaltung der Allyletherschutzgruppen erfolgte mit einer Lösung von Tetrakis(triphenylphosphin)palladium (272mg), Triphenylphosphin (272 mg) und Diethylammoniumhydrogencarbonat in CH₂Cl₂ (15ml) nach 5 Stunden bei RT. Die Glasträger werden danach mit CH₂CL₂ (30ml), Aceton (30ml) und Wasser (30ml) gewaschen. Um Palladiumkomplexreste zu entfernen, wurde das Harz mit einer wäßrigen 0,1 M Natriumdiethyldithiocarbamathydratlösung gespült. Die oben erwähnte Waschoperation wurde in einer umgekehrten Reihe noch einmal durchgeführt. Anschließend wurde das Harz am Hochvakuum 10 Minuten getrocknet. Der Abspaltungsschritt vom Glasträger bei gleichzeitiger Debenzoylierung wurde in 24% Hydrazinhydratlösung (6ml) bei 4°C durchgeführt. Nach HPLC-Kontrolle an RP 18 (18-25 Stunden) wurde das Oligonucleotid "Trityl ON" mittels einer aktiviert (Acetonitril, 20 ml) Waters Sep-Pak Cartridge vom Hydrazin befreit. Das Hydrazin wurde mit TEAB 0,1M (30ml) gewaschen. Das Oligonucleotid wurde dann mit Acetonitril/TEAB 0,1M (10ml) eluiert. Man reinigte mittels HPLC (zur Abtrennung von Abbruchsequenzen) und führte die DMT-Entschützung (30 ml 80%ig wäßrige Ameisensäure) durch. Abschließende Entsalzung (über Sep-Pak Kartuche, mit TEAB Puffer 0,1M/Acetonitril: 1/1) lieferte die reinen 'Cyanin- bzw. Biotin-markierten Oligomeren.

Ein Aliquot dieser Oligolösung wurde für die Durchführung einer ESI-MS verwendet.
4' Cy-AIndTTCCTA 2': berechnet M = 3026,
gefunden (M+H)⁺ = 3027. 4' Biotin-TAGGAAIndT 2': berechnet M = 3014,
gefunden (M+2H)²⁺ m/z 1508 und (m+H)⁺ m/z 3015.

Die Oligos wurden zur Lagerung gefriergetrocknet.

### Beispiel 10

### Jodacetylierung von p-RNA mit N-(jodacetyloxy)-succinimid

*p*-RNA-Sequenz : 4' AGGCAIndT 2' M_{w} = 2266,56 g/mol (Ind = Indol-CH₂-CH₂-NH₂-Linker)

1 eq. der *p*-RNA wurde in einer 0,1 molaren Natriumhydrogencarbonatlösung (pH 8,4) gelöst (1 ml pro 350 nmol) und mit einer Lösung von N-(jodacetyloxy)-succinimid in DMSO versetzt (40 µl pro mg). Man dunkelte den Ansatz mit Aluminiumfolie ab und beließ ihn bei Raumtemperatur für 30-90 Minuten.

Der Fortgang der Reaktion wurde mittels analytischer HPLC verfolgt. Die Standardbedingungen sind:
Puffer A : 0,1 molarer Triethylammoniumacetat-Puffer in Wasser
Puffer B : 0,1 molarer Triethylammoniumacetat-Puffer in Wasser:Acetonitril 1:4
Gradient : von 10% B startend auf 50% B in 40 Minuten
Säulenmaterial : 10 µM LiChrosphere ® 100 RP-18 von Merck Darmstadt GmbH; 250 x 4 mm
Retentionszeit der Edukte: 18,4 Minuten
Retentionszeit der Produkte in diesem Falle : 23,1 Minuten

Nach beendeter Reaktion wurde der Ansatz mit Wasser auf das vierfache Volumen verdünnt. Man aktivierte eine Waters Sep-Pak-Kartusche RP-18 (ab 15 OD 2 g Füllung) mit 2 x 10 ml Acetonitril und 2 x 10 ml Wasser, trug das Oligo auf, ließ es einsinken, wusch das Reaktionsgefäß mit 2 x 10 ml Wasser, wusch mit 3 x 10 ml Wasser nach, um Salz und Reagenz zu entfernen, und eluierte zuerst mit 5 x 1 ml 50:1 Wasser : Acetonitril und anschließend mit 1:1. Das Produkt eluierte in den 1:1-Fraktionen in sehr guter Reinheit. Die Fraktionen wurden in der Kälte und im Dunkeln eingeengt, vereinigt, und wieder eingeengt.

Die Ausbeuten wurden mittels UV-Absorptionspektrometrie bei 260 nm bestimmt.

| Massenspektrometrie : | |
|---|---|
| Sequenz | 4' AGGCAInd(CH₂CH₂NHCOCH₂-I)T 2' |
| berechnete Masse | 2434,50 g/mol |
| gefundene Masse MH₂²⁺ | 1217,9 g/mol = 2433 g/mol |

### Beispiel 11

### Konjugation von p-RNA an ein Peptid der Sequenz CYSKVG::

Die jodacetylierte *p*-RNA (M_{w} = 2434,50 g/mol) wurde in einem Puffersystem gelöst (1000µl pro 114 nmol) und dann mit einer Lösung des Peptides in Puffer versetzt (2 mol CYSKVG-Peptid; M_{w} = 655, 773 g/mol; 228 nmol in 20 µl Puffer).

Puffersystem : Borax/HCl-Puffer der Firma Riedel-de Haën, pH 8,0, wurde im Verhältnis 1:1 mit einer 10 millimolaren Lösung von EDTA-Dinatriumsalz in Wasser gemischt und auf pH 6,3 mit HCI eingestellt. Man erhielt dadurch eine Lösung, die 5 mM Na₂EDTA enthielt.

Man beließ den Ansatz bis zum vollständigen Umsatz bei Raumtemperatur im Dunkeln. Die Reaktion wurde mittels HPLC-Analytik verfolgt.
Die Standardbedingungen sind:
Puffer A: 0,1 molarer Triethylammoniumacetat-Puffer in Wasser
Puffer B: 0,1 molarer Triethylammoniumacetat-Puffer in Wasser: Acetonitril 1:4
Gradient: von 10% B startend auf 50% B in 40 Minuten
Säulenmaterial: 10 µM LiChrosphere® 100 RP-18 von Merck Darmstadt GmbH; 250 x 4
Retentionszeit des Eduktes: 17,6 Minuten
Retentionszeit des Produktes: 15,5 Minuten
Nach beendeter Reaktion wurde der Ansatz direkt mittels RP-HPLC gereinigt. (Standardbedingungen siehe oben).

Die Fraktionen wurden in der Kälte und im Dunkeln eingeengt, vereinigt, und wieder eingeengt. Man nahm sie in Wasser auf und entsalzte sie. Man aktivierte eine Waters Sep-Pak-Kartusche RP-18 (ab 15 OD 2 g Füllung) mit 2 x 10 ml Acetonitril und 2 x 10 ml Wasser, trug das Oligo auf, ließ es einsinken, wusch das Reaktionsgefäß mit 2 x 10 ml Wasser, wusch mit 3 x 10 ml Wasser nach, um das Salz zu entfernen, und eluierte mit Wasser : Acetonitril 1:1. Die Produkt-Fraktionen wurden eingeengt, vereinigt und wieder eingeengt.
Die Ausbeuten wurden mittels UV-Absorptionspektrometrie bei 260 nm bestimmt. Sie erreichten 70-95% der Theorie.

| Massenspektrometrie : | |
|---|---|
| Sequenz | 4' AGGCAInd(CH₂CH₂NHCOCH₂-CYSKVG)T 2' |
| berechnete Masse | 2962,36 g/mol |
| gefundene Masse MH₂²⁺ | 1482,0 g/mol = 2962 g/mol |

### Beispiel 12

### Konjugation von p-RNA an eine Peptidbibliothek

Die jodacetylierte *p*-RNA (M_{w} = 2434,50 g/mol wurde in einem Puffersystem gelöst (1300 µl pro 832 nmol) und dann mit einer Lösung der Peptidbibliothek (CKR-XX-OH); X = Arg, Asn, Glu, His, Leu, Lys, Phe, Ser, Trp, Tyr) in Puffer versetzt (8 mol; mittlere Molekülmasse Mₘ = 677,82 g/mol; 4,5 mg = 6,66 µmol in 200 µmol Puffer).

Puffersystem: Borax/HCl-Puffer der Firma Riedel-de Haen, pH 8,0, wurde im Verhältnis 1:1 mit einer 10 millimolaren Lösung von EDTA-Dinatriumsalz in Wasser gemischt und auf pH 6,6 mit HCl eingestellt. Man erhielt dadurch eine Lösung, die 5 mM Na₂EDTA enthält.

Man beließ den Ansatz bis zum vollständien Umsatz bei Raumtermperatur im Dunkeln. Die Reaktion wurde mittels HPLC-Analytik verfolgt. In diesem Fall war das Edukt nach 70 Stunden verschwunden.

Die Standardbedingungen der analytischen HPLC sind:
Puffer A: 0,1 molarer Triethylammoniumacetat-Puffer in Wasser
Puffer B : 0,1 molarer Triethylammoniumacetat-Puffer in Wasser:Acetonitril 1:4
Gradient: von 10% B startend auf 50% B in 40 Minuten
Säulenmaterial : 10 µM LiChrosphere® 100 RP-18 von Merck Darmstadt GmbH; 250 x 4
Retentionszeit des Eduktes : 18,8 Minuten
Retentionszeit des Produktes: mehrere Peaks von 13,9-36,2 Minuten

Nach beendeter Reaktion wurde der Ansatz mit Wasser auf das vierfache Volumen verdünnt. Man aktivierte eine Waters Sep-Pak-Kartusche RP-18 (ab 15 OD 2g Füllung) mit 3 x 10 ml Acetoniril und 3 x 10 ml Wasser, trug das Oligo auf, ließ es einsinken, wusch das Reaktionsgefäß mit 2 x 10 ml Wasser nach, wusch die Kartusche mit 3 x 10 ml Wasser nach, um Salz und überschüssiges Peptid zu entfernen, und eluierte mit 1:1 Wasser : Acetonitril, bis UV-spektroskopisch kein Prodikt mehr eluierte. Die Franktionen wurden in Kälte und im Dunkeln eingeengt, vereinigt und wieder eingeengt.

## Patentansprüche

1. Verfahren zur Herstellung eines Pentopyranosyl-Nucleosids, **dadurch gekennzeichnet, daß** ausgehend von dem ungeschützten Pentopyranosid
(a) in einem ersten Schritt zuerst die 2'-, 3'- oder 4'-Position des Pentopyranosids geschützt wird, und
(b) in einem zweiten Schritt eine der verbleibenden Positionen 2'-, 3'- oder 4'.

2. Verfahren nach Anspruch 1, **dadurch gekennzeichnet, daß** ein Pentopyranosyl-Nucleosid der Formel (I), 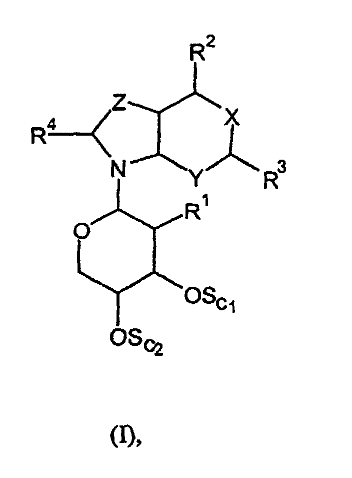 worin
R¹ gleich H, OH, Hal mit Hal gleich Br oder Cl oder ein Rest ausgewählt aus 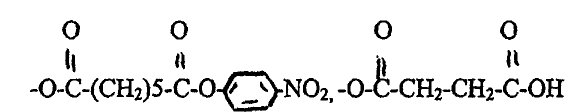 oder -O-P[N(i-Pr)₂] (OCH₂CH₂CN) mit i-Pr gleich Isopropyl, oder eine 0-2' Schutzgruppe, vorzugsweise eine basen- oder metallkatalysiert abspaltbaren Schutzgruppe, insbesondere eine Acylgruppe, besonders bevorzugt eine Benzoylgruppe, R², R³ und R⁴ unabhängig voneinander, gleich oder verschieden, jeweils H, Hal mit Hal gleich Br oder Cl, NR⁵R⁶, OR⁷, SR⁸, =O, CₙH₂ₙ₊₁ mit n eine ganze Zahl von 1-12, vorzugsweise 1-8, insbesondere 1-4 eine β-eliminierbare Gruppe, vorzugsweise eine Gruppe der Formel -OCH₂CH₂R¹⁸ mit R¹⁸ gleich ein Cyano- oder p-Nitrophenylrest oder ein Fluorenylmethyloxycarbonyl-(Fmoc)-Rest, oder (CₙH₂ₙ)NR¹⁰R¹¹, mit R¹⁰R¹¹ gleich H, CₙH₂ₙ₊₁ oder R¹⁰R¹¹ verbunden über einen Rest der Formel 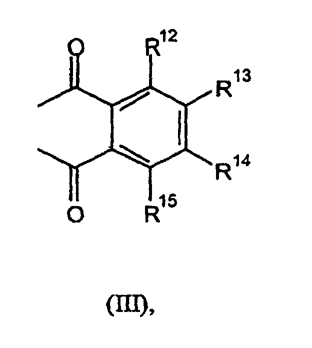 worin R¹², R¹³, R¹⁴ und R¹⁵ unabhängig voneinander, gleich oder verschieden, jeweils H, OR⁷, wobei R⁷ die oben genannte Bedeutung hat, oder CₙH₂ₙ₊₁, oder CₙH₂ₙ₋₁,wobei n die oben genannte Bedeutung hat, bedeuten und
R⁵, R⁶, R⁷ und R⁸ unabhängig voneinander, gleich oder verschieden, jeweils H, CₙH₂ₙ₊₁, oder CₙH₂ₙ₋₁, wobei n die oben genannte Bedeutung hat, -C(O)R⁹ mit R⁹ gleich ein linearer oder verzweigter, gegebenenfalls substituierter Alkyl-, Aryl-, vorzugsweise Phenyl-Rest,
X, Y und Z unabhängig voneinander, gleich oder verschieden, jeweils =N-, =C(R¹⁶)- oder -N(R¹⁷)- mit R¹⁶ und R¹⁷ unabhängig voneinander, gleich oder verschieden, jeweils H oder CₙH₂ₙ₊₁ oder (CₙH₂ₙ)NR¹⁰R¹¹ mit den oben genannten Bedeutungen, bedeutet, und
S_{c1} und S_{c2} unabhängig voneinander, gleich oder verschieden jeweils H oder eine Schutzgruppe ausgewählt aus einer Acyl-, Trityl- oder Allyloxycarbonylgruppe, vorzugsweise eine Benzoyl- oder 4, 4'-Dimethoxytrityl-(DMT-)gruppe,
oder der Formel (II) 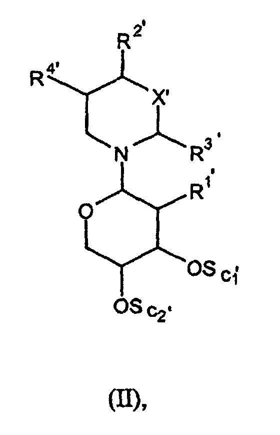 worin R^{1'} gleich H, OH, Hal mit Hal gleich Br oder Cl, oder ein Rest ausgewählt aus 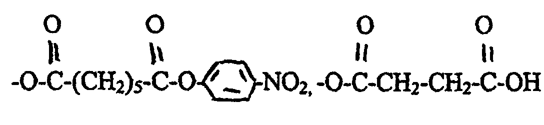 oder -O-P[N(i-Pr)₂] (OCH₂CH₂CN) mit i-Pr gleich Isopropyl, oder eine 0-2' Schutzgruppe, vorzugsweise eine basen- oder metallkatalysiert abspaltbaren Schutzgruppe, insbesondere eine Acylgruppe, besonders bevorzugt eine Benzoylgruppe, R^{2'},R^{3'} und R^{4'} unabhängig voneinander, gleich oder verschieden, jeweils H, Hal mit Hal gleich Br oder Cl =O, CₙH₂ₙ₊₁ oder CₙH₂ₙ₋₁, eine β-eliminierbare Gruppe, vorzugsweise eine Gruppe der Formel -OCH₂CH₂R¹⁸ mit R¹⁸ gleich ein Cyano- oder p-Nitrophenylrest oder ein Fluorenylmethyloxycarbonyl-(Fmoc)-Rest, oder (CₙH₂ₙ)NR^{10'}R^{11'}, wobei R^{10'}, R^{11'}, unabhängig voneinander die oben genannte Bedeutung von R¹⁰ bzw. R¹¹ hat, und
X' jeweils =N-, =C(R^{16'})- oder -N(R^{17'})- bedeutet, wobei R^{16'} und R^{17'} unabhängig voneinander die oben genannte Bedeutung von R¹⁶ bzw. R¹⁷ haben, und S_{c1'} bzw. S_{c2'} die oben genannte Bedeutung von S_{c1} bzw. S_{c2} haben, hergestellt wird.

3. Verfahren nach Anspruch 1 oder 2, **dadurch gekennzeichnet, daß** das Pentopyranosyl-Nucleosid ein Ribo-, Arabino-, Lyxo- und/oder Xylo-pyranosyl-Nucleosid, vorzugsweise ein Ribopyranosyl-Nucleosid, ist.

4. Verfahren nach einem der Ansprüche 1-3, **dadurch gekennzeichnet, daß** der Pentopyranosyl-Teil D- oder L-konfiguriert ist.

5. Verfahren nach einem der Ansprüche 1-4, **dadurch gekennzeichnet, daß** als Pentopyranosyl-Nucleosid ein Pentopyranosyl-purin, -2,6-diaminopurin, -6-purinthiol, -pyridin, -pyrimidin, -adenosin, -guanosin, -isoguanosin, -6-thioguanosin, -xanthin, -hypoxanthin, -thymidin, -cytosin, -isocytosin, -indol, -tryptamin, -N-phthaloyltryptamin, -uracil, -coffein, -theobromin, -theophyllin, -benzotriazol oder -acridin, eingesetzt wird.

6. Verfahren nach einem der Ansprüche 2-5, **dadurch gekennzeichnet, daß** R², R³, R⁴, R^{2'}, R^{3'} und/oder R^{4'} ein 2-Phthalimidoethyl- oder Allyloxy-Rest oder ein Rest der Formel -N[C(O)R⁹)₂ bedeutet.

7. Verfahren nach einem der Ansprüche 1-5, **dadurch gekennzeichnet, daß** das Pentopyranosyl-Nucleosid ein [2',4'-Di-O-Benzoyl-β-ribopyranosyl]-Nucleosid, insbesondere ein [2',4'-Di-O-Benzoyl)-β-ribopyranosyl]-adenin,guanin, -cytosin, -thymidin, -uracil, -xanthin oder hypoxanthin, ein N-Benzoyl-2',4'-di-O-benzoyl-ribopyranosyl-Nucleosid, insbesondere ein -adenin, -guanin oder -cytosin, ein N-Isobutyroyl-2',4'-di-O-benzoylribopyranosyl-Nucleosid, insbesondere ein -adenin, -guanin oder -cytosin, ein O⁶-(2-Cyanoethyl)-N²-isobutyroyl-2',4'-di-O-benzoyl- ribopyranosyl-Nucleosid, insbesondere ein -guanin, ein O⁶ (2-(4-Nitrophenyl)ethyl)-N²isobutyroyl-2',4'-di-O-benzoyl-ribopyranosyl-Nucleosid, insbesondere ein -guanin, ein β-Ribopyranosyl-Nucleosid, insbesondere ein β-Ribopyranosyladenin, -guanin, - cytosin, -thymidin oder -uracil, -xanthin oder hypoxanthin, ein N-Benzoyl-, N-Isobutyroyl-, O⁶-(2-Cyanoethyl)- oder O⁶-(2-(4-Nitrophenyl)ethyl)-N²-isobutylroyl-β-ribopyranosyl-Nucleosid, ein 4'-DMT-pentopyranosyl-Nucleoside, vorzugsweise ein 4'-DMT-ribopyranosyl-Nucleosid, insbesondere ein 4'-DMT-ribopyranosyl-adenin, -guanin,- cytosin, -thymidin, -uracil, -xanthin oder hypoxanthin, ein N-Benzoyl-4'-DMTribopyranosyl-Nucleosid, insbesondere ein N-Benzoyl-4'-DMTribopyranosyl-adenin,-guanin oder- cytosin, ein N-Isobutyroyl-4'-DMTribopyranosyl-Nucleosid, insbesondere ein N-Isobutyroyl-4'-DMTribopyranosyl-adenin, -guanin,- oder -cytosin, ein O⁶-(2-Cyanoethyl)-N²-isobutyroyl-4'-DMT-ribopyranosyl-Nucleosid, insbesondere ein O⁶-(2-Cyanoethyl)-N²-isobutyroyl-4'-DMT-ribopyranosyl-guanin, ein O⁶-(2-(-4Nitrophenyl) ethyl)-N²-isobutyroyl-4'-DMT-ribopyranosyl-Nucleosid, insbesondere ein O⁶-(2-(-4-Nitrophenyl)ethyl)-N²-isobutyroyl-4'-DMTribopyranosyl-guanin, oder ein β-Ribopyranosyl-N,N'-dibenzoyl-adenosin oder ein β-Ribopyranosyl-N,N'-dibenzoyl-guanosin ist.

8. Verfahren nach einem der Ansprüche 1 - 7, **dadurch gekennzeichnet, daß** im Falle einer 2'-geschützten Position eine Umlagerung der Schutzgruppe von der 2'-Position zur 3'-Position erfolgt.

9. Verfahren nach Anspruch 8, **dadurch gekennzeichnet, daß** die Umlagerung in Gegenwart einer Base, insbesondere in Gegenwart von N-Ethyldiisopropylamin und/oder Triethylamin durchgeführt wird.

10. Verfahren nach einem der Ansprüche 1-9, **dadurch gekennzeichnet, daß** das Pyranosyl-nucleosid durch eine säurelabile, basenlabile oder metallkatalysiert abspaltbare Schutzgruppe S_{c1}, S_{c2}, S_{c1}, oder S_{c2'} geschützt wird.

11. Verfahren nach einem der Ansprüche 1-10, **dadurch gekennzeichnet, daß** S_{c1} und S_{c1'} verschieden sind von S_{c2} bzw. S_{c2'}.

12. Verfahren nach einem der Ansprüche 1-11, **dadurch gekennzeichnet, daß** die Schutzgruppen S_{c1}, S_{c2}, S_{c1'} oder S_{c2'} ausgewählt werden aus Acylgruppen, vorzugsweise aus einer Acetyl-, Benzoyl-, Nitrobenzoyl- und/oder Methoxybenzoyl-Gruppe oder aus Tritylgruppen, vorzugsweise einer 4, 4'-Dimethoxytrityl-(DMT)-Gruppe oder aus β-elininierbaren Gruppen, vorzugsweise einer Gruppe der Formel -OCH₂CH₂R¹⁸ mit R¹⁸ gleich ein Cyanooder p-Nitrophenylrest oder einer Fluorenylmethyloxycarbonyl-(Fmoc)Gruppe.

13. Verfahren nach einem der Ansprüche 1-12, **dadurch gekennzeichnet, daß** die 2'- oder 3'-Position durch eine basenlabile oder metallkatalysiert abspaltbare Schutzgruppe, vorzugsweise durch eine Acylgruppe, insbesondere durch eine Acetyl-, Benzoyl-, Nitrobenzoyl- und/oder Methoxybenzoylgruppe, geschützt wird.

14. Verfahren nach einem der Ansprüche 1-13, **dadurch gekennzeichnet, daß** die 4'-Position durch eine säure- oder basenlabile Schutzgruppe, vorzugsweise durch eine Trityl- und/oder Fmoc-Gruppe, insbesondere durch eine DMT-Gruppe geschützt wird.

15. Verfahren nach einem der Ansprüche 1-14, **dadurch gekennzeichnet, daß** bei so tiefen Temperaturen gearbeitet wird, daß die Schutzgruppen hierdurch selektiv eingeführt werden.

16. Verfahren zur Herstellung eines Pentopyranosyl-Nucleosids, **dadurch gekennzeichnet, daß**
(a) eine geschützte Nucleobase mit einer geschützten Pentopyranose umgesetzt wird,
(b) die Schutzgruppen von dem Pentopyranosyl-Teil des Produktes aus Schritt (a) abgespalten werden, und
(c) das Produkt aus Schritt (b) gemäß dem Verfahren nach einem der Ansprüche 1-15 umgesetzt wird.

17. Verfahren nach Anspruch 16, **dadurch gekennzeichnet, daß** die geschützte Pentopyranose Anomeren-rein ist.

18. Verfahren nach Anspruch 16 zur Herstellung eines Pentopyranosyl-Nucleosid-Linkers gemäß Formel (II), worin R^{4'} (CₙH₂ₙ)NR^{10'}R^{11'} bedeutet und R^{10'}R^{11'} über einen Rest der Formel (III) mit der in Anspruch 2 bezeichneten Bedeutung verbunden ist, **dadurch gekennzeichnet, daß**
(a) eine Verbindung der Formel (II) mit R^{4'} gleich (CₙH₂ₙ)OS_{c3} oder (CₙH₂ₙ)Hal, worin n die oben genannte Bedeutung hat, S_{c3} eine Schutzgruppe, vorzugsweise eine Mesylat-Gruppe, und Hal Chlor oder Brom bedeutet,
mit einem Azid umgesetzt wird,
(b) das Reaktionsprodukt aus (a) reduziert wird,
(c) das Reaktionsprodukt aus (b) mit einem entsprechenden Phthalimid umgesetzt wird,
(d) die Hydroxyl-Schutzgruppen des Pyranosylteils des Produkts aus (c) abgespalten werden, und
(e) die Schritte gemäß einem der Ansprüche 7-17 durchgeführt werden.

19. Verfahren nach Anspruch 16 zur Herstellung eines Pentopyranosyl-Nucleosid-Linkers gemäß Formel (II), worin R^{4'} (CₙH₂ₙ)NR^{10'}R^{11'} bedeutet und R^{10'}R^{11'} über einen Rest der Formel (III) mit der in Anspruch 2 bezeichneten Bedeutung verbunden ist, **dadurch gekennzeichnet, daß**
(a) Hydroxyethyluracil mesyliert und anschließend mit einem Azid umgesetzt wird,
(b) das Reaktionsprodukt aus (a) reduziert wird,
(c) das Reaktionsprodukt aus (b) mit einem entsprechenden Phthalimid umgesetzt wird,
(d) das Reaktionsprodukt aus (c) mit einer entsprechenden geschützten Pyranose umgesetzt wird, und
(e) die Schutzgruppen des Pyranosylteils abgespalten werden, und
(f) die Schritte gemäß einem der Ansprüche 7-17 durchgeführt werden.

20. Verfahren nach Anspruch 16 zur Herstellung eines Pentopyranosyl-Nucleosid-Linkers gemäß Formel (I), worin X und Y unabhängig voneinander, gleich oder verschieden, jeweils =C(R¹⁶) mit R¹⁶ gleich H oder CₙH₂ₙ und Z =C(R¹⁶)- mit R¹⁶ gleich (CₙH₂ₙ)NR¹⁰R¹¹ mit der in Anspruch 2 bezeichneten Bedeutung, **dadurch gekennzeichnet, daß**
(a) das entsprechende Indolin
mit einer Pyranose zum Nucleosidtriol umgesetzt wird,
(b) die Hydroxylgruppen des Pyranosyl-Teils des Produktes aus (a) vorzugsweise mit Acylgruppen geschützt werden,
(c) das Produkt aus (b) oxidiert wird,
(d) die Hydroxyl-Schutzgruppen des Pyranosyl-Teils des Produktes aus (c) abgespalten werden und
(e) die Schritte gemäß einem der Ansprüche 7-17 durchgeführt werden.

21. Verfahren nach einem der Ansprüche 1-20, **dadurch gekennzeichnet, daß** das 4'- geschützte Pentopyranosyl-nucleosid in einem weiteren Schritt phosphityliert oder an eine feste Phase gebunden wird.

22. Verfahren zur Herstellung einer Pentopyranosyl-Nucleinsäure, **dadurch gekennzeichnet, daß**
(a) in einem ersten Schritt ein geschütztes Pentopyranosyl-Nucleosid hergestellt nach einem Verfahren gemäß einem der Anspräche 1 - 21 an eine feste Phase gebunden wird und
(b) in einem zweiten Schritt das gemäß Schritt (a) an eine feste Phase gebundene 3'-, 4'-geschützte Pentopyranosylnukleosid in 4'-Position entschützt, um ein phosphityliertes 3'-, 4'-geschütztes Pentopyranosyl-Nucleosid verlängert wird, und
(c) Schritt (b) wiederholt wird.

23. Verfahren nach Anspruch 22, **dadurch gekennzeichnet, daß** in Schritt (a) und/oder Schritt (b) auch Pentofuranosyl-nucleoside eingebaut werden.

24. Verfahren nach Anspruch 22 oder 23, **dadurch gekennzeichnet, daß** als Kupplungsreagenz für die Verlängerung gemäß Schritt (b) saure Aktivatoren wie Pyridinium-Hydrochlorid, vorzugsweise Nitrophenyltetrazol, insbesondere Benzimidazoliumtriflat bei Einsatz von Phosphoramiditen und bei Einsatz von H-Phosphonaten Arylsulfonylchloride, Diphenylchlorophosphonat, Pivaloylchlorid oder Adamantoylchlorid eingesetzt wird.

25. Verfahren nach einem der Ansprüche 22-24, **dadurch gekennzeichnet, daß** in einem weiteren Schritt (d) die Schutzgruppen und das gebildete Oligomer von der festen Phase abgespalten wird.

26. Verfahren nach Anspruch 25, **dadurch gekennzeichnet, daß** die Abspaltung durch Hydrazinolyse vorzugsweise in Gegenwart eines Salzes durchgeführt wird.

27. Verfahren nach einem der Ansprüche 22-26, **dadurch gekennzeichnet, daß** in einem weiteren Schritt ein Allyloxy-Linker der Formel
S_{c4}NH(CₙH₂ₙ)CH(OPS_{c5}S_{c6})CₙH₂ₙS_{c7} (IV),
worin S_{c4} und S_{c7} unabhängig voneinander, gleich oder verschieden, jeweils eine Schutzgruppe insbesondere ausgewählt aus Fmoc und/oder DMT,
S_{c5} und S_{c6} unabhängig voneinander, gleich oder verschieden, jeweils eine Allyloxy- und/oder Diisopropylamino-Gruppe und n wie in Anspruch 2 bezeichnet, bedeuten,
eingebaut wird.

## Claims

1. Process for the preparation of a pentopyranosylnucleoside, **characterized in that**, starting from the unprotected pentopyranoside,
(a) in a first step the 2'-, 3'- or 4'-position of the pentopyranoside is first protected, and
(b) in a second step one of the remaining positions 2'-,3'- or 4' is protected.

2. Process according to Claim 1, **characterized in that** a pentopyranosylnucleoside of the formula (I), 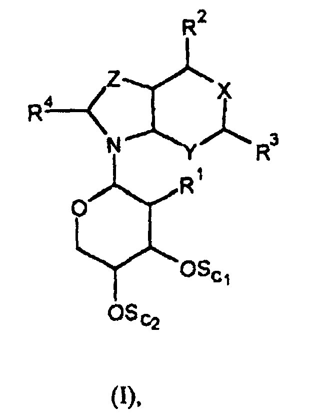 in which
R¹ is equal to H, OH, Hal where Hal is equal to Br or Cl or a radical selected from 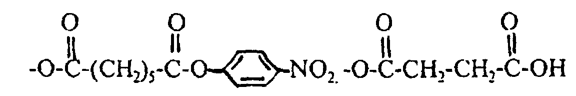 or
-O-P[N(i-Pr)₂]-(OCH₂CH₂CN) where i-Pr is equal to isopropyl, or a O-2'-protective group, preferably a protective group which can be removed by base or metal catalysis, in particular an acyl group, particularly preferably a benzoyl group, R², R³ and R⁴ independently of one another, identically or differently, are in each case H, Hal where Hal is equal to Br or Cl, NR⁵R⁶, OR⁷, SR⁸, =O, CₙH₂ₙ₊₁ where n is an integer from 1-12, preferably 1-8, in particular 1-4, a β-eliminable group, preferably a group of the formula -OCH₂CH₂R¹⁸ where R¹⁸ is equal to a cyano or p-nitrophenyl radical or a fluorenylmethyloxycarbonyl (Fmoc) radical, or (CₙH₂ₙ)NR¹⁰R¹¹ where R¹⁰R¹¹ is equal to H, CₙH₂ₙ₊₁ or R¹⁰R¹¹ linked via a radical of the formula 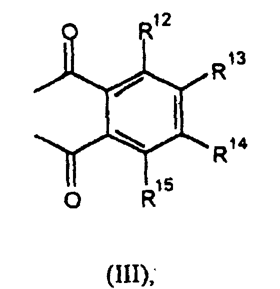 in which R¹², R¹³, R¹⁴ and R¹⁵ independently of one another, identically or differently, are in each case H, OR⁷, where R⁷ has the abovementioned meaning, or CₙH₂ₙ₊₁, or CₙH₂ₙ₋₁, where n has the abovementioned meaning, and
R⁵, R⁶, R⁷ and R⁸ independently of one another, identically or differently, is in each case H, CₙH₂ₙ₊₁, or CₙH₂ₙ₋₁, where n has the abovementioned meaning, -C(O)R⁹ where R⁹ is equal to a linear or branched, optionally substituted alkyl or aryl radical, preferably a phenyl radical,
X, Y and Z independently of one another, identically or differently, is in each case =N-, =C(R¹⁶)- or -N(R¹⁷)- where R¹⁶ and R¹⁷ independently of one another, identically or differently, is in each case H or CₙH₂ₙ₊₁ or (CₙH₂ₙ)NR¹⁰R¹¹ having the abovementioned meanings, and
S_{c1} and S_{c2} independently of one another, identically or differently, is in each case H or a protective group selected from an acyl, trityl or allyloxycarbonyl group, preferably a benzoyl or 4,4'-dimethoxytrityl (DMT) group,
or of the formula (II) 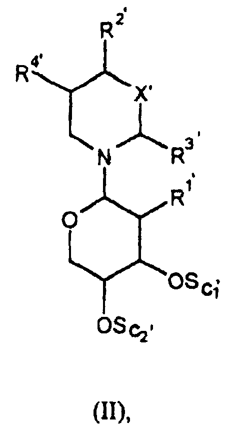 in which R^{1'} is equal to H, OH, Hal where Hal is equal to Br or Cl, or a radical selected from 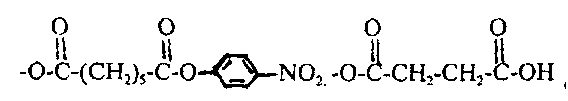 or
-O-P[N(i-Pr)₂]-(OCH₂CH₂CN) where I-Pr is equal to isopropyl, or a O-2'- protective group, preferably a protective group which can be removed by base or metal catalysis, in particular an acyl group, particularly preferably a benzoyl group, R^{2'}, R^{3'} and R^{4'} independently of one another, identically or differently, are in each case H, Hal where Hal is equal to Br or Cl, =O, CₙH₂ₙ₊₁ or -CₙH₂ₙ₋₁, a β-eliminable group, preferably a group of the formula -OCH₂CH₂R¹⁸ where R¹⁸ is equal to a cyano or p-nitrophenyl radical or a fluorenylmethyloxycarbonyl (Fmoc) radical, or (CₙH₂ₙ)NR^{10'}R^{11'}, where R^{10'}, R^{11'}, independently of one another has the abovementioned meaning of R¹⁰ or R¹¹, and
X' is =N-, =C(R^{16'})- or -N(R^{17'})-, where R^{16'} and R^{17'} independently of one another have the abovementioned meaning of R¹⁶ or R¹⁷, and S_{c1'} and S_{c2'} have the abovementioned meaning of S_{c1} and S_{c2}.

3. Process according to Claim 1 or 2, **characterized in that** the pentopyranosylnucleoside is a ribo-, arabino-, lyxo- and/or xylopyranosylnucleoside, preferably a ribopyransoylnucleoside.

4. Process according to one of the Claims 1-3, **characterized in that** the pentopyranosyl moiety of the pentopyranosylnucleoside is in the D or L configuration.

5. Process according to one of the Claims 1-4, **characterized in that** the pentopyranosylnucleoside employed is a pentopyranosyl purine, -2,6-diaminopurine, -6-purinethiol, -pyridine, -pyrimidine, -adenosine, -guanosine, -isoguanosine, -6-thioguanosine, -xanthine, -hypoxanthine, -thymidine, -cytosine, -isocytosine, -indole, -tryptamine, -N-phthaloyltryptamine, -uracil, -caffeine, -theobromine, -theophylline, -benzotriazole or -acridine.

6. Process according to one of the Claims 2-5, **characterized in that** R², R³, R⁴, R^{2'}, R^{3'} and/or R^{4'} is a 2-phthalimidoethyl or allyloxy radical or a radical of the formula -N[C(O)R⁹]₂.

7. Process according to one of the Claims 1-5, **characterized in that** the pentopyranosylnucloside is a [2',4'-di-O-benzoyl-β-ribopyranosyl]nucleoside, in particular a [2',4'-di-O-benzoyl)-β-ribopyranosyl]adenine, -guanine, -cytosine, -thymidine, -uracil, -xanthine or hypoxanthine, an N-benzoyl-2',4'-di-O-benzoylribopyranosylnucleoside, in particular an -adenine, -guanine or -cytosine, an N-isobutyroyl-2',4'-di-O-benzoylribopyranosylnucleoside, in particular an -adenine, -guanine or -cytosine, an O⁶-(2-cyanoethyl)-N²-isobutyroyl-2',4'-di-O-benzoylribopyranosylnucleoside, in particular a -guanine, an O⁶-(2-(4'-nitrophenyl)ethyl)-N²-isobutyroyl-2',4'-di-O-benzoylribopyranosylnucleoside, in particular a -guanine, a β-ribopyranosylnucleoside, in particular a β-ribopyranosyladenine, -guanine, -cytosine, -thymidine or -uracil, -xanthine or hypoxanthine, an N-benzoyl-, N-isobutyroyl-, O⁶-(2-cyanoethyl)- or O⁶-(2-(4-nitrophenyl)ethyl)-N²-isobutyroyl-β-ribopyranosylnucleoside, a 4'-DMT-pentbpyranosylnucleoside, preferably a 4'-DMT-ribopyranosylnucleoside, in particular a 4'-DMT-ribopyranosyladenine, -guanine, -cytosine, -thymidine, -uracil, -xanthine or hypoxanthine, an N-benzoyl-4'-DMT-ribopyranosylnucleoside, in particular an N-benzoyl-4'-DMT-ribopyranosyl-adenine, -guanine or -cytosine, an N-isobutyroyl-4'-DMT-ribopyranosylnucleoside, in particular an N-isobutyroyl-4'-DMT-ribopyranosyladenine, -guanine,- or -cytosine, an O⁶-(2-cyanoethyl)-N²-isobutyroyl-4'-DMT-ribopyranosylnucleoside, in particular an O⁶-(2-cyanoethyl)-N²-isobutyroyl-4'-DMT-ribopyranosyl-guanine, an O⁶-(2-(-4-nitrophenyl)ethyl)-N²-isobutyroyl-4'-DMT-ribopyranosylnucleoside, in particular an O⁶-(2-(-4-nitrophenyl)ethyl)-N²-isobutyroyl-4'-DMT-ribopyranosyl-guanine or a β-ribopyranosyl-N,N'-dibenzoyladenosine or a β-ribopyranosyl-N,N'-dibenzoylguanosine.

8. Process according to one of the Claims 1-7, **characterized in that** the case of a 2'-protected position, a rearrangement of the protective group takes place from the 2'-position to the 3'-position.

9. Process according to Claim 8, **characterized in that** the rearrangement is carried out in the presence of N-ethyldiisopropylamine and/or triethylamine.

10. Process according to one of the Claims 1-9, **characterized in that** the pyranosylnucleoside is protected by a protective group S_{c1}, S_{c2}, S_{c1'} or S_{c2'} which is acid-labile or can removed by metal catalysis.

11. Process according to one of the Claims 1-10, chracterized in that S_{c1} and S_{c1'} are other than S_{c2} or S_{c2'}.

12. Process according to one of the Claims 1-11, chrarcterized in that the protective groups S_{c1}, S_{c2}, S_{c1'} or S_{c2'} are selected from acyl groups, preferably from an acetyl, benzoyl, nitrobenzoyl and/or methoxybenzoyl group or from trityl groups, preferably a 4,4'-dimethoxytrityl (DMT) group or from β-eliminable groups, preferably a group of formula -OCH₂CH₂R¹⁸ where R¹⁸ is equal to a cyano or p-nitrophenyl radical, or a fluorenylmethyloxycarbonyl(Fmoc) group.

13. Process according to one of the Claims 1-12, **characterized in that** the 2'- or 3'-position is protected by a protective group which is base-labile or can be removed by metal catalysis, preferably by an acyl group, in particular by an acetyl, benzoyl, nitrobenzoyl and/or methoxybenzoyl group.

14. Process according to one of Claims 1-13, **characterized in that** the 4' -position is protected by an acid- or baselabile protective group, preferably by a trityl and/or Fmoc group, in particular by a DMT group.

15. Process according to one of the Claims 1-14, **characterized in that** the reaction is carried out at temperatures which are so low that the protective groups are selectively introduced as a result.

16. Process for the preparation of a pentopyranosyl nucleoside, **characterized in that**
(a) a protected nucleobase is reacted with a protected pentopyranose,
(b) the protective groups are removed from the pentopyranosyl moiety of the product from step (a), and
(c) the product from step (b) is reacted according to the process according to one of the Claims 1-15.

17. Process according to Claim 16, **characterized in that** the protected pentopyranose is anomerically pure.

18. Process according to Claim 16 for the preparation of a pentopyranosylnucleoside linker according to formula (II), in which R^{4'} is (CₙH₂ₙ) NR^{10'}R^{11'} and R¹⁰R^{11'} is linked to the meaning designated in Claim 2 via a radical of the formula (III), **characterized in that**
(a) a compound of the formula (II) where R^{4'} is equal to (CₙH₂ₙ)OS_{c3} or (CₙH₂ₙ)Hal, in which n has the above mentioned meaning, S_{c3} is a protective group, preferably a mesylate group, and Hal is chlorine or bromine,
is reacted with an azide,
(b) the reaction product from (a) is reduced,
(c) the reaction product from (b) is reacted with an appropriate phthalimide,
(d) the hydroxyl protective groups of the pyranosyl moiety of the product from (c) are removed, and
(e) the steps according to one of the Claims 7-17 are carried out.

19. Process according to Claim 16 for the preparation of a pentopyranosylnucleoside linker according to formula (II), in which R⁴' is (CₙH₂ₙ) NR^{10'}R^{11'} and R¹⁰R^{11'} is linked to the meaning designated in Claim 2 via a radical of the formula (III), **characterized in that**
(a) hydroxyethyluracil is mesylated and subsequently reacted with an azide,
(b) the reaction product from (a) is reduced,
(c) the reaction product from (b) is reacted with an appropriate phthalimide,
(d) the reaction product from (c) is reacted with an appropriate procted pyranose,
(e) the protective groups of the pyranosyl moiety are removed, and
(f) the steps according to one of the Claims 7-17 are carried out.

20. Process according to Claim 16 for the preparation of a pentopyranosylnucleoside linker according to formula (I), in which X and Y independently of one another, identically or differently, in each case =C(R¹⁶) where R¹⁶ is equal to H or CₙH₂ₙ and Z is =C(R¹⁶)-where R¹⁶ is equal to (CₙH₂ₙ)NR¹⁰R¹¹ having the meaning designated in Claim 2, **characterized in that**
(a) the appropriate indoline
is reacted with a pyranose to give the nucleoside triol,
(b) the hydroxyl groups of the pyranosyl moiety of the product from (a) are protected, preferably with acyl groups,
(c) the product from (b) is oxidized,
(d) the hydroxyl protective groups of the pyranosyl moiety of the product from (c) are removed and
(e) the steps according to one of the Claims 7-17 are carried out.

21. Process according to one of the Claims 1-20, **characterized in that** the 4'-protected pentopyranosylnucleoside is phosphitylated in a further step or bonded to a solid phase.

22. Process for the preparation of a pentopyranosylnucleic acid, **characterized in that**
(a) in a first step a protected pentopyranosylnucleoside prepared by a process according to one of the Claims 1-21 is bonded to a solid phase and
(b) in a second step the 3'-, 4'-protected pentopyranosylnucleoside bonded to a solid phase according to step (a) is deprotected in the 4'-position, is lengthened by a phosphitylated 3'-, 4'-protected pentopyranosylnucleoside, and
(c) step (b) is repeated.

23. Process according to Claim 22, **characterized in that** in step (a) and/or step (b) pentofuranosylnucleosides are also incorporated.

24. Process according to Claim 22 or 23, **characterized in that** the coupling reagent employed for the lengthening according to step (b) is acidic activators such as pyridinium hydrochloride, preferably nitrophenyltetrazole, in particular benzimidazolium triflate when employing phosphoramidites and, when employing H-phosphonates arylsulphonyl chlorides, diphenyl chlorophosphonate, pivaloyl chloride or adamantoyl chloride.

25. Process according to one of the Claims 22-24, **characterized in that** in a further step (d) the protective groups and the oligomer formed is removed from the solid phase.

26. Process according to Claim 25, **characterized in that** the removal by means of hydrazinolysis is preferably carried out in the presence of a salt.

27. Process according to one of the Claims 22-26, **characterized in that** in a further step an allyloxy linker of the formula
Sc4NH (C,H2n) CH (OPSc5Sc6) CnH2nSc7 (IV)
in which S_{c4} and S_{c5}, independently of one another, identically or differently, are in each case a protective group in particular selected from Fmoc and/or DMT,
S_{c5} and S_{c6} independently of one another, identically or differently, are in each case an allyloxy and/or diisopropylamino group and n is as designated in Claim 2,
is incorporated.

## Revendications

1. Procédé de préparation d'un pentopyranosylnucléoside, **caractérisé en ce que**, à partir du pentopyranoside non protégé,
(a) dans une première étape, on protège d'abord la position 2', 3' ou 4' du pentopyranoside, et
(b) dans une deuxième étape, on protège l'une des positions 2', 3' ou 4' restantes.

2. Procédé selon la revendication 1, **caractérisé en ce que** l'on prépare un pentopyranosylnucléoside de formule (I) 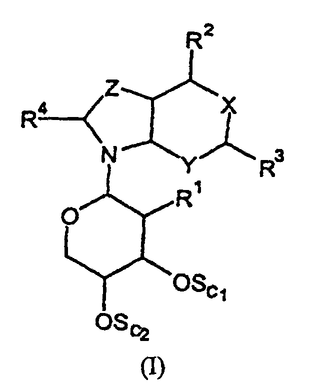 dans laquelle
R¹ représente H, OH, Hal, Hal étant Br ou Cl, ou un reste choisi parmi 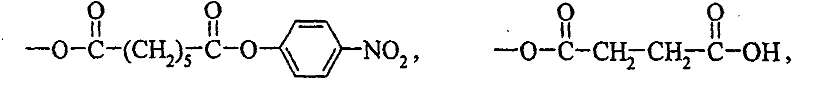 ou -O-P[N(i-Pr)₂](OCH₂CH₂CN) où i-Pr est un groupe isopropyle, ou un groupe protecteur de O-2', de préférence un groupe protecteur dissociable par catalyse avec une base ou un métal, en particulier un groupe acyle, de façon particulièrement préférée un groupe benzoyle, R², R³ et R⁴ sont identiques ou différents et représentent chacun, indépendamment les uns des autres, H, Hal, Hal étant Br ou Cl, NR⁵R⁶, OR⁷, SR⁸, =O, CₙH₂ₙ₊₁, n étant un nombre entier de 1 à 12, de préférence de 1 à 8, de façon particulièrement préférée de 1 à 4, un groupe β-éliminable, de préférence un groupe de formule -OCH₂CH₂R¹⁸ dans laquelle R¹⁸ est un reste cyano ou p-nitrophényle ou un reste fluorénylméthyloxycarbonyle (Fmoc), ou (CₙH₂ₙ)NR¹⁰R¹¹, dans lequel R¹⁰ et R¹¹ sont H, CₙH₂ₙ₊₁, ou R¹⁰ et R¹¹ forment ensemble un reste 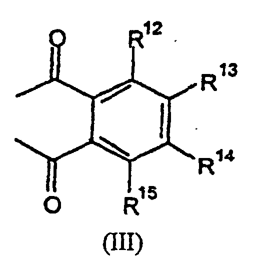 dans laquelle R¹², R¹³, R¹⁴ et R¹⁵ sont identiques ou différents et représentent chacun, indépendamment les uns des autres, H, OR⁷, R⁷ ayant la signification donnée ci-dessus, Ou CₙH₂ₙ₊₁, ou CₙH₂ₙ₋₁, n ayant la signification donnée ci-dessus, et
R⁵, R⁶, R⁷ et R⁸ sont identiques ou différents et représentent chacun, indépendamment les uns des autres, H, CₙH₂ₙ₊₁, ou CₙH₂ₙ₋₁, n ayant la signification donnée ci-dessus, -C(O)R⁹, R⁹ étant un reste alkyle linéaire ou ramifié, aryle, de préférence phényle, éventuellement substitué,
X, Y et Z sont identiques ou différents et représentent chacun, indépendamment les uns des autres, =N-, =C(R¹⁶)- ou -N(R¹⁷)-, R¹⁶ et R¹⁷ étant identiques ou différents et représentant chacun, indépendamment l'un de l'autre, H ou CₙH₂ₙ₊₁ ou (CₙH₂ₙ)NR¹⁰R¹¹ avec les significations indiquées ci-dessus, et
S_{c1} et S_{c2} sont identiques ou différents et représentent chacun, indépendamment l'un de l'autre, H ou un groupe protecteur choisi parmi les groupes acyle, trityle et allyloxycarbonyle, de préférence un groupe benzoyle ou 4,4'-diméthoxytrityle (DMT),
ou de formule (II) 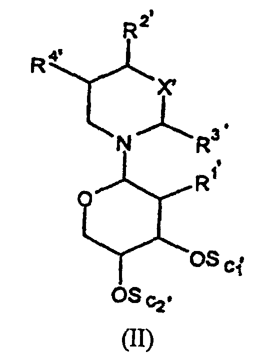 dans laquelle R^{1'} représente H, OH, Hal, Hal étant Br ou Cl, ou un reste choisi parmi 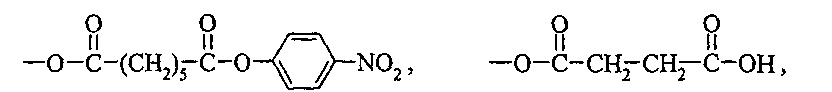 ou -O-P[N(i-Pr)₂](OCH₂CH₂CN) où i-Pr est un groupe isopropyle, ou un groupe protecteur de O-2', de préférence un groupe protecteur dissociable par catalyse avec une base ou un métal, en particulier un groupe acyle, de façon particulièrement préférée un groupe benzoyle, R^{2'}, R^{3'} et R^{4'} sont identiques ou différents et représentent chacun, indépendamment les uns des autres, H, Hal, Hal étant Br ou Cl, =O, CₙH₂ₙ₊₁ ou CₙH₂ₙ₋₁, un groupe β-éliminable, de préférence un groupe de formule -OCH₂CH₂R¹⁸ dans laquelle R¹⁸ est un reste cyano ou p-nitrophényle ou un reste fluorénylméthyloxycarbonyle (Fmoc), ou (CₙH₂ₙ)NR^{10'}R^{11'}, dans lequel R^{10'} et R^{11'} ont indépendamment l'un de l'autre la signification indiquée ci-dessus respectivement pour R¹⁰ et R¹¹, et
X' représente =N-, =C(R^{16'})- ou -N(R^{17'})-, R^{16'} et R^{17'} ayant indépendamment l'un de l'autre la signification donnée ci-dessus respectivement pour R¹⁶ et R¹⁷,
et S_{c1'} et S_{c2'} ont la signification donnée ci-dessus respectivement pour S_{c1} et S_{c2}.

3. Procédé selon la revendication 1 ou 2, **caractérisé en ce que** le pentopyranosylnucléoside est un ribo-, arabino-, lyxo- et/ou xylopyranosylnucléoside, de préférence un ribopyranosylnucléoside.

4. Procédé selon l'une des revendications 1 à 3, **caractérisé en ce que** la partie pentopyranosyle a la configuration D ou L.

5. Procédé selon l'une des revendications 1 à 4, **caractérisé en ce que** l'on utilise comme pentopyranosylnucléoside une pentopyranosylpurine, une pentopyranosyl-2,4-diaminopurine, un pentopyranosyl-6-purinethiol, une pentopyranosylpyridine, une pentopyranosylpyrimidine, une pentopyranosyladénosine, une pentopyranosylguanosine, une pentopyranosylisoguanosine, une pentopyranosyl-6-thioguanosine, une pentopyranosylxanthine, une pentopyranosylhypoxanthine, une pentopyranosylthymidine, une pentopyranosylcytosine, une pentopyranosylisocytosine, un pentopyranosylindole, une pentopyranosyltryptamine, une pentopyranosyl-N-phtaloyltryptamine, un pentopyranosyluracile, une pentopyranosylcaféine, une pentopyranosylthéobromine, une pentopyranosylthéophylline, un pentopyranosylbenzotriazole ou une pentopyranosylacridine.

6. Procédé selon l'une des revendications 2 à 5, **caractérisé en ce que** R², R³, R⁴, R^{2'}, R^{3'} et/ou R^{4'} représentent un reste 2-phtalimidoéthyle ou allyloxy ou un reste de formule -N[C(O)R⁹]₂.

7. Procédé selon l'une des revendications 1 à 5, **caractérisé en ce que** le pentopyranosylnucléoside est un [2',4'-di-O-benzoyl-β-ribopyranosyl]nucléoside, en particulier une [2',4'-di-O-benzoyl-β-ribopyranosyl]adénine, une [2',4'-di-O-benzoyl-β-ribopyranosyl]guanine, une [2',4'-di-O-benzoyl-β-ribopyranosyl]cytosine, une [2',4'-di-O-benzoyl-β-ribopyranosyl]thymidine, un [2',4'-di-O-benzoyl-β-ribopyranosyl]uracile, une [2',4'-di-O-benzoyl-β-ribopyranosyl]xanthine, ou une [2',4'-di-O-benzoyl-β-ribopyranosyl]hypoxanthine, un N-benzoyl-2',4'-di-O-benzoylribopyranosylnucléoside, en particulier une N-benzoyl-2',4'-di-O-benzoylribopyranosyladénine, -guanine ou -cytosine, un N-isobutyroyl-2',4'-di-O-benzoylribopyranosylnucléoside, en particulier une N-isobutyroyl-2',4'-di-O-benzoylribopyranosyladénine, -guanine ou -cytosine, un O⁶-(2-cyanoéthyl)-N²-isobutyroyl-2',4'-di-O-benzoylribopyranosylnucléoside, en particulier une O⁶-(2-cyanoéthyl)-N²-isobutyroyl-2',4'-di-O-benzoylribopyranosylguanine, un O⁶-(2-(4-nitrophényl)éthyl)-N²-isobutyroyl-2',4'-di-O-benzoylribopyranosylnucléoside, en particulier une O⁶-(2-(4-nitrophényl)éthyl)-N²-isobutyroyl-2',4'-di-O-benzoylribopyranosylguanine, un β-ribopyranosylnucléoside, en particulier une β-ribopyranosyladénine, une β-ribopyranosylguanine, une β-ribopyranosylcytosine, une β-ribopyranosylthymidine, un β-ribopyranosyluracile, une β-ribopyranosylxanthine, ou une β-ribopyranosylhypoxanthine, un N-benzoyl-, N-isobutyroyl-, O⁶-(2-cyanoéthyl)- ou O⁶-(2-(4-nitrophényl)éthyl)-N²-isobutyroyl-β-ribopyranosylnucléoside, un 4'-DMT-pentopyranosylnucléoside, de préférence un 4'-DMT-ribopyranosylnucléoside, en particulier une 4'-DMT-ribopyranosyladénine, une 4'-DMT-ribopyranosylguanine, une 4'-DMT-ribopyranosylcytosine, une 4'-DMT-ribopyranosylthymidine, un 4'-DMT-ribopyranosyluracile, une 4'-DMT-ribopyranosylxanthine ou une 4'-DMT-ribopyranosylhypoxanthine, un N-benzoyl-4'-DMT-ribopyranosylnucléoside, en particulier une N-benzoyl-4'-DMT-ribopyranosyladénine, -guanine ou -cytosine, un N-isobutyroyl-4'-DMT-ribopyranosylnucléoside, en particulier une N-isobutyroyl-4'-DMT-ribopyranosyladénine, -guanine ou -cytosine, un O⁶-(2-cyanoéthyl)-N²-isobutyroyl-4'-DMT-ribopyranosylnucléoside, en particulier une O⁶-(2-cyanoéthyl)-N²-isobutyroyl-4'-DMT-ribopyranosylguanine, un O⁶-(2-(4-nitrophényl)éthyl)-N²-isobutyroyl-4'-DMT-ribopyranosylnucléoside, en particulier une O⁶-(2-(4-nitrophényl)éthyl)-N²-isobutyroyl-4'-DMT-ribopyranosylguanine, ou une β-ribopyranosyl-N,N'-dibenzoyladénosine ou une β-ribopyranosyl-N,N'-dibenzoylguanosine.

8. Procédé selon l'une des revendications 1 à 7, **caractérisé en ce que**, dans le cas d'une position 2' protégée, il se produit une transposition du groupe protecteur de la position 2' à la position 3'.

9. Procédé selon la revendication 8, **caractérisé en ce que** la transposition s'effectue en présence d'une base, en particulier en présence de N-éthyldiisopropylamine et/ou de triéthylamine.

10. Procédé selon l'une des revendications 1 à 9, **caractérisé en ce que** le pyranosylnucléoside est protégé par un groupe protecteur S_{c1}, S_{c2}, S_{c1'} ou S_{c2'}, labile en milieu acide, labile en milieu basique, ou dissociable par catalyse avec un métal.

11. Procédé selon l'une des revendications 1 à 10, **caractérisé en ce que** S_{c1} et S_{c1'} sont respectivement différents de S_{c2} et S_{c2'}.

12. Procédé selon l'une des revendications 1 à 12, **caractérisé en ce que** les groupes protecteurs S_{c1}, S_{c2}, S_{c1'} ou S_{c2'} sont choisis parmi des groupes acyle, en particulier des groupes acétyle, benzoyle, nitrobenzoyle et/ou méthoxybenzoyle, ou des groupes trityle, de préférence un groupe 4,4'-diméthoxytrityle (DMT) ou des groupes β -éliminables, de préférence un groupe de formule - OCH₂CH₂R¹⁸ dans laquelle R¹⁸ est un reste cyano ou p-nitrophényle ou un groupe fluorénylméthyloxycarbonyle (Fmoc).

13. Procédé selon l'une des revendications 1 à 12, **caractérisé en ce que** la position 2' ou 3' est protégée par un groupe protecteur labile en milieu basique ou dissociable par catalyse avec un métal, de préférence par un groupe acyle, en particulier par un groupe acétyle, benzoyle, nitrobenzoyle et/ou méthoxybenzoyle.

14. Procédé selon l'une des revendications 1 à 13, **caractérisé en ce que** la position 4' est protégée par un groupe protecteur labile en milieu acide ou basique, de préférence par un groupe trityle et/ou Fmoc, en particulier par un groupe DMT.

15. Procédé selon l'une des revendications 1 à 14, **caractérisé en ce que** l'on travaille à des températures assez basses pour entraîner l'introduction sélective des groupes protecteurs.

16. Procédé de préparation d'un pentopyranosylnucléoside, **caractérisé en ce que**
(a) on fait réagir une base nucléique protégée avec un pentopyranose protégé,
(b) on sépare les groupes protecteurs de la partie pentopyranosyle du produit de l'étape (a), et
(c) on fait réagir le produit de l'étape (b) par le procédé selon l'une des revendications 1 à 15.

17. Procédé selon la revendication 16, **caractérisé en ce que** le pentopyranose protégé est un anomère pur.

18. Procédé selon la revendication 16 pour la préparation d'un lieur pentopyranosylnucléoside selon la formule (II) dans laquelle R^{4'} représente (CₙH₂ₙ)NR^{10'}R^{11'} et R^{10'}et R^{11'} forment ensemble un reste de formule (III) ayant la signification indiquée dans la revendication 2, **caractérisé en ce que**
(a) on fait réagir avec un azide un composé de formule (II) dans laquelle R^{4'} représente (CₙH₂ₙ)OS_{c3} ou (CₙH₂ₙ)Hal, où n a la signification indiquée ci-dessus, S_{c3} représente un groupe protecteur, de préférence un groupe mésylate, et Hal représente le chlore ou le brome,
(b) on réduit le produit de réaction de (a),
(c) on fait réagir le produit de réaction de (b) avec un phtalimide correspondant,
(d) on sépare les groupes protecteurs d'hydroxyle de la partie pyranosyle du produit de (c), et
(e) on effectue les étapes selon l'une des revendications 7 à 17.

19. Procédé selon la revendication 16 pour la préparation d'un lieur pentopyranosylnucléoside selon la formule (II) dans laquelle R^{4'} représente (CₙH₂ₙ)NR^{10'}R^{11'} et R^{10'} et R^{11'} forment ensemble un reste de formule (III) ayant la signification indiquée dans la revendication 2, **caractérisé en ce que**
(a) on soumet de l'hydroxyéthyluracile à une mésylation, puis on le fait réagir avec un azide,
(b) on réduit le produit de réaction de (a),
(c) on fait réagir le produit de réaction de (b) avec un phtalimide correspondant,
(d) on fait réagir le produit de réaction de (c) avec un pyranose protégé correspondant,
(e) on sépare les groupes protecteurs d'hydroxyle de la partie pyranosyle, et
(f) on effectue les étapes selon l'une des revendications 7 à 17.

20. Procédé selon la revendication 16 pour la préparation d'un lieur pentopyranosylnucléoside de formule (I) dans laquelle X et Y sont identiques ou différents et représentent chacun, indépendamment l'un de l'autre, un groupe =C(R¹⁶) dans lequel R¹⁶ représente H ou CₙH₂ₙ, et Z représente un groupe =C(R¹⁶) dans lequel R¹⁶ est un groupe (CₙH₂ₙ)NR¹⁰R¹¹ ayant la signification donnée dans la revendication 2, **caractérisé en ce que**
(a) on fait réagir l'indoline correspondante avec un pyranose pour former le nucléosidetriol,
(b) on protège les groupes hydroxyle de la partie pyranosyle du produit de (a), de préférence avec des groupes acyle,
(c) on oxyde le produit de (b),
(d) on sépare les groupes protecteurs d'hydroxyle de la partie pyranosyle du produit de (c) et
(e) on effectue les étapes selon l'une des revendications 7 à 17.

21. Procédé selon l'une des revendications 1 à 20, **caractérisé en ce que**, dans une autre étape, on soumet le pentopyranosylnucléoside protégé en 4' à une phosphitylation ou on le fixe à une phase solide.

22. Procédé de préparation d'un acide pentopyranosylnucléique, **caractérisé en ce que**
(a) dans une première étape, on fixe un pentopyranosylnucléoside protégé par un procédé selon l'une des revendications 1 à 21 sur une phase solide et
(b) dans une deuxième étape, on élimine le groupe protecteur en position 4' du pentopyranosylnucléoside protégé en 3',4' fixé sur une phase solide selon l'étape (a), et on l'allonge d'un pentopyranosylnucléoside protégé en 3' et 4' et phosphitylé, et
(c) on répète l'étape (b).

23. Procédé selon la revendication 22, **caractérisé en ce que**, dans l'étape (a) et/ou l'étape (b), on incorpore aussi des pentofuranosylnucléosides.

24. Procédé selon la revendication 22 ou 23, **caractérisé en ce que** l'on utilise comme réactif de couplage pour l'allongement de l'étape (b) des activateurs acides comme le chlorhydrate de pyridinium, de préférence du nitrophényltétrazole, en particulier du triflate de benzimidazolium lorsque l'on utilise des phosphoramidites, et des chlorures d'arylsulfonyle, du chlorophosphonate de diphényle, du chlorure de pivaloyle ou du chlorure d'adamantoyle lorsque l'on utilise des H-phosphonates.

25. Procédé selon l'une des revendications 22 à 24, **caractérisé en ce que**, dans une étape (d) ultérieure, on élimine les groupes protecteurs et on sépare l'oligomère formé de la phase solide.

26. Procédé selon la revendication 25, **caractérisé en ce que** la séparation s'effectue par hydrazinolyse, de préférence en présence d'un sel.

27. Procédé selon l'une des revendications 22 à 26, **caractérisé en ce que**, dans une autre étape, on incorpore un lieur allyloxy de formule
S_{c4}NH(CₙH₂ₙ)CH(OPS_{c5}S_{c6})CₙH₂ₙS_{c7}
dans laquelle S_{c4} et S_{c7} sont identiques ou différents et représentent chacun, indépendamment l'un de l'autre, un groupe protecteur, en particulier choisi parmi les groupes Fmoc et/ou DMT,
S_{c5} et S_{c6} sont identiques ou différents et représentent chacun, indépendamment l'un de l'autre, un groupe allyloxy et/ou diisopropylamino, et n a la signification donnée dans la revendication 2.
